(19) Europäisches Patentamt / European Patent Office / Office européen des brevets

(11) **EP 4 158 058 B1**

(12) **EUROPEAN PATENT SPECIFICATION**

(45) Date of publication and mention
of the grant of the patent:
**06.08.2025 Bulletin 2025/32**

(21) Application number: **21734724.4**

(22) Date of filing: **01.06.2021**

(51) International Patent Classification (IPC):
**C12Q 1/6844** (2018.01)

(52) Cooperative Patent Classification (CPC):
(C-Sets available)
**C12Q 1/6844** (Cont.)

(86) International application number:
**PCT/US2021/035311**

(87) International publication number:
**WO 2021/247618 (09.12.2021 Gazette 2021/49)**

(54) **ENRICHMENT OF NUCLEIC ACID SEQUENCES**

ANREICHERUNG VON NUKLEINSÄURESEQUENZEN

ENRICHISSEMENT DE SÉQUENCES D'ACIDES NUCLÉIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.06.2020 US 202063033787 P**
**13.07.2020 US 202063051257 P**

(43) Date of publication of application:
**05.04.2023 Bulletin 2023/14**

(73) Proprietor: **10X Genomics, Inc.**
**Pleasanton, CA 94588 (US)**

(72) Inventors:
• **MCDONNELL, Wyatt James**
**Pleasanton, California 94588 (US)**
• **PFEIFFER, Katherine**
**Pleasanton, California 94588 (US)**
• **RAMENANI, Ravi**
**Pleasanton, California 94588 (US)**
• **STUBBINGTON, Michael**
**Counterslip, Bristol**
**BS1 6BX (GB)**

(74) Representative: **J A Kemp LLP**
**80 Turnmill Street**
**London EC1M 5QU (GB)**

(56) References cited:
WO-A1-2018/075693    WO-A1-2019/010486
WO-A1-2019/084055    US-A1- 2018 105 808
US-A1- 2020 002 764

(52) Cooperative Patent Classification (CPC): (Cont.)

C-Sets
**C12Q 1/6844, C12Q 2525/149, C12Q 2525/155,
C12Q 2531/113**

**Description**

**BACKGROUND**

A sample may be processed for various purposes, such as identification of a type of moiety within the sample. The sample may be a biological sample. Biological samples may be processed, such as for detection of a disease (e.g., cancer) or identification of a particular species. There are various approaches for processing samples, such as polymerase chain reaction (PCR) and sequencing.

Biological samples may be processed within various reaction environments, such as partitions. Partitions may be wells or droplets. Droplets or wells may be employed to process biological samples in a manner that enables the biological samples to be partitioned and processed separately. For example, such droplets may be fluidically isolated from other droplets, enabling accurate control of respective environments in the droplets.

Biological samples in partitions may be subjected to various processes, such as chemical processes or physical processes. Samples in partitions may be subjected to heating or cooling, or chemical reactions, such as to yield species that may be qualitatively or quantitatively processed. Particular species yielded from the biological samples, e.g., nucleic acids, may be processed in selection and enrichment reactions to more efficiently recover nucleic acid sequences of interest.

WO 2018/075693 and US 2018/105808 disclose methods and compositions for analyzing individual cells or cell populations through the partitioned analysis of contents of individual cells or cell populations.

**SUMMARY OF THE INVENTION**

The present invention, as defined in the claims, provides a method for enriching for a nucleic acid sequence of interest, comprising: (a) providing a plurality of nucleic acid molecules comprising a plurality of identification sequences, wherein a nucleic acid molecule of said plurality of nucleic acid molecules comprises: (i) an identification sequence of said plurality of identification sequences that identifies said nucleic acid molecule, wherein the identification sequence comprises a barcode sequence, and/or a unique molecule identifier (UMI) sequence; and (ii) the nucleic acid sequence of interest, wherein the nucleic acid sequence of interest comprises a nucleic acid sequence coding for a T cell receptor (TCR), a B cell receptor (BCR), or a fragment thereof; (b) using a first nucleic acid primer complementary to at least a portion of the identification sequence in a first round of amplification to amplify said nucleic acid sequence of interest; and (c) using a second nucleic acid primer complementary to at least a portion of a V(D)J sequence in a second round of amplification to further amplify said nucleic acid sequence of interest.

In some embodiments, the identification sequence comprises the barcode sequence. In some embodiments, the identification sequence comprises the UMI sequence.

In some embodiments, the first round of amplification further comprises using a third primer comprising a sequence complementary to at least a portion of a complement of a nucleic acid sequence coding for a constant region of the TCR, BCR, or fragment thereof, optionally a sequence complementary to at least a portion of a complement of a nucleic acid sequence coding for a constant region of the BCR, or fragment thereof.

In some embodiments, the second round of amplification further comprises using a fourth primer comprising a sequence complementary to at least a portion of a complement of a nucleic acid sequence coding for a sequence downstream of the V(D)J sequence, optionally wherein the fourth primer comprises a non-binding handle. In some embodiments, the second primer comprises a non-binding handle.

In some embodiments, the plurality of provided nucleic acid molecules comprise complementary deoxyribonucleic acid (cDNA) molecules.

In some embodiments, the nucleic acid sequence of interest codes for the BCR, or fragment thereof.

The present invention also provides a method for enriching a nucleic acid sequence of interest, comprising: (a) providing a plurality of nucleic acid molecules comprising a plurality of identification sequences, wherein a nucleic acid molecule of said plurality of nucleic acid molecules comprises: (i) identification sequence of said plurality of identification sequences that identifies said nucleic acid molecule, wherein the identification sequence comprises a barcode sequence and a unique molecule identifier (UMI) sequence; and (ii) the nucleic acid sequence of interest, wherein the nucleic acid sequence of interest comprises a nucleic acid sequence encoding a B cell receptor (BCR) or a fragment thereof; (b) performing a first amplification reaction with a first set of primers comprising: a first primer comprising a sequence complementary to at least a portion of the barcode sequence and/or the UMI sequence, and a second primer comprising a sequence complementary to a complement of at least a portion of the nucleic acid sequence of interest that encodes a junction (J) region and/or isotype region of the BCR or fragment thereof; (c) performing a second amplification reaction with a second set of primers comprising: a third primer comprising a sequence complementary to nucleotides of at least a portion of the leader sequence and/or encoding framework region (FWR)1 of the BCR, or fragment thereof; and a fourth primer comprising a sequence complementary to a complement of at least a portion of the nucleic acid sequence of interest that encodes a complementarity region (CDR)3, a FWR4, a J region, a D region, and/or a V region, or a junction between any one or more thereof, of the BCR or fragment thereof.

In some embodiments, the first primer comprises: (i) a sequence complementary to at least a portion of the barcode sequence and the UMI sequence; or (ii) a sequence complementary to the barcode sequence and the UMI sequence. In some embodiments, the second primer comprises: (i) a sequence complementary to the complement of the nucleic acid sequence of interest that encodes at least a portion of the J region of the BCR or fragment thereof; (ii) a sequence complementary to the complement of the nucleic acid sequence of interest that encodes at least a portion of the isotype region of the BCR or fragment thereof; or (iii) a sequence complementary to the complement of the nucleic acid sequence of interest that encodes at least a portion of the J region and the isotype region of the BCR or fragment thereof. In some embodiments, the third primer comprises: (i) a sequence complementary to nucleotides of at least a portion of the leader sequence of the BCR, or fragment thereof; or (ii) a sequence complementary to nucleotides encoding at least a portion of the FWR1 of the BCR, or fragment thereof. In some embodiments, the fourth primer comprises: (i) a sequence complementary to the complement of at least a portion of the nucleic acid sequence of interest that encodes the CDR3, and junction extending into the J region of the BCR or fragment thereof; (ii) a sequence complementary to the complement of at least a portion of the nucleic acid sequence of interest that encodes the D and J regions, or a junction between the D and J regions of the BCR or fragment thereof; (iii) a sequence complementary to the complement of at least a portion of the nucleic acid sequence of interest that encodes the V and J regions, or a junction between the V and J regions, of the BCR or fragment thereof; (iv) a sequence complementary to the complement of at least a portion of the nucleic acid sequence of interest that encodes the V and D regions, or a junction between the V and D regions, of the BCR or fragment thereof; or (v) a sequence complementary to the complement of at least a portion of the nucleic acid sequence of interest that encodes the V, D and J regions of the BCR or fragment thereof. In some embodiments, the third and the fourth primer comprise a non-binding handle. In some embodiments, the method further comprising cloning the enriched nucleic acid sequence of interest in a vector.

In some embodiments, the plurality of nucleic acid molecules are prepared from a cell sample of a donor or donors, optionally wherein: (i) the donor or donors had been exposed to a target antigen and wherein the plurality of nucleic acid molecules that comprise the nucleic acid sequence of interest comprise a nucleic acid sequence encoding a selected B cell receptor (BCR) or a fragment thereof that binds the target antigen; or (ii) the plurality of nucleic acid molecules comprising the nucleic acid sequence encoding the selected B cell receptor (BCR), or fragment thereof, that binds the target antigen are prepared from the cell sample of the donor or donors comprising steps of: (a) partitioning a reaction mixture into a plurality of partitions, wherein the reaction mixture comprises: (i) a plurality of cells of the cell sample, and (ii) the target antigen, wherein the target antigen is coupled to a reporter oligonucleotide, wherein the reaction mixture comprises a cell bound to the target antigen, wherein the partitioning provides a partition comprising: (i) the partitioned cell bound to the target antigen, and (ii) a plurality of nucleic acid barcode molecules comprising a partition-specific barcode sequence, (b) in the partition, generating barcoded nucleic acid molecules, wherein the barcoded nucleic acid molecules comprise: (i) a first barcoded nucleic acid molecule comprising a sequence of the reporter oligonucleotide or a reverse complement thereof and the partition-specific barcode sequence or reverse complement thereof, and (ii) a second barcoded nucleic acid molecule comprising a nucleic acid molecule of interest for inclusion in the plurality of nucleic acid molecules if the first barcoded nucleic molecule is detected.

## SUMMARY OF THE TECHNICAL TEACHINGS

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

Provided herein are methods for the enriching a nucleic acid sequence of interest from a plurality of nucleic acid molecules, such as a library of nucleic acid molecules. The methods provided herein can be used, for example, to enrich a nucleic acid sequence of interest so that it may be further cloned or analyzed. Methods herein can provide a low noise, high specificity, or both. In some instances, methods herein can be useful for selection of a nucleic acid sequence of interest (for example, a candidate antibody) or antibody discovery applications.

Provided herein are methods for enriching for a nucleic acid sequence of interest. In the methods, a plurality of nucleic acid molecules having a plurality of identification sequences is provided. A nucleic acid molecule of said plurality of nucleic acid molecules includes: (i) an identification sequence of said plurality of identification sequences which identifies the nucleic acid molecule, and which includes a barcode sequence, a template switch oligonucleotide (TSO) sequence, and/or a unique molecule identifier (UMI) sequence; and (ii) the nucleic acid sequence of interest, wherein the nucleic acid sequence of interest includes a nucleic acid sequence coding for a T cell receptor (TCR), a B cell receptor (BCR), or a fragment thereof. A first nucleic acid primer complementary to at least a portion of the identification sequence is used in a first round of amplification to amplify the nucleic acid sequence of interest. A second nucleic acid primer complementary to at least a portion of a V(D)J sequence is used in a second round of amplification to further amplify the nucleic acid sequence.

Provided herein are methods for enriching for a nucleic acid sequence of interest, comprising: (a) providing a plurality of nucleic acid molecules comprising a plurality of identification sequences, wherein a nucleic acid molecule of said plurality of nucleic acid molecules comprises an (i) identification sequence of said plurality of identification sequences that identifies said nucleic acid molecule and (ii) said nucleic acid sequence of interest; and (b) using a nucleic acid primer complementary to said identification sequence to amplify said nucleic acid sequence of interest, thereby enriching for said nucleic acid sequence of interest.

In some instances, said providing comprises generating said plurality of nucleic acid molecules comprising said plurality of identification sequences that identify said plurality of nucleic acid molecules

In some instances, said plurality of nucleic acid molecules corresponds to a plurality of cell surface proteins from said plurality of cells. In some instances, said plurality of cells comprises a plurality of T cells. In some instances, said plurality of cell surface proteins comprises a plurality of T cell receptors. In some instances, said plurality of cells comprises a plurality of B cells. In some instances, said plurality of cell surface proteins comprises a plurality of B cell receptors.

In some instances, said plurality of nucleic acid molecules comprises a library of nucleic acid molecules coding for a plurality of variants of an amino acid sequence. In some instances, said plurality of variants of said amino acid sequence are variants of a T cell receptor. In some instances, said plurality of variants of said amino acid sequence are variants of an antibody or antigen binding fragment thereof. In some instances, said plurality of nucleic acid molecules comprises complementary deoxyribonucleic acid (cDNA) molecules. In some instances, said nucleic acid sequence of interest comprises a nucleic acid sequence coding for a T cell receptor or fragment thereof. In some instances, said nucleic acid sequence of interest comprises a nucleic acid sequence coding for an antibody or antigen binding fragment thereof. In some instances, said nucleic acid sequence of interest comprises a nucleic acid sequence coding for a V(D)J sequence.

In some instances, said identification sequence comprises a barcode sequence. In some instances, said nucleic acid sequence that is complementary to said identification sequence is complementary to at least a portion of said barcode sequence. In some instances, said nucleic acid sequence that is complementary to said identification sequence is complementary to said barcode sequence and a read sequence of said nucleic acid sequence of interest. In some instances, said identification sequence comprises a template switch oligonucleotide sequence. In some instances, said nucleic acid sequence that is complementary to said identification sequence is complementary to at least a portion of said template switch oligonucleotide sequence. In some instances, said identification sequence comprises a unique molecular identifier sequence. In some instances, said nucleic acid sequence that is complementary to said identification sequence is complementary to at least a portion of said unique molecular identifier sequence. In some instances, said nucleic acid primer further comprises a nucleic acid sequence that is complementary to a portion of said coding sequence. In some instances, said nucleic acid primer further comprises a nucleic acid sequence that is complementary to at least a portion of said V(D)J sequence. In some instances, said nucleic acid sequence that is complementary to at least a portion of said V(D)J sequence is complementary to a V sequence of said V(D)J sequence. In some instances, said nucleic acid primer further comprises a non-binding handle.

Some instances further comprise using another nucleic acid primer to amplify said nucleic acid sequence of interest, wherein said another nucleic acid primer is different from said nucleic acid primer. In some instances, said another nucleic acid primer comprises a non-binding handle. In some instances, said nucleic acid primer and said another nucleic acid primer are configured to anneal to sequence flanking at least a portion of said nucleic acid sequence of interest.

Some instances further comprise a polymerase chain reaction, thereby further enriching said nucleic acid sequence of said plurality of nucleic acid molecules.

In some instances, others of said plurality of nucleic acid molecules are not amplified. In some instances, said nucleic acid sequence of interest is enriched by a factor of at least 1000 at least 10,000, or at least 100,000.

Some instances further comprise contacting said plurality of nucleic acid sequences with a second nucleic acid primer, wherein said nucleic acid primer comprises a nucleic acid sequence that is complementary to a binding sequence on a complement of said nucleic acid sequence of interest. In some instances, said second nucleic acid primer further comprises a non-binding handle. In some instances, said nucleic acid sequence of interest comprises a sequence coding for an antibody or antigen binding fragment thereof. In some instances, said second nucleic acid primer is complementary to at least a portion of a complement of a nucleic acid sequence coding for V(D)J sequence of said antibody or antigen binding fragment thereof. In some instances, said second nucleic acid primer is complementary to at least a portion of a complement of a nucleic acid sequence coding for a constant region of said antibody or antigen binding fragment thereof. In some instances, said second nucleic acid primer is further complementary to at least a portion of a complement of a nucleic acid sequence coding for a J region of said antibody or antigen binding fragment thereof.

Some instances further comprise cloning said nucleic acid sequence of interest into a vector. In some instances, said vector is selected from the group consisting of a viral vector, a plasmid, a bacteriophage, a cosmid, and an artificial chromosome. In some instances, said cloning comprises combining two or more nucleic acid sequences. In some instances, said two or more nucleic acid sequences comprises a nucleic acid sequence of a heavy chain of an antibody or antigen binding fragment and a nucleic acid sequence of a light chain. In some instances, said two or more nucleic acid sequences comprises a nucleic acid sequence of an alpha chain of a T cell receptor and a nucleic acid sequence of a beta chain of a T cell receptor. In some instances, said vector comprises at least a portion of a constant region of a T cell receptor. In some instances, said vector comprises at least a portion of a constant region of an antibody or antigen binding fragment thereof. In some instances, said vector comprises a promoter.

Some instances further comprise determining an enrichment level of said nucleic acid sequence of interest. Some instances further comprise performing a second round of amplification to further enrich said nucleic acid sequence of interest. Some such instances comprise contacting said nucleic acid sequence of interest with a third primer and a fourth primer. In some instances, said third primer is complementary to a portion of a barcode of said identification sequence. In some instances, said third primer is complementary to a 5' end of said barcode of said identification sequence. In some instances, said third primer is further complementary to a read sequence. In some instances, said third primer is complementary to a portion of said identification sequence upstream of a barcode of said identification sequence. In some instances, said fourth primer is complementary to at least a portion of the complement of a constant region of said nucleic acid sequence of interest.

Some instances further comprise performing fragmentation of said nucleic acid sequence of interest. Some instances further comprise A-tailing said nucleic acid sequence of interest. Some instances further comprise performing SI-PCR on said nucleic acid sequence of interest. Some instances further comprise V(D)J enrichment of said nucleic acid sequence of interest. In some instances, said nucleic acid sequence of interest comprises a restriction site.

Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Also provided herein are methods comprising: enriching a nucleic acid sequence of interest based at least on at least a portion of a constant region of said nucleic acid sequence of interest yielding an enriched nucleic acid sequence of interest; and modification of said enriched nucleic acid sequence yielding a modified enriched nucleic acid sequence compatible with a vector.

In some instances, said enriching is performed using a first nucleic acid primer and a second nucleic acid primer. In some instances, one of said first nucleic acid primer and said second nucleic acid primer is a framework leader (FWR1) primer. In some instances, said first nucleic acid primer is complementary at least to a barcode or portion thereof on said nucleic acid sequence of interest. In some instances, said first nucleic acid primer is complementary at least to a unique molecular identification sequence or a portion thereof on said nucleic acid sequence of interest. In some instances, said first nucleic acid primer is complementary at least to a 5' untranslated region (5' UTR) or a portion thereof on said nucleic acid sequence of interest. In some instances, said second nucleic acid primer is complementary at least to a constant region or portion thereof on said nucleic acid sequence of interest. In some instances, said second nucleic acid primer is complementary at least to a J sequence or portion thereof on said nucleic acid sequence of interest. In some instances, said second nucleic acid primer is complementary at least to a nucleic acid sequence of a junction region or portion thereof on said nucleic acid sequence of interest. In some instances, said enriching is performed using hybridization capture. In some instances, said hybridization capture is based on hybridization of a primer to a junction sequence on said nucleic acid sequence of interest. In some instances, said junction sequence is a V(D)J sequence or a portion thereof. In some instances, said nucleic acid primer is selected based on Rapid Amplification of cDNA Ends (RACE) sequencing. In some instances, said nucleic acid sequence of interest comprises complementary deoxyribonucleic acid (cDNA) molecules. In some instances, said nucleic acid sequence of interest further comprises a barcode. In some instances, said nucleic acid sequence of interest codes for a at least a portion of a cell surface protein of a cell.

In some instances, said cell surface protein is a T cell receptor or a fragment thereof. In some instances, said cell surface protein is a B cell receptor or a fragment thereof. In some instances, said cell is a T cell. In some instances, said cell is a B cell.

In some instances, said constant region of nucleic acid sequence of interest comprises a nucleic acid sequence coding for a V(D)J sequence or a portion thereof. In some instances, said portion is a V sequence. In some instances, said portion is a J sequence.

In some instances, said modification comprises addition of Gibson ends to said amplified nucleic acid sequences. In some instances, said addition of said Gibson ends is performed using a polymerase chain reaction (PCR). In some instances, said modification comprises combining a second nucleic acid of interest with said enriched nucleic acid of interest. In some instances, said combining comprises using overlap extension primers to link said enriched nucleic acid sequence of interest to said second nucleic acid sequence of interest. In some instances, said combining comprises using a nucleic acid linker to join said second nucleic acid sequence of interest to said enriched nucleic acid sequence of interest. In some instances, said second nucleic acid sequence of interest is enriched. In some instances, said second nucleic acid sequence of interest codes for at least a portion of a cell surface protein of a cell. In some instances, said cell surface protein is a T cell receptor or a fragment thereof. In some instances, said cell surface protein is a B cell receptor or a fragment thereof. In some instances, said cell is a T cell.

Some instances further comprise cloning said modified enriched nucleic acid sequence into said vector. In some instances, said cloning comprises a vector restriction digest. In some instances, said vector restriction digest comprises digestion at an fspI restriction site. In some instances, said vector comprises a native leader sequence.

Also provided herein is a further method for enriching a nucleic sequence of interest. In the method, a plurality of nucleic acid molecules is provided. The plurality of nucleic acid molecules includes a plurality of identification sequences. A nucleic acid molecule of the plurality of nucleic acid molecules includes: (i) an identification sequence of the plurality of identification sequences, which identifies said nucleic acid molecule, and which includes a barcode sequence and a unique molecule identifier (UMI) sequence; and (ii) the nucleic acid sequence of interest, wherein the nucleic acid sequence of interest includes a nucleic acid sequence encoding a B cell receptor (BCR) or a fragment thereof. A first amplification reaction is performed with a first set of primers. The first set of primers includes a first primer having a sequence complementary to at least a portion of the barcode sequence and/or the UMI sequence, and a second primer having a sequence complementary to a complement of at least a portion of the nucleic acid sequence of interest that encodes a junction (J) region and/or isotype region of the BCR or fragment thereof. A second amplification reaction is performed with a second set of primers. The second set of primers includes a third primer having a sequence complementary to nucleotides of at least a portion of the leader sequence and/or encoding framework region (FWR)1 of the BCR, or fragment thereof ; and a fourth primer having a sequence complementary to a complement of at least a portion of the nucleic acid sequence of interest that encodes a complementarity region (CDR)3, a FWR4, a J region, a D region, and/or a V region, or a junction between any one or more thereof, of the BCR or fragment thereof.

In any of the aspects or instances of the methods provided herein, the plurality of nucleic acid molecules may have been prepared from a cell sample of a donor or donors. In some of these instances, the donor or donors may have been exposed to a target antigen and the plurality of nucleic acid molecules that include the nucleic acid sequence of interest may have a nucleic acid sequence encoding a selected B cell receptor (BCR) or a fragment thereof that binds the target antigen. Furthermore, in aspects of these instances of the methods, the plurality of nucleic acid molecules comprising the nucleic acid sequence encoding the selected B cell receptor (BCR), or fragment thereof, (that binds the target antigen) may be prepared from the cell sample of the donor or donors according to steps that may include a first step of partitioning a reaction mixture into a plurality of partitions, in which the reaction mixture comprises (i) a plurality of cells of the cell sample, and (ii) the target antigen, which may be coupled to a reporter oligonucleotide. The reaction mixture includes a cell bound to the target antigen. The first step of partitioning provides a partition that includes: (i) the partitioned cell bound to the target antigen, and (ii) a plurality of nucleic acid barcode molecules comprising a partition-specific barcode sequence. The second step of preparing from the cell sample of the donor or donors may be to generate, in the partition, barcoded nucleic acid molecules. The barcoded nucleic acid molecules may include first and second barcoded nucleic acid molecules. The first barcoded nucleic acid molecule may include a sequence of the reporter oligonucleotide or a reverse complement thereof and the partition-specific barcode sequence or reverse complement thereof. The second barcoded nucleic acid molecules may include a nucleic acid molecule of interest for inclusion in the plurality of nucleic acid molecules if the first barcoded nucleic molecule is detected.

Additional aspects and advantages of the present disclosure will become readily apparent to those skilled in this art from the following detailed description, wherein only illustrative instances of the present disclosure are shown and described. As will be realized, the present disclosure is capable of other and different instances, and its several details are capable of modifications in various obvious respects, all without departing from the disclosure. Accordingly, the drawings and description are to be regarded as illustrative in nature, and not as restrictive.

To the extent publications and patents or patent applications referenced contradict the disclosure contained in the specification, the specification is intended to supersede and/or take precedence over any such contradictory material.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

FIG. 1 shows an example of a microfluidic channel structure for partitioning individual biological particles.
FIG. 2 shows an example of a microfluidic channel structure for delivering barcode carrying beads to droplets.
FIG. 3 shows an example of a microfluidic channel structure for co-partitioning biological particles and reagents.
FIG. 4 shows an example of a microfluidic channel structure for the controlled partitioning of beads into discrete droplets.

**FIG.** 5 shows an example of a microfluidic channel structure for increased droplet generation throughput.

**FIG.** 6 shows another example of a microfluidic channel structure for increased droplet generation throughput.

**FIG.** 7A shows a cross-section view of another example of a microfluidic channel structure with a geometric feature for controlled partitioning. FIG. 7B shows a perspective view of the channel structure of FIG. 7A.

**FIG.** 8 illustrates an example of a barcode carrying bead.

**FIG.** 9 illustrates a workflow for the enrichment of a nucleic acid sequence of interest.

**FIG. 10** illustrates a nested PCR scheme for amplification of a nucleic acid sequence of interest.

FIG. 11 shows exemplary labelling agents comprising reporter oligonucleotides attached thereto.

FIG. 12A shows a workflow for the analysis of one or more analytes.

FIG. 12B-C show processing of nucleic acid molecules derived from a cell to append a barcode sequence.

FIG. 13A-C show a workflow for the analysis of multiple analytes using labelling agents.

FIG. 14 shows a computer system that is programmed or otherwise configured to implement methods provided herein.

FIG. 15 shows exemplary labelling agents comprising reporter oligonucleotides attached

FIG. 16 illustrates an example of primer design configured to yield a clonable sequence from a nucleic acid sequence of interest using enrichment methods provided herein.

FIG. 17 provides a pictorial outline for a method of enriching a nucleic acid sequence of interest.

FIG. 18 provides a pictorial outline of a nucleic acid sequence that is compatible with a vector, including incorporation of the nucleic acid sequence into the vector.

FIG. 19 provides a pictorial outline of an exemplary probe and scheme for capture-based enrichment of nucleic acid sequences of interest.

FIG. 20 shows products of a nested (FIG. 20A) versus one-step (FIG. 20B) PCR amplification reaction to enrich for a target nucleic acid sequence of interest, e.g., encoding a fragment of a BCR.

**FIG. 21** shows BioA results indicating that nested PCR cleanly amplifies a target product of interest, e.g., nucleic acid sequence encoding a fragment of a BCR, for three out of four cell clones from a pooled barcoded cDNA library. **(FIG. 21B-D;** clone B, C, and D). A nested PCR amplification targeting a fourth cell clone yielded multiple products **(FIG. 21A;** clone A).

**FIG. 22** shows sequencing results of the enrichment products following nested amplification for a nucleic acid sequence of interest from a pooled barcoded cDNA library, e.g., a target nucleic acid sequence encoding a fragment of a BCR produced from Clone A (an expanded clonotype with multiple subclonotypes), when the forward outer primer lacked sufficient specificity.

**FIG. 23** shows sequencing results of the enrichment products following nested amplification for a nucleic acid sequence of interest from a pooled barcoded cDNA library, e.g., a target nucleic acid sequence encoding a fragment of a BCR produced from Clone C (a single cell clone with many valid UMIs), when the forward outer primer lacked sufficient specificity.

**FIG. 24** shows sequencing results of the enrichment products following nested amplification for a nucleic acid sequence of interest from a pooled barcoded cDNA library, e.g., a target nucleic acid sequence encoding a fragment of a BCR produced from Clone B (an expanded clonotype with a single unique subclone), when the forward outer primer bound with sufficient specificity to the cell barcode and UMI.

## DETAILED DESCRIPTION

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

While various embodiments of the invention have been shown and described herein, it will be obvious to those skilled in the art that such embodiments are provided by way of example

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The terms "a," "an," and "the," as used herein, generally refers to singular and plural references unless the context clearly dictates otherwise. "A and/or B" is used herein to include all of the following alternatives: "A", "B", "A or B", and "A and B".

Headings, e.g., (a), (b), (i) etc., are presented merely for ease of reading the specification and claims. The use of headings in the specification or claims does not require the steps or elements be performed in alphabetical or numerical order or the order in which they are presented.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, the use of these terms in the specification does not by itself connote any required priority, precedence, or order.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode may be unique. Barcodes can have a variety of different formats. For example, barcodes can include: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

The term "real time," as used herein, can refer to a response time of less than about 1 second, a tenth of a second, a hundredth of a second, a millisecond, or less. The response time may be greater than 1 second. In some instances, real time can refer to simultaneous or substantially simultaneous processing, detection or identification.

The term "subject," as used herein, generally refers to an animal, such as a mammal (e.g., human) or avian (e.g., bird), or other organism, such as a plant. For example, the subject can be a vertebrate, a mammal, a rodent (e.g., a mouse), a primate, a simian or a human. Animals may include, but are not limited to, farm animals, sport animals, and pets. A subject can be a healthy or asymptomatic individual, an individual that has or is suspected of having a disease (e.g., cancer) or a pre-disposition to the disease, and/or an individual that is in need of therapy or suspected of needing therapy. A subject can be a patient. A subject can be a microorganism or microbe (e.g., bacteria, fungi, archaea, viruses).

The term "genome," as used herein, generally refers to genomic information from a subject, which may be, for example, at least a portion or an entirety of a subject's hereditary information. A genome can be encoded either in DNA or in RNA. A genome can comprise coding regions (e.g., that code for proteins) as well as non-coding regions. A genome can include the sequence of all chromosomes together in an organism. For example, the human genome ordinarily has a total of 46 chromosomes. The sequence of all of these together may constitute a human genome.

The terms "adaptor(s)", "adapter(s)" and "tag(s)" may be used synonymously. An adaptor or tag can be coupled to a polynucleotide sequence to be "tagged" by any approach, including ligation, hybridization, or other approaches.

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina®, Pacific Biosciences (PacBio®), Oxford Nanopore®, or Life Technologies (Ion Torrent®). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. In some situations, systems and methods provided herein may be used with proteomic information.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or non-magnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

The term "sample," as used herein, generally refers to a biological sample of a subject. The biological sample may comprise any number of macromolecules, for example, cellular macromolecules. The sample may be a cell sample. The sample may be a cell line or cell culture sample. The sample can include one or more cells. The sample can include one or more microbes. The biological sample may be a nucleic acid sample or protein sample. The biological sample may also be a carbohydrate sample or a lipid sample. The biological sample may be derived from another sample. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swab. The sample may be a plasma or serum sample. The sample may be a cell-free or cell free sample. A cell-free sample may include extracellular polynucleotides. Extracellular polynucleotides may be isolated from a bodily sample that may be selected from the group consisting of blood, plasma, serum, urine, saliva, mucosal excretions, sputum, stool and tears.

The term "biological particle," as used herein, generally refers to a discrete biological system derived from a biological sample. The biological particle may be a macromolecule. The biological particle may be a small molecule. The biological particle may be a virus. The biological particle may be a cell or derivative of a cell. The biological particle may be an organelle. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle may be a constituent of a cell. The biological particle may be or may include DNA, RNA, organelles, proteins, or any combination thereof. The biological particle may be or may include a matrix (e.g., a gel or polymer matrix) comprising a cell or one or more constituents from a cell (e.g., cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when comprising a gel or polymer matrix.

The term "macromolecular constituent," as used herein, generally refers to a macromolecule contained within or from a biological particle. The macromolecular constituent may comprise a nucleic acid. In some cases, the biological particle may be a macromolecule. The macromolecular constituent may comprise DNA. The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

The term "partition," as used herein, generally, refers to a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments.

### Enriching a sequence of interest from a nucleic acid molecule library

Provided herein are methods for the enriching a nucleic acid sequence of interest from a plurality of nucleic acid molecules, such as a library of nucleic acid molecules. The methods provided herein can be used, for example, to enrich a nucleic acid sequence of interest so that it may be further cloned or analyzed. Methods herein can provide a low noise, high specificity, or both. In some embodiments, methods herein can be useful for selection of a nucleic acid sequence of interest (for example, a candidate antibody) or antibody discovery applications.

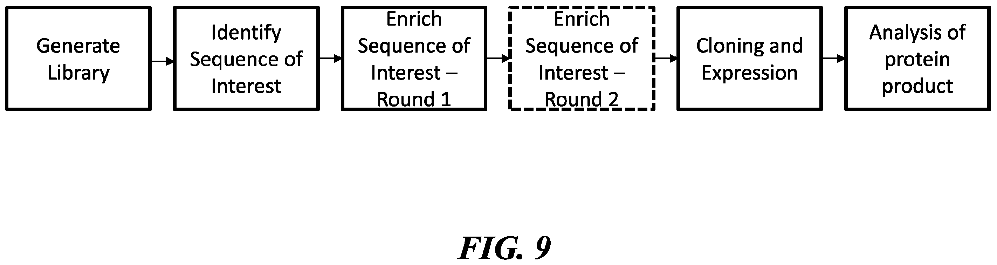

A general workflow of methods provided herein is provided in FIG. 9. Briefly, (1) a library can be generated (e.g., a barcoded library of sequences of a single cell immune repertoire of a subject); (2) sequences of interest can be identified (e.g., a V(D)J sequence, such as paired TCR (e.g., TRA/TRB), BCR, or antibody (e.g., heavy/light chain) sequences), for example by sequencing; (3) the sequence(s) of interest can be enriched from the library (e.g., by using 1 or 2 rounds of PCR); (4) cloning (and optionally expressing) the enriched sequence(s) of interest into an appropriate expression vector; and (5) optionally analyzing the protein (e.g., antibody) encoded by the sequence of interest.

**Methods**

Libraries of nucleic acid molecules that have nucleic acid sequences, e.g., nucleic acid sequences encoding proteins, such as paired T cell receptors (TCRs), B cell receptors (BCRs), and antibodies or antigen binding fragments thereof, can be employed to provide an enriched nucleic acid sequence of interest of the nucleic acid sequences, e.g., encoding an amino acid sequence of interest (e.g., a specific T cell receptor, B cell receptor, or antibody or antigen binding fragment thereof). The library can be generated, for example, by isolating and/or amplifying RNA encoding the amino acid sequence of interest, or using DNA, e.g., genomic DNA. The RNA library can be reverse transcribed to yield a cDNA library, and identification sequences (e.g., barcode sequence or unique molecular identification sequences) can be appended to members of the library and can be used to identify members of the library.

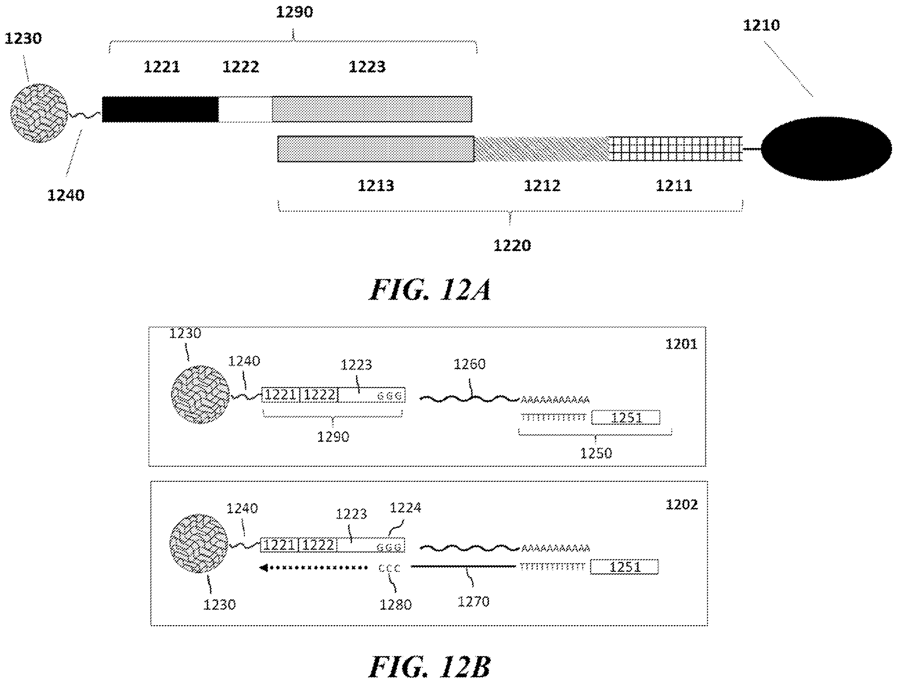

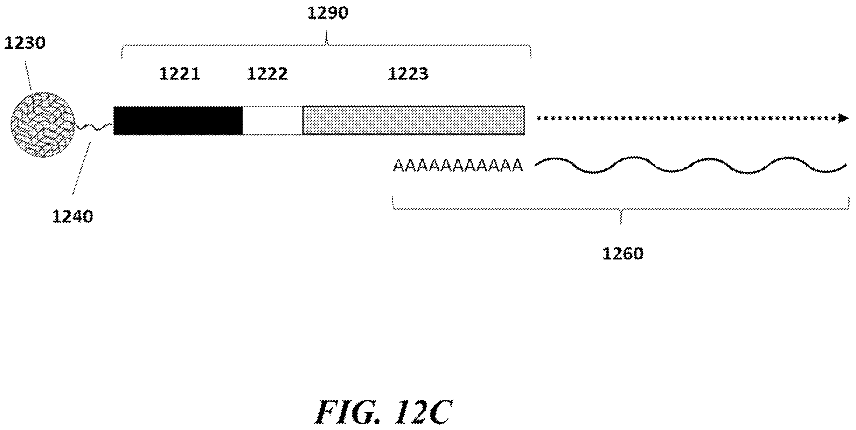

In some instances, a barcoded nucleic acid library comprising immune molecules (e.g., from single cells) is generated as described herein. For example, in some embodiments, RNA molecules are processed as generally described in FIGS. 12B-C. Referring to FIG. 12B, in some instances, nucleic acid molecules derived from a cell (such as RNA molecules) are processed to append a cell (e.g., partition) specific barcode sequence 1222 to these molecules or derivatives thereof (e.g., cDNA molecules). For example, referring to FIG. 12B, in some embodiments, primer 1250 comprises a sequence complementary to a sequence of RNA molecule 1260 from a cell (such as an RNA encoding for an immune molecule, such as a light or heavy chain antibody sequence). In some instances, primer 1250 comprises one or more adapter sequences 1251 that are not complementary to RNA molecule 1260. In some instances, primer 1250 comprises a poly-T sequence. In some instances, primer 1250 comprises a sequence complementary to a target sequence in an RNA molecule. In some instances, primer 1250 comprises a sequence complementary to a region of an immune molecule, such as the constant region of an RNA encoding a TCR, BCR, or antibody molecule. Primer 1250 is hybridized to RNA molecule 1260 and cDNA molecule 1270 is generated in a reverse transcription reaction. In some instances, the reverse transcriptase enzyme is selected such that several non-templated bases **1280** (e.g., a poly-C sequence) are appended to the cDNA. Nucleic acid barcode molecule **1290** comprises a sequence **1224** complementary to the non-templated bases, and the reverse transcriptase performs a template switching reaction onto nucleic acid barcode molecule **1290** to generate a barcoded nucleic acid molecule comprising cell (e.g., partition specific) barcode sequence **1222** (or a reverse complement thereof) and a sequence of cDNA 1270 (or a portion thereof). In another example, referring to **FIG. 12C,** in some embodiments, nucleic acid barcode molecule **1290** comprises sequence **1223** complementary to a sequence of RNA molecule **1260** from a cell. In some instances, sequence **1223** comprises a sequence specific for an RNA molecule. In some instances, sequence **1223** comprises a poly-T sequence. In some instances, sequence **1223** comprises a sequence specific for an RNA molecule. In some instances, sequence **1223** comprises a sequence complementary to a region of an immune molecule, such as the constant region of an RNA encoding a TCR, BCR, or antibody molecule. Sequence **1223** is hybridized to RNA molecule **1260** and a cDNA molecule 1270 is generated in a reverse transcription reaction generating a barcoded nucleic acid molecule comprising cell (e.g., partition specific) barcode sequence **1222** (or a reverse complement thereof) and a sequence of cDNA 1270 (or a portion thereof). Barcoded nucleic acid molecules can then be optionally processed as described elsewhere herein, e.g., to amplify the molecules and/or append sequencing platform specific sequences to the fragments. See, e.g., U.S. Pat. Pub. 20180105808. Barcoded nucleic acid molecules, or derivatives generated therefrom, can then be sequenced on a suitable sequencing platform. In some instances, one or more labelling agents capable of binding to or otherwise coupling to one or more cell features may be used to characterize cells and/or cell features as described herein (e.g., to characterize immune receptor or antigen specificity of immune molecules).

Molecules of the library can have the structure, from 5' to 3', of identification sequence to coding sequence. For example, molecules of the library can have the structure, from 5' to 3', of: (1) barcode sequence; (2) unique molecular identifier sequence; (3) template switch oligonucleotide sequence (4) immune molecule variable sequence (e.g., V(D)J sequence, as provided herein); and (5) immune receptor constant sequence. In some embodiments, one or more adapter sequences (such as sequencing platform specific sequences, such as a sequencing primer or primer binding sequence, e.g., an Illumina R1 or R2) can be located either 5' or 3' to the sequence of a molecule of a library, or both.

In some instances, a barcoded gene expression library is generated (e.g., from single cells as described herein) from a plurality of cells comprising an immune molecule, such as a TCR, BCR, or antibody. The barcoded library can then be sequenced and analyzed to identify paired immune molecule sequences from single cells (e.g., comprising a common barcode sequence), such as paired TCRs (e.g., TRA/TRB), paired BCRs (light/heavy chain sequences), and paired antibody sequences (light/heavy chain sequences). Immune molecules of interest (e.g., paired light/heavy chain antibodies) can then be directly enriched (e.g., amplified) from the barcoded library for subsequent processing and analysis in, e.g., an expression vector. In some instances, primers are designed to amplify paired immune molecules, e.g.,

light and heavy chain antibody sequences, from the library for cloning into one or more suitable expression vectors.

Enrichment of a nucleic acid sequence of interest from, e.g., a barcoded gene expression library, can allow expedited isolation of the nucleic acid, and the expression and/or analysis for the amino acid sequence which it encodes. For example, enrichment (e.g., using one or more PCR reactions) of sequences of interest (e.g., a V(D)J sequence, such as paired TCR (e.g., TRA/TRB), BCR, or antibody (e.g., heavy/light chain) sequences) and direct cloning of those enriched sequences (such as a light and heavy chain sequence of an antibody) into an appropriate expression vector can be utilized to avoid costly and time consuming methodologies (such as gene synthesis) employed to generate an expression vector configured to express immune molecules (e.g., antibodies) of interest. A nucleic acid sequence of interest can be enriched by amplifying the nucleic acid sequence of interest based on an identification sequence (e.g., barcode or UMI) associated with the nucleic acid sequence of interest, for example, by using a scheme such as is illustrated in FIG. 9. In some embodiments, a nested amplification approach can be employed to further enrich the nucleic acid sequence of interest.

As part of an enrichment protocol, a nucleic acid primer can be designed that anneals to one or more identification sequences in a molecule that harbors a nucleic acid sequence of interest, e.g., a barcode sequence or unique molecule identifier. Another primer can be designed to anneal to a sequence downstream of the identification sequence, and can be configured such that the nucleic acid sequence can be amplified using the primers, e.g., by polymerase chain reaction. A second round of amplification can be performed using a different set of primers to further enrich the nucleic acid sequence of interest.

After enriching a nucleic acid sequence of interest, it can be cloned into a vector and subsequently expressed in an expression system. Such cloning and expression can yield protein for analysis. For example, a candidate T cell receptor, B cell receptor, or antibody or antigen binding fragment thereof can be expressed in an expression system where such a nucleic acid sequence of interest is cloned. Such a protein can be a therapeutic candidate, a gene of interest, a protein variant of interest, or another protein to be analyzed. In some instances, primers are designed to amplify paired immune molecule sequences from single cells (e.g., comprising a common barcode sequence), such as paired TCRs (e.g., TRA/TRB), paired BCRs (light/heavy chain sequences), and paired antibody sequences (light/heavy chain sequences). These amplified, paired immune molecule sequences (e.g., paired light and heavy chain antibody sequences) can then be optionally processed for subsequent cloning into an expression vectors for expression of functional immune molecules (e.g., a plasmid configured to co-express paired immune molecule subunits, such as an antibody heavy and light chain).

*Nucleic acid molecules*

Methods provided herein can comprise providing a plurality of nucleic acid molecules. Nucleic acid molecules described herein can comprise ribonucleic acids (e.g., RNA, such as RNA molecules provided herein) or deoxyribonucleic acids (e.g., DNA or cDNA). A nucleic acid molecule can comprise G, A, T, U, C, or bases that are capable of base pairing reliably with a complementary nucleotide. 7-deaza-adenine, 7-deaza-guanine, adenine, guanine, cytosine, thymine, uracil, 2-deaza-2-thio-guanosine, 2-thio-7-deaza-guanosine, 2-thio- adenine, 2-thio- 7-deaza-adenine, isoguanine, 7-deaza-guanine, 5,6-dihydrouridine, 5,6- dihydrothymine, xanthine, 7-deaza-xanthine, hypoxanthine, 7-deaza-xanthine, 2,6 diamino-7- deaza purine, 5- methyl-cytosine, 5-propynyl-uridine, 5-propynyl-cytidine, 2-thio-thymine or 2-thio-uridine are examples of such bases, although many others are known A nucleic acid molecule can comprise an LNA, a PNA, a UNA, or an morpholino oligomer, for example. A nucleic acid molecule used herein may contain natural or non- natural nucleotides or linkages.

A nucleic acid molecule can comprise an identification sequence. An identification sequence can identify, for example, the nucleic acid molecule, the source of the nucleic acid sample, or another property of the nucleic acid sample. For example, the nucleic acid molecule can comprise one or more of: an adapter sequence, a primer or primer binding sequence, a sequencing primer or sequencing primer binding sequence (such as an R1 or partial R1 sequence), a unique molecular identifier (UMI), a polynucleotide sequence (such as a poly-A or poly-C sequence), or a sequence configured to bind to the flow cell of a sequencer (such as a P5 or P7, or partial sequences thereof). It is to be understood that the nucleic acid molecule can further comprise a cell barcode sequence, e.g., a partition-specific barcode.

In some embodiments, a nucleic acid molecule of a plurality of nucleic acid molecules can comprise two or more of a barcode, a unique molecular identification sequence, and a template switch oligonucleotide sequence. For example, a nucleic acid molecule can comprise a barcode and a unique molecular identification sequence, a barcode and a template switch oligonucleotide sequence, or a unique molecular identification sequence and a template switch oligonucleotide sequence.

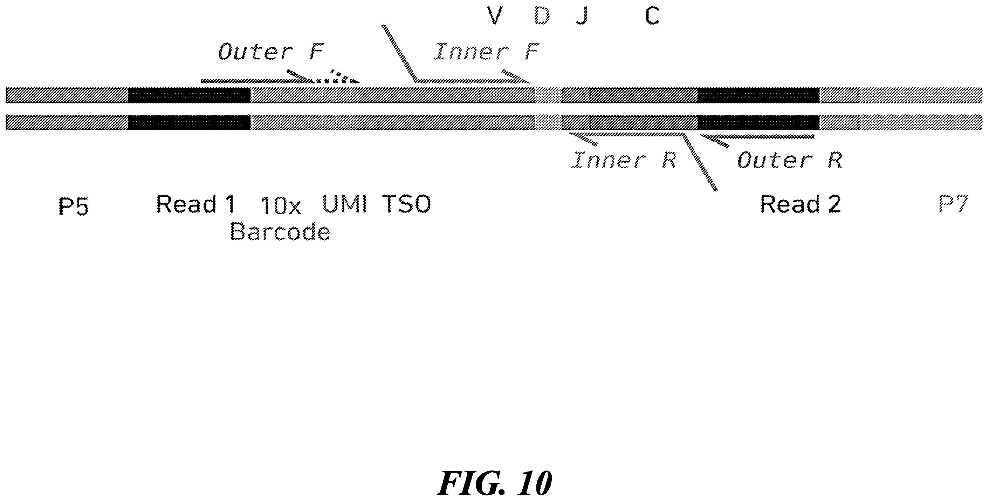

An example of such a nucleic acid molecule is included in the top panel of FIG. 10.

In some embodiments, a nucleic acid sequence of interest can be engineered to comprise a restriction site (e.g., using PCR primers comprising restriction sites). In some embodiments, a restriction site can be utilized for cloning after enrichment of the nucleic acid sequence of interest.

A nucleic acid molecule of a plurality of nucleic acid molecules can comprise a nucleic acid sequence that can code for an amino acid sequence. In some embodiments, the amino acid sequence can be of a T cell receptor or a B cell receptor. In some embodiments, the amino acid sequence can be of an antibody or antigen binding fragment thereof.

In some embodiments, a nucleic acid molecule of a plurality of nucleic acid molecules can comprise a nucleic acid sequence of interest, such as a nucleic acid sequence described herein.

As used herein, the term "antibody" can refer to an immunoglobulin (Ig), polypeptide, or a protein having a binding domain which is, or is homologous to, an antigen-binding domain. The term can further include "antigen-binding fragments" and other interchangeable terms for similar binding fragments as described herein Native antibodies and native immunoglobulins (Igs)can be heterotetrameric glycoproteins of about 150,000 Daltons, composed of two identical light chains and two identical heavy chains. Antibodies can further refer to camelid antibodies. In some cases, camelid antibodies are not tetrameric. Each light chain can be linked to a heavy chain by one covalent disulfide bond, while the number of disulfide linkages can vary among the heavy chains of different immunoglobulin isotypes. Each heavy and light chain can have regularly spaced intrachain disulfide bridges. Each heavy chain can have at one end a variable domain ("$V_H$") followed by a number of constant domains ("$C_H$"). Each light chain can have a variable domain at one end ("$V_L$") and a constant domain ("$C_L$") at its other end; the constant domain of the light chain can be aligned with the first constant domain of the heavy chain, and the light-chain variable domain can be aligned with the variable domain of the heavy chain. Particular amino acid residues can form an interface between the light- and heavy-chain variable domains.

In some instances, an antibody or an antigen-binding fragment thereof comprises an isolated antibody or antigen-binding fragment thereof, a purified antibody or antigen-binding fragment thereof, a recombinant antibody or antigen-binding fragment thereof, a modified antibody or antigen-binding fragment thereof, or a synthetic antibody or antigen-binding fragment thereof.

Antibodies and antigen-binding fragments herein can be partly or wholly synthetically produced. An antibody or antigen-binding fragment can be a polypeptide or protein having a binding domain which can be, or can be homologous to, an antigen binding domain. In some instances, an antibody or an antigen-binding fragment thereof can be produced in an appropriate *in vivo* animal model and then isolated and/or purified.

Depending on the amino acid sequence of the constant domain of its heavy chains, immunoglobulins (Igs) can be assigned to different classes. Major classes of immunoglobulins can include: IgA, IgD, IgE, IgG, and IgM, and several of these may be further divided into subclasses (isotypes), *e.g.,* IgG1, IgG2, IgG3, IgG4, IgA1 and IgA2. An Ig or portion thereof can, in some cases, be a human Ig. In some instances, a $C_H3$ domain can be from an immunoglobulin. In some cases, a chain or a part of an antibody or antigen binding fragment thereof, a modified antibody or antigen-binding fragment thereof, or a binding agent can be from an Ig. In such cases, an Ig can be IgG, an IgA, an IgD, an IgE, or an IgM. In cases where the Ig is an IgG, it can be a subtype of IgG, wherein subtypes of IgG can include IgG1, an IgG2a, an IgG2b, an IgG3, and an IgG4. In some cases, a $C_H3$ domain can be from an immunoglobulin selected from the group consisting of an IgG, an IgA, an IgD, an IgE, and an IgM.

The "light chains" of antibodies (immunoglobulins) from any vertebrate species can be assigned to one of two clearly distinct types, called kappa ("κ" or "K") or lambda ("λ"), based on the amino acid sequences of their constant domains.

A "variable region" of an antibody can refer to the variable region of the antibody light chain or the variable region of the antibody heavy chain, either alone or in combination. The variable regions of the heavy and light chain can consist of four framework regions (FR) connected by three complementarity determining regions (CDRs) also known as hypervariable regions. The CDRs in each chain can be held together in close proximity by the FRs and, with the CDRs from the other chain, contribute to the formation of the antigen-binding site of antibodies. CDRs can be determined by methods such as: (1) an approach based on cross-species sequence variability *(i.e.,* Kabat et al., Sequences of Proteins of Immunological Interest, (5th ed., 1991, National Institutes of Health, Bethesda Md.)); and (2) an approach based on crystallographic studies of antigen-antibody complexes (Al-Iazikani et al. (1997) J. Molec. Biol. 273:927-948)). As used herein, a CDR may refer to CDRs defined by either approach or by a combination of both approaches.

With respect to antibodies, the term "variable domain" can refer to the variable domains of antibodies that are used in the binding and specificity of each particular antibody for its particular antigen. In some cases, the variability is not evenly distributed throughout the variable domains of antibodies. In some cases, it is concentrated in three segments called hypervariable regions (also known as CDRs) in both the light chain and the heavy chain variable domains. More highly conserved portions of variable domains can be called the "framework regions" or "FRs." The variable domains of unmodified heavy and light chains can contain four FRs (FR1, FR2, FR3, and FR4), largely adopting a β-sheet configuration interspersed with three CDRs which can form loops connecting and, in some cases, part of the β-sheet structure. The CDRs in each chain can be held together in close proximity by the FRs and, with the CDRs from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669).

"Antibodies" useful in the present disclosure can encompass monoclonal antibodies, polyclonal antibodies, chimeric antibodies, bispecific antibodies, multispecific antibodies, heteroconjugate antibodies, humanized antibodies, human antibodies, deimmunized antibodies, mutants thereof, fusions thereof, immunoconjugates thereof, antigen-binding fragments thereof, and/or any other modified configuration of the immunoglobulin molecule that comprises an antigen recognition site of the required specificity, including glycosylation variants of antibodies, amino acid sequence variants of antibodies, and covalently modified antibodies. In some embodiments, an antibody can be a murine antibody.

An antibody can be a human antibody. As used herein, a "human antibody" can be an antibody having an amino acid sequence corresponding to that of an antibody produced by a human and/or that has been made using any suitable technique for making human antibodies. Human antibodies can include antibodies comprising at least one human heavy chain polypeptide or at least one human light chain polypeptide. One such example is an antibody comprising murine light chain and human heavy chain polypeptides. In one embodiment, the human antibody is selected from a phage library, where that phage library expresses human antibodies (Vaughan et al., 1996, Nature Biotechnology, 14:309-314; Sheets et al., 1998, PNAS USA, 95:6157-6162; Hoogenboom and Winter, 1991, J. Mol. Biol., 227:381; Marks et al., 1991, J. Mol. Biol., 222:581). Human antibodies can also be made by introducing human immunoglobulin loci into transgenic animals, e.g., mice in which the endogenous immunoglobulin genes have been partially or completely inactivated. This approach is described in U.S. Pat. Nos. 5,545,807; 5,545,806; 5,569,825; 5,625,126; 5,633,425; and 5,661,016. Alternatively, the human antibody may be prepared by immortalizing human B lymphocytes that produce an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., 1991, J. Immunol., 147 (1):86-95; and U.S. Pat. No. 5,750,373.

Any of the antibodies herein can be bispecific. Bispecific antibodies can be antibodies that have binding specificities for at least two different antigens and can be prepared using the antibodies disclosed herein. Exemplary methods for making bispecific antibodies are described (see, e.g., Suresh et al., 1986, Methods in Enzymology 121:210). The recombinant production of bispecific antibodies can be based on the coexpression of two immunoglobulin heavy chain-light chain pairs, with the two heavy chains having different specificities (Millstein and Cuello, 1983, Nature, 305, 537-539). Bispecific antibodies can be composed of a hybrid immunoglobulin heavy chain with a first binding specificity in one arm, and a hybrid immunoglobulin heavy chain-light chain pair (providing a second binding specificity) in the other arm. This asymmetric structure, with an immunoglobulin light chain in only one half of the bispecific molecule, can facilitate separation of the desired bispecific compound from unwanted immunoglobulin chain combinations. This approach is described, for example, in PCT Publication No. WO 94/04690.

Functional fragments of any of the antibodies herein are also contemplated. The terms "antigen-binding portion of an antibody," "antigen-binding fragment," "antigen-binding domain," "antibody fragment," or a "functional fragment of an antibody" can refer to one or more fragments of an antibody that retain the ability to specifically bind to an antigen. Representative antigen-binding fragments include a Fab, a Fab', a $F(ab)_2$, a Fv, a scFv, a dsFv, a variable heavy domain, a variable light domain, a variable NAR domain, bi-specific scFv, a bi-specific $Fab_2$, a tri-specific $Fab_3$, an A VIMER®, a minibody, a diabody, a maxibody, a camelid, a VHH, a minibody, an intrabody, fusion proteins comprising an antibody portion (e.g., a domain antibody), and a single chain binding polypeptide.

"$F(ab')_2$" and "Fab'" moieties can be produced by treating an Ig with a protease such as pepsin and papain, and include antibody fragments generated by digesting immunoglobulin near the disulfide bonds existing between the hinge regions in each of the two heavy chains. For example, papain can cleave IgG upstream of the disulfide bonds existing between the hinge regions in each of the two heavy chains to generate two homologous antibody fragments in which an light chain composed of $V_L$ and $C_L$ (light chain constant region), and a heavy chain fragment composed of $V_H$ and $C_{H\gamma1}$ ($\gamma$1) region in the constant region of the heavy chain) are connected at their C terminal regions through a disulfide bond. Each of these two homologous antibody fragments can be called Fab'. Pepsin can also cleave IgG downstream of the disulfide bonds existing between the hinge regions in each of the two heavy chains to generate an antibody fragment slightly larger than the fragment in which the two above-mentioned Fab' are connected at the hinge region. This antibody fragment can be called $F(ab')_2$.

The Fab fragment can also contain the constant domain of the light chain and the first constant domain ($C_H1$) of the heavy chain. Fab' fragments can differ from Fab fragments by the addition of a few residues at the carboxyl terminus of the heavy chain $C_H1$ domain including one or more cysteine(s) from the antibody hinge region. Fab'-SH can be a Fab' in which the cysteine residue(s) of the constant domains bear a free thiol group. $F(ab')_2$ antibody fragments can be produced, for example, as pairs of Fab' fragments which have hinge cysteines between them. Other chemical couplings of antibody fragments can also be employed.

A "Fv" as used herein can refer to an antibody fragment which contains a complete antigen-recognition and antigen-binding site. This region can consist of a dimer of one heavy chain and one light chain variable domain in tight, non-covalent or covalent association (disulfide linked Fvs have been described, see, e.g., Reiter et al. (1996) Nature Biotechnology 14:1239-1245). In this configuration that the three CDRs of each variable domain can interact to define an antigen-binding site on the surface of the $V_H$-$V_L$ dimer. Collectively, a combination of one or more of the CDRs can from each of the $V_H$ and $V_L$ chains confer antigen-binding specificity to the antibody. For example, the CDRH3 and CDRL3 can be sufficient to confer antigen-binding specificity to an antibody when transferred to $V_H$ and $V_L$ chains of a recipient antibody or antigen-binding fragment thereof and this combination of CDRs can be tested for binding, specificity, affinity, *etc.* using, for example, techniques described herein. In some cases, even a single variable domain (or half of an Fv comprising only three CDRs specific for an antigen) can have the ability to recognize and bind antigen, although likely at a lower specificity or affinity than when combined with a second variable domain. Furthermore, although the two domains of a Fv fragment ($V_L$ and $V_H$) can be coded for by separate genes, they can be joined using recombinant methods, for example by a synthetic linker that enables them to be made as a single protein chain in which the $V_L$ and $V_H$ regions pair to form monovalent molecules (known as single chain Fv (scFv); Bird et al. (1988) Science 242:423-426; Huston et al. (1988) Proc. Natl. Acad. Sci. USA 85:5879-5883; and Osbourn et al. (1998) Nat. Biotechnol. 16:778). Such scFvs can be encompassed within the term "antigen-binding portion" of an antibody. Any $V_H$ and $V_L$ sequences of specific scFv can be linked to an Fc region cDNA or genomic sequences in order to generate expression vectors encoding complete Ig (*e.g.,* IgG) molecules or other isotypes. $V_H$ and $V_L$ can also be used in the generation of Fab, Fv, or other fragments of Igs using either protein chemistry or recombinant DNA technology.

"Single-chain Fv" or "sFv" antibody fragments can comprise the $V_H$ and $V_L$ domains of an antibody, wherein these domains can be present in a single polypeptide chain. In some embodiments, the Fv polypeptide can further comprise a polypeptide linker between the $V_H$ and $V_L$ domains which enables the sFv to form the desired structure for antigen binding. For a review of sFvs, *see, e.g.,* Pluckthun in The Pharmacology of Monoclonal Antibodies, Vol. 113, Rosenburg and Moore eds. Springer-Verlag, New York, pp. 269-315 (1994).

Also contemplated herein is the use of an A VIMER® as an antigen or antigen binding moiety. The term "A VIMER®" can refer to a class of therapeutic proteins of human origin, which can be unrelated to antibodies and antibody fragments, and can be composed of several modular and reusable binding domains, referred to as A-domains (also referred to as class A module, complement type repeat, or LDL-receptor class A domain). They can be developed from human extracellular receptor domains by *in vitro* exon shuffling and phage display (Silverman et al., 2005, Nat. Biotechnol. 23:1493-1494; Silverman et al., 2006, Nat. Biotechnol. 24:220). The resulting proteins can contain multiple independent binding domains that can exhibit improved affinity and/or specificity compared with single-epitope binding proteins. Each of the known 217 human A-domains can comprise ~35 amino acids (~4 kDa); and these domains can be separated by linkers that can average five amino acids in length. Native A-domains fold quickly and efficiently to a uniform, stable structure mediated primarily by calcium binding and disulfide formation. A conserved scaffold motif of only 12 amino acids can be required for this common structure. The end result can be a single protein chain containing multiple domains, each of which represents a separate function. Each domain of the proteins can bind independently, and the energetic contributions of each domain can be additive.

Antigen-binding polypeptides can also include heavy chain dimers such as, for example, antibodies from camelids and sharks. Camelid and shark antibodies can comprise a homodimeric pair of two chains of V-like and C-like domains (neither has a light chain). Since the $V_H$ region of a heavy chain dimer IgG in a camelid does may not have to make hydrophobic interactions with a light chain, the region in the heavy chain that normally contacts a light chain can be changed to hydrophilic amino acid residues in a camelid. $V_H$ domains of heavy-chain dimer IgGs can be called $V_{HH}$ domains. Shark Ig-NARs can comprise a homodimer of one variable domain (termed a V-NAR domain) and five C-like constant domains (C-NAR domains). In camelids, the diversity of antibody repertoire can be determined by the CDRs 1, 2, and 3 in the $V_H$ or $V_{HH}$ regions. The CDR3 in the camel $V_{HH}$ region can be characterized by its relatively long length, averaging 16 amino acids (Muyldermans et al., 1994, Protein Engineering 7(9): 1129). This can be in contrast to CDR3 regions of antibodies of many other species. For example, the CDR3 of mouse $V_H$ can have an average of 9 amino acids. Libraries of camelid-derived antibody variable regions, which can maintain the *in vivo* diversity of the variable regions of a camelid, can be made by, for example, the methods disclosed in U.S. Patent Application Ser. No. 20050037421.

As used herein, a "maxibody" can refer to a bivalent scFv covalently attached to the Fc region of an immunoglobulin, *see, e.g.,* Fredericks et al., Protein Engineering, Design & Selection, 17:95-106 (2004) and Powers et al., Journal of Immunological Methods, 251:123-135 (2001).

As used herein, a "dsFv" can be a Fv fragment obtained, for example, by introducing a Cys residue into a suitable site in each of a heavy chain variable region and a light chain variable region, and then stabilizing the heavy chain variable region and the light chain variable region by a disulfide bond. The site in each chain, into which the Cys residue can be introduced, can be determined based on a conformation predicted by molecular modeling. In the present disclosure, for example, a conformation can be predicted from the amino acid sequences of the heavy chain variable region and light chain variable region of the above-described antibody, and DNA encoding each of the heavy chain variable region and the light chain variable region, into which a mutation has been introduced based on such prediction, can be then constructed. The DNA construct can be incorporated then into a suitable vector and prepared from a transformant obtained by transformation with the aforementioned vector.

Single chain variable region fragments ("scFv") of antibodies are described herein. Single chain variable region fragments may be made by linking light and/or heavy chain variable regions by using a short linking peptide. Bird et al. (1988) Science 242:423-426. The single chain variants can be produced either recombinantly or synthetically. For synthetic production of scFv, an automated synthesizer can be used. For recombinant production of scFv, a suitable plasmid containing polynucleotide that encodes the scFv can be introduced into a suitable host cell, either eukaryotic, such as yeast, plant, insect, or mammalian cells, or prokaryotic, such as *E. coli.* Polynucleotides encoding the scFv of interest can be made by routine manipulations such as ligation of polynucleotides. The resultant scFv can be isolated using any suitable protein purification techniques.

Diabodies can be single chain antibodies. Diabodies can be bivalent, bispecific antibodies in which VH and VL domains can be expressed on a single polypeptide chain, but using a linker that is too short to allow for pairing between the two domains on the same chain, thereby forcing the domains to pair with complementary domains of another chain and creating two antigen binding sites *(see, e.g.,* Holliger, P., et al., Proc. Natl. Acad. Sci. USA, 90:6444-6448 (1993); and Poljak, R. J., et al., Structure, 2:1121-1123 (1994)).

As used herein, a "minibody" can refer to a scFv fused to CH3 via a peptide linker (hingeless) or via an IgG hinge has been described in Olafsen, et al., Protein Eng Des Sel. April 2004; 17(4):315-23.

As used herein, an "intrabody" can refer to a single chain antibody which can demonstrate intracellular expression and can manipulate intracellular protein function (Biocca, et al., EMBO J. 9:101-108, 1990; Colby et al., Proc Natl Acad. Sci. USA. 101:17616-21, 2004). Intrabodies, which can comprise cell signal sequences which can retain the antibody construct in intracellular regions, may be produced, for example, as described in Mhashilkar et al., (EMBO J., 14:1542-51, 1995) and Wheeler et al. (FASEB J. 17:1733-5. 2003). Transbodies can be cell-permeable antibodies in which a protein transduction domains (PTD) can be fused with single chain variable fragment (scFv) antibodies as in, for example, Heng et al. (Med Hypotheses. 64:1105-8, 2005).

An antibody or antigen binding fragment can bind an epitope. An epitope can be a portion of an antigen or other macromolecule capable of forming a binding interaction with the variable region binding pocket of an antigen binding molecule such as an antibody or antigen binding fragment thereof. Such binding interactions can be manifested as an intermolecular contact with one or more amino acid residues of one or more CDRs. Antigen binding can involve, for example, a CDR3, a CDR3 pair or, in some cases, interactions of up to all six CDRs of the $V_H$ and $V_L$ chains. An epitope can be a linear peptide sequence *(i.e.,* "continuous") or can be composed of noncontiguous amino acid sequences *(i.e.,* "conformational" or "discontinuous"). An antigen binding molecule such as an antibody or antigen binding fragment thereof can recognize one or more amino acid sequences; therefore, an epitope can define more than one distinct amino acid sequence. Epitopes recognized by antigen binding molecules such as antibodies or antigen binding fragments thereof can be determined by peptide mapping or sequence analysis techniques. In some embodiments, binding interactions can be manifested as intermolecular contacts between an epitope on an antigen and one or more amino acid residues of a CDR. Epitopes recognized by antigen binding molecules such as antibodies or antigen binding fragments thereof can be determined, for example, by peptide mapping or sequence analysis techniques. Binding interactions can manifest as intermolecular contacts between an epitope on an antigen and one or more amino acid residues of a complementarity determining region (CDR).

An epitope can be determined, for example, using one or more epitope mapping techniques. Epitope mapping can comprise experimentally identifying the epitope on an antigen. Epitope mapping can be performed by any acceptable method, for example X-ray co-crystallography, cryogenic electron microscopy, array-based oligo-peptide scanning, site-directed mutagenesis mapping, high-throughput shotgun mutagenesis epitope mapping, hydrogen-deuterium exchange, cross-linking coupled mass spectrometry, yeast display, phage display, proteolysis, or a combination thereof.

An antibody or antigen binding fragment thereof can comprise a V(D)J sequence. The variable region of each immunoglobulin heavy or light chain can be encoded by a plurality of subgenes. These subgenes can comprise variable (V), diversity (D) and joining (J) segments, and can be combined to yield a V(D)J sequence. A heavy chain can comprise V, D and/or J segments, and a light chain can comprise V and/or J segments. Multiple copies of the V, D and J gene segments exist, and can be tandemly arranged in the genomes of mammals. In the bone marrow, each developing B cell can assemble an immunoglobulin variable region, for example by randomly selecting and combining one V, one D and one J gene segment (or one V and one J segment in the light chain). As there can be multiple copies of each type of gene segment, and different combinations of gene segments can be used to generate each immunoglobulin variable region, this process can generate a large number of antibodies with different paratopes, and in some embodiments, different antigen specificities. The rearrangement of several subgenes (e.g., in the V2 family) for lambda light chain immunoglobulin can be coupled with the activation of microRNA miR-650, which can further influence the biology of B-cells.

A plurality of nucleic acid molecules can comprise a library of nucleic acid molecules. As a non-limiting example, a library of nucleic acid molecules can comprise complementary deoxyribonucleic acid (cDNA molecules). In some cases, a library of nucleic acid molecules can comprise a library of cDNA molecules. In some embodiments, a library of nucleic acid molecules can comprise a library of variants of a nucleic acid molecule. Variants of a nucleic acid molecule can comprise variants of a nucleic acid molecule that codes for an amino acid sequence, such as an amino acid sequence of an antibody or antigen binding fragment thereof. In some embodiments, variants of a nucleic acid molecule can comprise a nucleic acid sequence coding for an amino acid sequence of a T cell receptor or a B cell receptor.

For example, variants of an antibody or antigen binding fragment thereof can comprise variants in a variable region. In some embodiments, variants in a variable region can comprise a variant in a V sequence, a variant in a D sequence, a variant in a J sequence, or a combination thereof. Variants of an antibody or antigen binding fragment thereof can have different specificity (e.g., having specificity for different antigens) or different affinity (e.g., having different affinity for a same antigen).

A nucleic acid molecule of a plurality of nucleic acid molecules can comprise a plurality of nucleic acid molecules that can comprise a nucleic acid sequence coding for a V amino acid sequence, a D amino acid sequence, a J amino acid sequence, or a combination thereof. In some embodiments, a nucleic acid molecule of a plurality of nucleic acid molecules can comprise a nucleic acid sequence coding for a V(D)J amino acid sequence. In some embodiments, a nucleic acid sequence of interest can comprise a nucleic acid sequence coding for a V(D)J amino acid sequence. In some embodiments, different nucleic acid sequences of a plurality of nucleic acid molecules can comprise nucleic acid sequences coding for different V(D)J amino acid sequences. Different V(D)J amino acid sequences can comprise different V sequences, different D sequences, different J sequences, or a combination thereof.

A plurality of nucleic acid molecules can correspond to a plurality of cell surface proteins from a plurality of cells. In some cases, the plurality of cell surface proteins from a plurality of cells can be different. In some cases, different cell surface proteins can be variations of a cell surface protein. Examples of cell surface proteins can include T cell receptors (e.g., of T cells), B cell receptors (e.g., of B cells), or antibodies or antigen binding fragments thereof. Cell surface proteins can be naturally occurring or synthetic. In some cases, a cell surface protein can be a modified natural protein.

In some embodiments, providing the plurality of nucleic acid molecules can comprise generating the plurality of nucleic acid molecules. The plurality of nucleic acid molecules generated can comprise a plurality of identification sequences that identify said plurality of nucleic acid molecules.

Generation of a library of nucleic acid molecules can be accomplished for example by collecting nucleic acid molecules and appending identification sequences (e.g., as described herein to include barcodes, unique molecular identifiers, and/or template switch oligonucleotides) to the nucleic acid molecules. For example, nucleic acid molecules coding for T cell receptors, B cell receptors, or antigens or antibody fragments thereof can be isolated from samples (e.g., cells) and labeled with an identification sequence using methods provided herein. A library can be generated, for example, as described in U.S. Patent No.: 10,550,429. Once a library is created, a member of the library can be created, one of the library can be enriched using a primer complementary to at least a portion of the identification sequence. In some embodiments, the enriched member of the library (i.e., nucleic acid sequence of interest) can be cloned and expressed, and in some cases further analysis can be performed on the amino acid product of the nucleic acid sequence of interest (e.g., T cell receptor, B cell receptor, or antibody or antigen binding fragment thereof).

### *Enrichment*

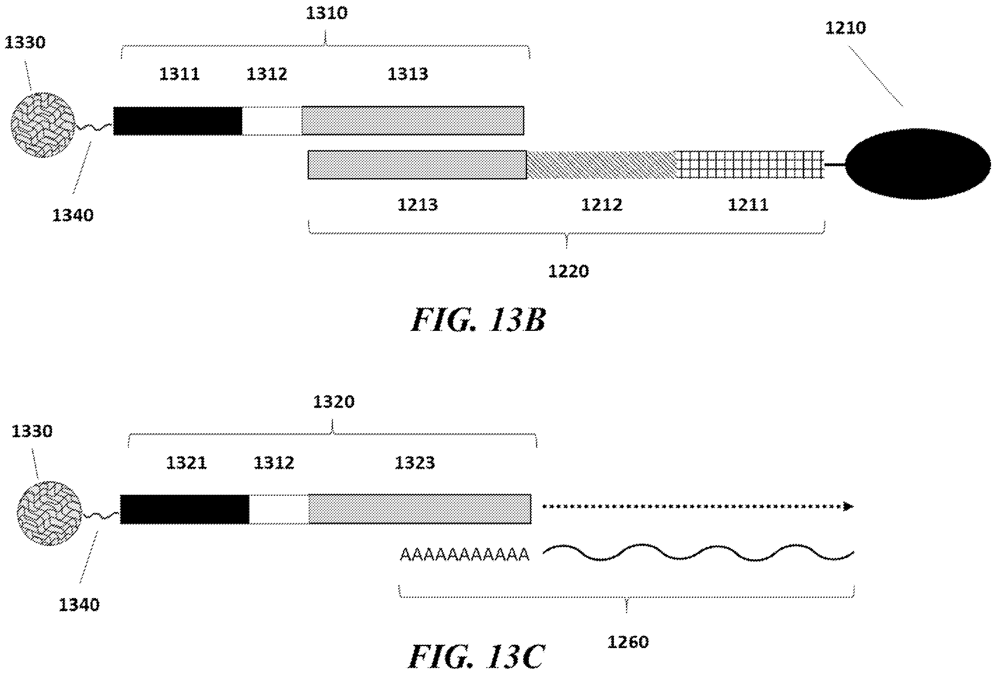

A nucleic acid sequence of interest can be enriched from a library of nucleic acid molecules. A library of nucleic acid molecules may be a cDNA library generated from a single cell, e.g., B cell, from the immune repertoire of a subject. Such a library may be generated, for example, by isolating and/or amplifying RNA, and reverse transcribing the RNA library to yield a cDNA library. The library may be a barcoded gene expression library generated from a cell, e.g., B cell, partitioned with a barcoded bead. The cell, following lysis or permeabilization, may have its RNA reverse transcribed, and during reverse transcription, have identification sequences (e.g., barcode sequence or unique molecular identification sequences) appended thereto (e.g., generating a whole transcriptome barcoded gene expression library). See, e.g., **FIG. 12B** or **FIG. 13C.** Depending on sequences included in the identification sequence, the library may be a sequencing library. Methods for enriching a nucleic acid sequence of interest can comprise an amplification reaction. Examples of amplification reactions can include linear amplification, polymerase chain reaction (PCR), and nested PCR. In some embodiments, a different amplification reaction can be employed.

PCR can comprise denaturation, annealing, and extension steps. Denaturation can comprise exposing the nucleic acid to a temperature capable of melting the nucleic acid. In some cases, denaturation can occur between 94 °C and 98 °C. In some cases, denaturation can occur at 94 °C, 95 °C, 96 °C, 97 °C, or 98 °C. Denaturation can last for at least 15 seconds, at least 30 seconds, at least 45 seconds, at least 60 seconds, at least 75 seconds, at least 90 seconds, at least 105 seconds, at least 120 seconds, at least 135 seconds, at least 150 seconds, at least 165 seconds, or at least 180 seconds. Annealing can comprise exposing the melted nucleic acid to a temperature which can allow the binding of a primer to the nucleic acid. In some cases, annealing can occur between 50 °C and 75 °C. In some cases, annealing can occur between 55 °C and 70 °C. In some instances, annealing can occur at 55 °C, 56 °C, 57 °C, 58 °C, 59 °C, 60 °C, 61 °C, 62 °C, 63 °C, 64 °C, 65 °C, 66 °C, 67 °C, 68 °C, 69 °C, or 70 °C. Annealing can last for at least 15 seconds, at least 30 seconds, at least 45 seconds, at least 60 seconds, at least 75 seconds, at least 90 seconds, at least 105 seconds, at least 120 seconds, at least 135 seconds, at least 150 seconds, at least 165 seconds, or at least 180 seconds. Extension can comprise exposing the nucleic acid to a temperature at which extension can occur, thereby amplifying the nucleic acid, for example by a polymerase present in the partition with the nucleic acid. Extension can occur between 65 °C and 75 °C. In some cases, extension can occur at 65 °C, 66 °C, 67 °C, 68 °C, 69 °C, 70 °C, 71 °C, 72 °C, 73 °C, 74 °C, or 75 °C. The steps of denaturation, annealing, and extension can be repeated for a number of cycles. In some cases, PCR cycling can proceed for at least 1 cycle. In some cases, PCR cycling can proceed for at least 5, 10, 15, 20, 25, 30, 35, or 40 cycles. In some cases, PCR cycling can proceed for between 1 cycle and 40 cycles, between 1 cycle and 35 cycles, between 1 cycle and 30 cycles, between 1 cycle and 25 cycles, between 1 cycle and 20 cycles, between 1 cycle and 15 cycles, between 1 cycle and 10 cycles, between 1 cycle and 5 cycles, between 5 cycles and 40 cycles, between 5 cycles and 35 cycles, between 5 cycles and 30 cycles, between 5 cycles and 25 cycles, between 5 cycles and 20 cycles, between 5 cycles and 15 cycles, between 5 cycles and 10 cycles, between 10 cycles and 40 cycles, between 10 cycles and 35 cycles, between 10 cycles and 30 cycles, between 10 cycles and 25 cycles, between 10 cycles and 20 cycles, between 10 cycles and 15 cycles, between 15 cycles and 40 cycles, between 15 cycles and 35 cycles, between 15 cycles and 30 cycles, between 15 cycles and 25 cycles, between 15 cycles and 20 cycles, between 20 cycles and 40 cycles, between 20 cycles and 35 cycles, between 20 cycles and 30 cycles, between 20 cycles and 25 cycles, between 25 cycles and 40 cycles, between 25 cycles and 35 cycles, between 25 cycles and 30 cycles, between 30 cycles and 40 cycles, between 30 cycles and 35 cycles, or between 35 cycles and 40 cycles.

Methods for enriching a nucleic acid sequence of interest, such as an amplification reaction, can comprise contacting the nucleic acid sequence of interest with PCR reaction, e.g., reagents for a PCR reaction. In some cases, reagents for a PCR reaction can comprise a polymerase, one or more sets primers, and a dNTP mixture. In some cases, e.g., in an amplification reaction, a polymerase can be a DNA polymerase, an RNA polymerase, or a reverse transcriptase. In some cases, a set of primers can comprise at least 2 primers which can be complementary to a region of a nucleic acid of interest, such that the region of the nucleic acid of interest can be amplified via PCR using the primer pair. In some cases, a partition can comprise at least 1, 2, 3, 4, 5, 6, 7, 8, 9, 10, 11, 12, 13, 14, 15, 16, 17, 18, 19, or 20 sets of two primers. In some cases, a partition can comprise no more than 1, 2, 3, 4, 5, 6, 7, 8, 9, 10, 11, 12, 13, 14, 15, 16, 17, 18, 19, or 20 sets of two primers. In some cases, a partition can comprise one or more probes. A probe can be a DNA binding dye, a hydrolysis probe, a molecular beacon, a dual hybridization probe, an eclipse probe, or an ampliflouor probe. In some cases, a probe can be a SYBR green probe, a Taqman probe, a Scorpions PCR primer, a LUX PCR primer, or a QZyme PCR primer. In some cases, a probe can comprise a label, which can be colored, opaque, radiopaque, fluorescent, radioactive, or otherwise detectable. In some cases, a partition can comprise additional reagents, which can comprise magnesium, salt, glycerol, buffer, dye, or other reagents. A first set of partitions and a second set of partitions can be obtained. In some cases, these partitions can each comprise a nucleic acid molecule, e.g., a target nucleic acid molecule, which can be amplified and detected. A set of partitions can comprise a plurality of partitions. In some cases, a set of partitions can comprise at least 1, at least 10, at least 100, at least 1,000, at least 10,000, at least 100,000, at least 1,000,000, or at least 10,000,000 partitions. In some cases, a set of partitions can comprise a set of droplets, e.g., an aqueous droplet in an emulsion. For example, a first set of partitions can comprise a first set of droplets, and a second set of partitions can comprise a second set of droplets.

Methods for enriching a nucleic acid sequence of interest, such as an amplification reaction, can comprise contacting the nucleic acid sequence of interest with a nucleic acid primer. In some cases, a nucleic acid primer can be an oligonucleotide suitable for a PCR reaction (e.g., a PCR primer).

A nucleic acid primer can comprise an oligonucleotide. An oligonucleotide can be a molecule which can be a chain of nucleotides. Oligonucleotides described herein can comprise ribonucleic acids. Oligonucleotides described herein can comprise deoxyribonucleic acids. In some cases, oligonucleotides can be of any sequence, including a user-specified sequence.

In some embodiments, an oligonucleotide can comprise G, A, T, U, C, or bases that are capable of base pairing reliably with a complementary nucleotide. 7-deaza-adenine, 7-deaza-guanine, adenine, guanine, cytosine, thymine, uracil, 2-deaza-2-thio-guanosine, 2-thio-7-deaza-guanosine, 2-thio- adenine, 2-thio- 7-deaza-adenine, isoguanine, 7-deaza-guanine, 5,6-dihydrouridine, 5,6- dihydrothymine, xanthine, 7-deaza-xanthine, hypoxanthine, 7-deaza-xanthine, 2,6 diamino-7- deaza purine, 5- methyl-cytosine, 5-propynyl-uridine, 5-propynyl-cytidine, 2-thio-thymine or 2-thio-uridine are examples of such bases, although many others are known. An oligonucleotide can comprise an LNA, a PNA, a UNA, or an morpholino oligomer, for example. The oligonucleotides used herein may contain natural or non- natural nucleotides or linkages.

An oligonucleotide can be at least 5, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, or at least 100 nucleotides long. In some cases, an oligonucleotide can be between 10-30, between 10-50, between 10-70, between 10-100, between 20-50, between 20-70, between 20-100, between 30-50, between 30-70, between 30-100, between 40-70, between 40-100, between 50-70, between 50-100, between 60-70, between 60-80, between 60-90, or between 60-100 nucleotides in length. In some cases, an oligonucleotide can be no more than 5, no more than 10, no more than 15, no more than 20, no more than 25, no more than 30, no more than 35, no more than 40, no more than 45, no more than 50, no more than 55, no more than 60, no more than 65, no more than 70, no more than 75, no more than 80, no more than 85, no more than 90, no more than 95, or no more than 100 nucleotides long.

In some cases, an oligonucleotide can be wholly single stranded. In some cases, an oligonucleotide can be partially double stranded. A partially double stranded region can be at the 3' end of the oligonucleotide, at the 5' end of the oligonucleotide, or between the 5' end and 3' end of the oligonucleotide. In some cases, there may be more than one double stranded region.

Methods can comprise using a nucleic acid primer complementary to a portion of the nucleic acid sequence of interest (e.g., the identification sequence or a portion thereof). In some embodiments, a nucleic acid primer complementary to at least a portion of the identification sequence of a nucleic acid sequence of interest can be complimentary to at least a portion of a barcode sequence, at least a portion of a template switch oligonucleotide sequence, at least a portion of a unique molecular identifier sequence, or a combination thereof. In some embodiments, a nucleic acid primer can be complementary to a barcode sequence and a read sequence of the nucleic acid sequence of interest, or a portion thereof. In some embodiments, a nucleic acid primer can be complementary to a barcode sequence and a unique molecular identifier sequence, or a portion thereof. This can be done to amplify the nucleic acid sequence of interest, for example using an amplification reaction provided herein (e.g., PCR or nested PCR).

In some embodiments, a nucleic acid primer can further comprise a nucleic acid sequence that can be complementary to at least a portion of a coding sequence of the nucleic acid sequence of interest. In some embodiments, the nucleic acid primer can comprise a nucleic acid sequence that can be complementary to a variable region of the nucleic acid sequence of interest, such as the variable region of a T cell receptor, the variable region of a B cell receptor, or the variable region of an antigen or antibody binding fragment thereof. As an example, the nucleic acid primer can comprise a nucleic acid sequence that is complementary to a V(D)J sequence or a portion thereof, a V sequence of the V(D)J sequence or a portion thereof, a D sequence of the V(D)J sequence or a portion thereof, or a J sequence of the V(D)J sequence or a portion thereof. In some embodiments, a nucleic acid primer can be complementary to a portion of the variable region of the nucleic acid sequence of interest that is different than that of a different nucleic acid molecule.

In some embodiments, a nucleic acid primer can further comprise a non-binding handle. A non-binding handle can be a nucleic acid sequence on the nucleic acid primer that is not complementary to a segment of the nucleic acid sequence of interest. In some embodiments, a non-binding handle may not bind any nucleic acid sequence in the plurality of nucleic acid molecules. In some embodiments, a non-binding handle can be utilized for cloning into recipient vector or to enable pairing of specific heavy/light (or TRA/TRB) sequences using overlap extension or a similar method after enrichment of the nucleic acid molecule.

In some embodiments, methods provided herein can further comprise using another nucleic acid primer to amplify said nucleic acid sequence of interest, wherein said another nucleic acid primer is different from said nucleic acid primer. For example, a method can comprise using a first nucleic acid primer and a second nucleic acid primer (e.g., a forward primer and a reverse primer for a PCR reaction).

In some embodiments, the another nucleic acid primer can comprise a non-binding handle. Such a non-binding handle can be a nucleic acid sequence on the nucleic acid primer that is not complementary to a segment of the nucleic acid sequence of interest. In some embodiments, a non-binding handle may not bind any nucleic acid sequence in the plurality of nucleic acid molecules. In some embodiments, a non-binding handle can be utilized for cloning into recipient vector or to enable pairing of specific heavy/light (or TRA/TRB) sequences using overlap extension or a similar method after enrichment of the nucleic acid molecule.

In some methods, the nucleic acid primer and another (e.g., a second) nucleic acid primer (e.g., a forward primer and a reverse primer) can be configured to anneal to sequences flanking at least a portion of said nucleic acid sequence of interest. For example, a nucleic acid primer can be configured to anneal to a sequence upstream of the nucleic acid sequence of interest, and a second nucleic acid primer can be configured to anneal to a complement of a sequence downstream of the nucleic acid sequence of interest. In some embodiments, two such nucleic acid primers can be configured to yield a copy of the nucleic acid sequence of interest after an amplification reaction such as PCR.

In some embodiments, a second nucleic acid primer can comprise a nucleic acid sequence complementary to a binding sequence on the nucleic acid sequence of interest, or a complement thereof. Such a binding sequence can be on a coding section of the nucleic acid sequence of interest, or upstream or downstream of the coding section of the nucleic acid sequence of interest. In some embodiments, the second nucleic acid primer can be complementary to at least a portion of a nucleic acid sequence coding for a constant region of an amino acid sequence coded for by the nucleic acid sequence of interest, such as a T cell receptor, a B cell receptor, or an antibody or antigen binding fragment thereof, or a complement of such a nucleic acid sequence.

The second nucleic acid primer can be at least partially complementary to a variable region of a nucleic acid sequence of interest (e.g., a V(D)J sequence, a V sequence, a D sequence, or a J sequence). For example, the second nucleic acid primer can be further complementary to at least a portion of a nucleic acid sequence coding for a J region of an antibody or antigen binding fragment thereof.

Methods can further comprise a second enrichment step, such as a second amplification reaction. A second amplification reaction can comprise linear amplification, PCR, or another amplification scheme. In some embodiments, a second PCR reaction can be implemented to enrich or further enrich a nucleic acid sequence of the plurality of nucleic acid molecules. As an example, a nested PCR scheme can be utilized to provide enrichment of a nucleic acid sequence of interest.

A second round of amplification can comprise contacting the nucleic acid sequence with a third primer and a fourth primer. A third primer or a fourth primer can comprise an oligonucleotide as provided herein. A third primer and a fourth primer can be configured to specifically enrich the nucleic acid sequence. In some embodiments, the third primer can be different than the first primer, or the fourth primer can be different than the second primer.

The third primer can be complementary to at least a portion of the identification sequence. In some embodiments, the third primer can be complementary to a portion of a barcode of the identification sequence. In some cases, the third primer can be complementary to a 5' end of a barcode of the identification sequence.

In some embodiments, a third primer can be complementary to a portion of the identification sequence upstream of a barcode of the identification sequence. For example, a third primer can be complementary to at least a portion of a read sequence of a nucleic acid molecule. In some embodiments, a third primer can be complementary to at least a portion of a variable sequence, such as a nucleic acid sequence coding for a V(D)J sequence.

A fourth primer can be complementary to the complement of another segment of the nucleic acid molecule, such that the nucleic acid sequence of interest can be flanked by the third primer and the fourth primer. In some embodiments, the fourth primer can be complementary to a nucleic acid sequence downstream of a coding sequence of the nucleic acid sequence of interest. In some embodiments, the fourth primer can be complementary to at least a portion of the complement of a constant region of the nucleic acid sequence of interest.

In some methods, others of the plurality of nucleic acid molecules can be not amplified. For example, nucleic acid molecules that do not comprise the nucleic acid sequence of interest can be not amplified. In some methods, others of the plurality of nucleic acid molecules can be amplified by less than a threshold amount. For example, a nucleic acid sequence of interest can be amplified by more than 100 times, more than 1000 times, more than 10,000 times, more than 100,000 times, more than 1,000,000 times, or more than 10,000,000 times more than others of the plurality of nucleic acid molecules.

Methods provided herein can further comprise determining an enrichment level of the nucleic acid sequence of interest. Enrichment can be determined, for example, by fluorescence, gel electrophoresis, sequencing, or another acceptable method for determining enrichment.

A nucleic acid sequence of interest can be enriched by a factor of at least 1000 at least 10,000, at least 100,000, at least 1,000,000, or at least 10,000,000. In some embodiments, a nucleic acid sequence of interest can be enriched by a factor sufficient for cloning the nucleic acid sequence of interest. In some embodiments, a nucleic acid sequence of interest can be further enriched by a second amplification step by a factor of at least 1000, at least 10,000, at least 100,000, at least 1,000,000, or at least 10,000,000.

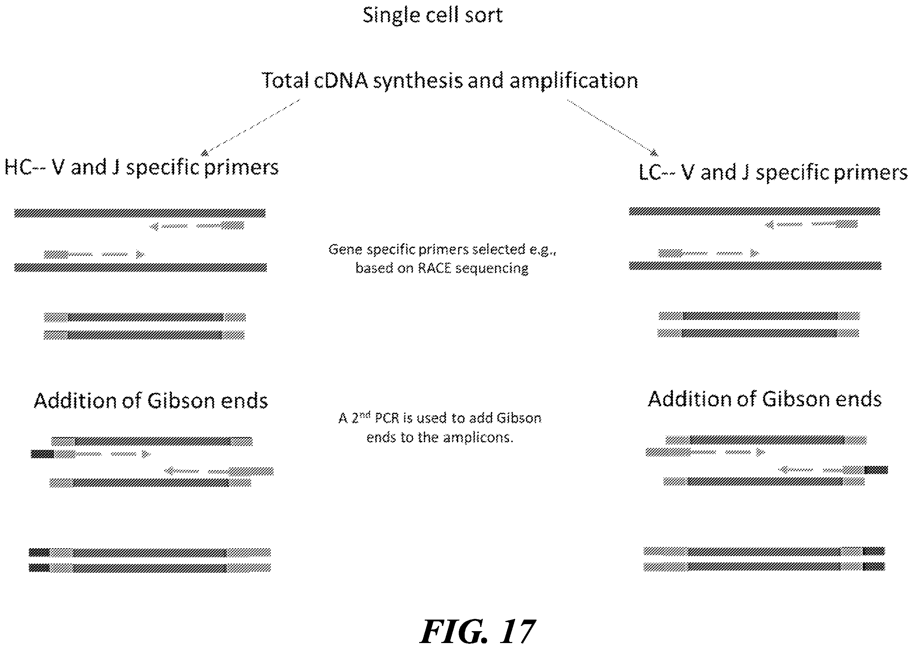

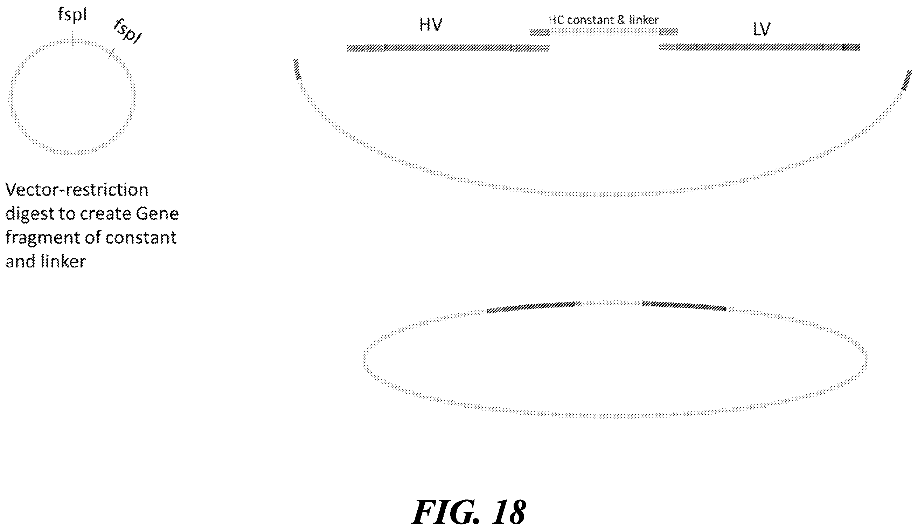

Also provided herein are methods comprising enriching a nucleic acid sequence of interest based on at least a portion of a constant region of said nucleic acid sequence of interest. This enrichment can yield an enriched nucleic acid sequence of interest. In some embodiments, the method can further comprise modification of the enriched nucleic acid sequence yielding a modified enriched nucleic acid sequence. A pictorial outline of such a method is provided in FIG. 17. The modified enriched nucleic acid sequence can be compatible with a vector. For example, the modified enriched nucleic acid sequence can have a structure (e.g., a nucleic acid sequence) that can be directly incorporated into a vector. A vector can be a vector suitable for cloning or expression of the modified enriched nucleic acid sequence or other use of the modified enriched nucleic acid sequence. A vector can be any vector described herein. A pictorial outline of a nucleic acid sequence that is compatible with a vector (including incorporation of the nucleic acid sequence into the vector) is provided in FIG. 18.

A nucleic acid sequence of interest can be a nucleic acid sequence described herein. For example, a nucleic acid sequence of interest can code for at least a portion of a cell surface protein of a cell, such as a T cell receptor (or fragment thereof) or a B cell receptor (or fragment thereof). A nucleic acid sequence of interest can comprise a constant region. In some embodiments, the nucleic acid sequence of interest can comprise a sequence encoding a V(D)J sequence or a portion thereof, such as a V sequence (or portion thereof), a D sequence (or portion thereof), or a J sequence (or portion thereof), as described herein. In some embodiments, the constant region of a nucleic acid sequence of interest can comprise a sequence encoding a V(D)J sequence or a portion thereof, such as a V sequence (or portion thereof), a D sequence (or portion thereof), or a J sequence (or portion thereof), as described herein. In some embodiments, a nucleic acid sequence of interest can comprise a barcode (e.g., as provided herein), a UMI (e.g., as provided herein), or a 5' untranslated region (5' UTR) of a gene of interest (e.g., a TCR gene or BCR gene). In some embodiments, the nucleic acid sequence of interest can comprise complementary deoxyribonucleic acid (cDNA) of an RNA transcript of interest (e.g., a TCR or BCR transcript).

Enriching can be performed using a first nucleic acid primer. A first nucleic acid primer can be complementary to a region of the nucleic acid sequence of interest. For example, the first nucleic acid primer can be complementary at least to a barcode or portion thereof on said nucleic acid sequence of interest. In some embodiments, the first nucleic acid primer can be complementary to a UMI sequence or a portion thereof on said nucleic acid sequence of interest. In some embodiments, the first nucleic acid primer can be complementary at least to a 5' untranslated region (5' UTR) or a portion thereof on said nucleic acid sequence of interest. In some embodiments, the first nucleic acid primer can be a framework leader (FWR1) primer.

Enriching can be performed using a second nucleic acid primer. In some embodiments, the second nucleic acid primer can be used with the first nucleic acid primer to enrich the nucleic acid sequence of interest. In some embodiments, the second nucleic acid primer can be complementary at least to a constant region or portion thereof on said nucleic acid sequence of interest. In some embodiments, the second nucleic acid primer can be complementary at least to a V(D)J sequence or portion thereof on said nucleic acid sequence of interest. In some embodiments, the second nucleic acid primer can be complementary at least to a J sequence or portion thereof on said nucleic acid sequence of interest. In some embodiments, the second nucleic acid primer can be complementary at least to a nucleic acid sequence of a junction region or portion thereof on said nucleic acid sequence of interest.

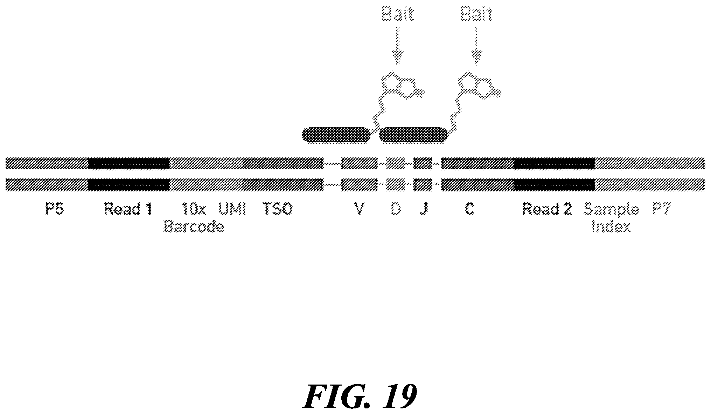

Enriching can be performed using hybridization capture. In some embodiments, the hybridization capture can be based on hybridization of a nucleic acid probe to a sequence on said nucleic acid sequence of interest such as a constant sequence or a junction sequence. For example, a probe can hybridize to a portion of a junction sequence such as a V(D)J sequence or a portion thereof, such as a V sequence or a portion thereof, a D sequence or a portion thereof, or a J sequence or a portion thereof. In some embodiments, a probe can hybridize to a V sequence and a D sequence (or a portion thereof) or a D sequence and a J sequence (or a portion thereof). The probe may comprise a functional group (such as a biotin molecule) to enable purification of the hybridized target nucleic acid molecule (e.g., using streptavidin conjugated beads, such as magnetic beads). See, e.g., FIG. 19.

A nucleic acid primer used for enriching can be selected based on Rapid Amplification of cDNA Ends (RACE) sequencing. RACE sequencing can be a technique used to obtain a sequence (e.g., 5' RACE) of a nucleic acid (e.g., an RNA transcript), such as a nucleic acid (e.g., an RNA transcript) found within a cell. RACE sequencing can result in the production of a cDNA copy of a sequence of interest, produced through reverse transcription, followed by PCR amplification of the cDNA copies (see RT-PCR). The amplified cDNA copies can be sequenced and can map to a unique genomic region. In some embodiments, the RACE-products can be sequenced by next generation sequencing technologies.

In some embodiments, a method can further comprise cloning a modified enriched nucleic acid into a vector, such as a vector the modified enriched nucleic acid sequence is compatible with. Cloning can be performed using any acceptable method, including methods provided herein (e.g., in the cloning section).

Nucleic acid primers should not be interpreted to be specific to a particular nucleic acid strand. For example, in some embodiments, a first nucleic acid molecule can be complementary to a complement of an identification sequence as described herein. In some embodiments, a second nucleic acid molecule can be complementary to a binding sequence as designed herein.

In yet further embodiments of enriching for a nucleic acid sequence of interest, in which the nucleic acid sequence of interest is a BCR, or fragment thereof, the enrichment may be performed via first and second amplification reactions. The first reaction may be performed with first and second primers in which: (i) the first primer has a sequence complementary to at least a portion of the barcode sequence and/or the UMI sequence, and (ii) the second primer has a sequence complementary to a complement of at least a portion of the nucleic acid sequence of interest that encodes a junction (J) region and/or isotype region of the BCR or fragment thereof. The second reaction may be performed with third and fourth primers in which: (i) the third primer includes a sequence complementary to nucleotides of at least a portion of the leader sequence and/or encoding framework region (FWR)1 of the BCR, or fragment thereof, and (ii) the fourth primer includes a sequence complementary to a complement of at least a portion of the nucleic acid sequence of interest that encodes a complementarity region (CDR)3, FWR4, a J region, a D region, and/or a V region, or a junction between any one or more thereof, of the BCR or fragment thereof.

In certain embodiments of the method, in the first amplification reaction, the first primer may include a sequence complementary to at least a portion of the barcode sequence and the UMI sequence. In certain other embodiments, the first primer may include a sequence complementary to the barcode sequence and the UMI sequence. In some embodiments, the second primer may include a sequence complementary to the complement of the nucleic acid sequence of interest that encodes at least a portion of the J region of the BCR or fragment thereof. In other embodiments, the second primer may include a sequence complementary to the complement of the nucleic acid sequence of interest that encodes at least a portion of the isotype region of the BCR or fragment thereof. In yet other embodiments, the second primer may include a sequence complementary to the complement of the nucleic acid sequence of interest that encodes at least a portion of the J region and the isotype region of the BCR or fragment thereof. In certain embodiments of the method, in the first amplification reaction, the first primer may include a sequence complementary to at least a portion of the barcode sequence and the UMI sequence and the second primer may include a sequence complementary to the complement of the nucleic acid sequence of interest that encodes at least a portion of the J region and the isotype region of the BCR or fragment thereof.

In certain embodiments of the method, in the second amplification reaction, the third primer may include a sequence complementary to nucleotides of at least a portion of the leader sequence or encoding FWR1 of the BCR, or fragment thereof. In other certain embodiments of the methods, the third primer may include a sequence complementary to nucleotides encoding at least a portion of the FWR1 of the BCR, or fragment thereof. In other embodiments of the method, the fourth primer may include a sequence complementary to the complement of at least a portion of the nucleic acid sequence of interest that encodes the CDR3, and junction extending into the J region of the BCR or fragment thereof. In yet other embodiments of the methods, the fourth primer may include a sequence complementary to the complement of at least a portion of the nucleic acid sequence of interest that encodes the D and J regions, or a junction between the D and J regions of the BCR or fragment thereof. In yet still other embodiments of the methods, the fourth primer may include a sequence complementary to the complement of at least a portion of the nucleic acid sequence of interest that encodes the V and J regions, or a junction between the V and J regions, of the BCR or fragment thereof. In still other embodiments of the methods, the fourth primer may include a sequence complementary to the complement of at least a portion of the nucleic acid sequence of interest that encodes the V and D regions, or a junction between the V and D regions, of the BCR or fragment thereof. In yet a further embodiment of the methods, the fourth primer may include a sequence complementary to the complement of at least a portion of the nucleic acid sequence of interest that encodes the V, D and J regions of the BCR or fragment thereof. In certain embodiments of the method, in the second amplification reaction, the third primer may include a sequence complementary to nucleotides of at least a portion of the leader sequence of or encoding FWR1 the BCR, or fragment thereof and the fourth primer may include sequence complementary to the complement of at least a portion of the nucleic acid sequence of interest that encodes the CDR3, and junction extending into the J region of the BCR or fragment thereof.

In an embodiment of the method, the first amplification reaction may employ a first primer that includes a sequence complementary to at least a portion of the barcode sequence and the UMI sequence and a second primer that includes a sequence complementary to the complement of the nucleic acid sequence of interest that encodes at least a portion of the J region and the isotype region of the BCR or fragment thereof. The first amplification may be followed by a second amplification reaction that may employ a third primer that includes a sequence complementary to nucleotides of at least a portion of the leader sequence of or that encodes FWR1 of the BCR, or fragment thereof and the fourth primer may include a sequence complementary to the complement of at least a portion of the nucleic acid sequence of interest that encodes the CDR3, and junction extending into the J region of the BCR or fragment thereof.

_Modification of an enriched nucleic acid sequence of interest_

Further modification of a nucleic acid sequence of interest can be performed, for example after the nucleic acid sequence of interest has been enriched. In some embodiments, modification of a nucleic acid sequence of interest can be performed in preparation for analysis of the nucleic acid sequence of interest, to analyze the nucleic acid sequence of interest, or to prepare the nucleic acid sequence of interest for cloning.

Methods can further comprise performing fragmentation of a nucleic acid sequence of interest. Nucleic acid fragmentation (e.g. footprinting), such as by OH radicals, can be a tool to probe nucleic acid structure and nucleic acid-protein interactions. Such methods can provide structural information with single base pair resolution. Footprinting can refer to assays in which either the binding of a ligand to a specific sequence of bases or the conformation of the nucleic acid inhibits nicking of the phosphodiester backbone of nucleic acid polymer by a reagent. Intimate interactions between proteins and nucleic acids can be widely examined by footprinting methods. A prerequisite of such assays can be the ability to produce and detect high-quality nucleic acid fragmentation around the protein-protected areas. Nucleic acid fragmentation can be achieved by using a variety of enzymatic and chemical reagents. This can be highly related to the development of chemical hydroxyl radical footprinting using Fenton chemistry and peroxonitrous acid. Hydroxyl radicals can engender breaks of the phosphodiester backbone in a non-specific sequence manner and, hence, can be utilized for footprinting assays. Using hydroxyl radical methods over enzymatic footprinting can be advantageous because it can provide great sensitivity to nucleic acid structures, such as sequence-dependent curvature and RNA folding.

Methods can further comprise A-tailing of a nucleic acid sequence of interest. A- tailing can comprise an enzymatic method for adding a non-templated nucleotide to the 3' end of a blunt, double-stranded DNA molecule. A-tailing can be performed to prepare a T-vector for use in TA cloning or to A-tail a PCR product produced by a high-fidelity polymerase (e.g., other than Taq) for use in TA cloning. TA cloning can be a rapid method of cloning PCR products that can utilize stabilization of the single-base extension (adenosine) produced by Taq polymerase by the complementary T (thymidine) of the T-vector prior to ligation and transformation. This technique may not utilize restriction enzymes and PCR products can be used directly without modification. Additionally, in some embodiments PCR primers do not need to be designed with restriction sites, making the process less complicated. In some embodiments, A-tailing can be non-directional, meaning the insert can go into the vector in both orientations.

Methods can further comprise performing a sample index polymerase chain reaction (SI-PCR) on a nucleic acid sequence of interest. SI-PCR can utilize different pairs of index primers on a nucleic acid molecule. In some cases, index primers can beadded to individual samples in a second thermocycling step, for example after initial amplification of the target region. This can allow mixing of many samples together (e.g., up to 96) and simultaneous sequencing of the samples. Following sequencing, for example on an Illumina MiSeq, software can be able to identify these indexes on each sequence read, in some cases allowing separation of the reads for each different nucleic acid molecule.

Methods can further comprise V(D)J enrichment of a nucleic acid sequence of interest. This can be accomplished, for example, using PCR or another amplification method to amplify a V(D)J sequence or a fragment thereof from the enriched nucleic acid sequence of interest.

Modification of a nucleic acid sequence of interest or enriched nucleic acid sequence of interest can comprise addition of Gibson ends to said amplified nucleic acid sequences. Addition of Gibson ends (e.g., Gibson Assembly) can allow cloning or joining of two nucleic acid sequences without restriction sites. In some cases, addition of Gibson ends can allow joining of any two fragments regardless of sequence. Gibson assembly can be performed in a manner to leave no scar between joined nucleic acid sequence. Gibson assembly can be used to combine a plurality (e.g., 2, 3, 4, 5, 6, 7, 8, 9, 10, or more) of fragments. Gibson assembly can be performed, for example, as described in Gibson DG, Young L, Chuang RY, Venter JC, Hutchison CA 3rd, Smith HO. Enzymatic assembly of DNA molecules up to several hundred kilobases. Nat Methods. 2009;6(5):343-345. doi:10.1038/nmeth.1318.

Gibson assembly can simultaneously combine a plurality of DNA fragments, e.g., based on sequence identity. The DNA fragments contain an about 20-40 base pair overlap with adjacent DNA fragments. These DNA fragments can be mixed with one or more enzymes (e.g., a cocktail of 3 enzymes), along with other buffer components. In some embodiments, the enzymes can include an exonuclease, a DNA polymerase, and a DNA ligase.

During Gibson assembly, modification of a nucleic acid sequence of interest or enriched nucleic acid sequence of interest can comprise combining a second nucleic acid of interest with the nucleic acid of interest or enriched nucleic acid of interest. In some embodiments, the second nucleic acid sequence of interest can be enriched. Such combining can comprise, for example, using one or more overlap extension primers to link the nucleic acid sequence of interest or enriched nucleic acid sequence of interest to the second nucleic acid sequence of interest. In some cases, such case can comprise using a nucleic acid linker to join the second nucleic acid sequence of interest to the nucleic acid sequence of interest or the enriched nucleic acid sequence of interest.

During Gibson assembly, a second nucleic acid sequence of interest can be a nucleic acid sequence described herein. For example, a second nucleic acid sequence of interest can code for at least a portion of a cell surface protein of a cell, such as a T cell receptor (or fragment thereof) or a B cell receptor (or fragment thereof). In some cases, an enriched nucleic acid sequence of interest can comprise one chain of a T cell receptor (or fragment thereof) or first chain of a B cell receptor (or fragment thereof), while a second nucleic acid sequence of interest can comprise a second chain of a T cell receptor (or fragment thereof) or a B cell receptor (or fragment thereof). A second nucleic acid sequence of interest can comprise a constant region. In some embodiments, the second nucleic acid sequence of interest can comprise a sequence coding for a V(D)J sequence or a portion thereof, such as a V sequence (or portion thereof), a D sequence (or portion thereof), or a J sequence (or portion thereof), as described herein. In some embodiments, the constant region of a second nucleic acid sequence of interest can comprise a sequence coding for a V(D)J sequence or a portion thereof, such as a V sequence (or portion thereof), a D sequence (or portion thereof), or a J sequence (or portion thereof), as described herein. In some embodiments, a second nucleic acid sequence of interest can comprise a barcode (e.g., as provided herein), a UMI (e.g., as provided herein), or a 5' untranslated region (5' UTR). In some embodiments, the second nucleic acid sequence of interest can comprise complementary deoxyribonucleic acid (cDNA).

During Gibson assembly, the exonuclease can chew back DNA from the 5' end, and in some cases does not inhibit polymerase activity, thus allowing the reaction to occur in one single process. The resulting single-stranded regions on adjacent DNA fragments can anneal. The DNA polymerase can incorporate nucleotides to fill in any gaps. The DNA ligase can covalently join the DNA of adjacent segments, thereby removing any nicks in the DNA. Either linear or closed circular molecules can be assembled. In some embodiments, PCR can be utilized to perform the Gibson assembly.

*Cloning*

Existing antibody cloning methods can be time-consuming or difficult, and can require considerable automation and expensive plate-based reagents in order to succeed. Human labor can be used instead, but this can become impractical when cloning thousands of antibodies, which can become a common procedure, for example during pandemic antibody discovery efforts and during antibody discovery campaigns for pharmaceutical research. Methods for enriching nucleic acid sequences (including those coding for antibodies or fragments thereof) and methods for cloning those sequences provided herein can leverage cDNA and amplified sequences to efficiently recover targets of interest from one or more single cell libraries. By way of example, employing primer and probes as provided herein, it is possible to enrich specifically for particular BCR/antibody nucleic acid sequences of interest from complex, pooled, cDNA libraries. Primers and probes that target sequences encoding a BCR/antibody target of interest at, for instance, a junction leading to or including J region sequences, are demonstrated to selectively enrich for specific BCRs/antibodies, including from a library of pooled donor samples comprising B cells expressing numerous BCRs/antibodies of different sequences, e.g., from a sequencing library prepared from pooled donor samples comprising thousands (e.g., 5,000-10,000) of BCR sequences. Accordingly, using the approaches described herein, users can sequence thousands to hundreds of thousands of antibodies and target select antibodies for recovery and cloning. This can be particularly powerful when combined with other components provided herein (e.g., barcoding) to screen, e.g. for antigen specificity or other multiomic data.

Methods provided herein can further comprise cloning a nucleic acid sequence of interest into a vector. A vector can be a nucleic acid (e.g., DNA) molecule used as a vehicle to artificially carry foreign genetic material into a cell, where it can be replicated and/or expressed. Examples of vectors can include a viral vector, a plasmid, a bacteriophage, a cosmid, or an artificial chromosome.

In some embodiments, a vector can be modified by the addition of genetic material coding for a protein. For example, a vector can comprise a nucleic acid sequence that can be combined with the nucleic acid sequence of interest. For example, a vector can comprise a nucleic acid sequence that can be combined with the nucleic acid sequence of interest to yield a nucleic acid sequence for a protein of interest, such as an antibody or antigen binding fragment thereof, T cell receptor, or B cell receptor. For example, a vector can comprise at least a portion of a constant region of a T cell receptor, a B cell receptor, or an antibody or antigen binding fragment thereof.

In some embodiments, a vector can comprise a promoter. a promoter can be a sequence of DNA to which one or more proteins can bind that can initiate transcription of a single RNA from the DNA downstream of it. This RNA may encode a protein, or can have a function in and of itself, such as tRNA, mRNA, or rRNA. Promoters are located near the transcription start sites of genes, upstream on the DNA (towards the 5' region of the sense strand). Promoters can be about 100-1000 base pairs long. Examples of promoters can include bacterial promoters or eukaryotic promoters.

In some embodiments, cloning can comprise a vector restriction digest (e.g., cutting of the nucleic acid sequence of the vector at a restriction site, or site recognized by a restriction enzyme). A restriction digest of a vector can comprise digesting the vector at a restriction site. A restriction site can be a DNA sequence on the vector that can contain a specific sequence of nucleotides (e.g., 4-8 bases long) that can be recognized by a restriction enzyme. In some embodiments, a restriction site can be a palindromic sequence. In some embodiments, a restriction enzyme (e.g., a restriction enzyme that can recognize the restriction site) can cut the sequence between two nucleic acids within the restriction site or nearby the restriction site. An example of a restriction site can be, for example, a fspI restriction site that can be recognized by the fspI restriction enzyme. Non-limiting examples of restriction sites that can be employed are provided in *Table 1*.

*Table 1: Restriction enzymes and their recognition sequences*

| Enzyme | Source | Recognition Sequence | Cut |
|---|---|---|---|
| EcoRI | Escherichia coli | 5'GAATTC | 5'---G AATTC---3' |
| | | 3'CTTAAG | 3'---CTTAA G---5' |
| EcoRII | Escherichia coli | 5'CCWGG | 5'--- CCWGG---3' |
| | | 3'GGWCC | 3'---GGWCC ---5' |
| BamHI | Bacillus amyloliquefaciens | 5'GGATCC | 5'---G GATCC---3' |
| | | 3'CCTAGG | 3'---CCTAG G---5' |
| HindIII | Haemophilus influenzae | 5'AAGCTT | 5'---A AGCTT---3' |
| | | 3'TTCGAA | 3'---TTCGA A---5' |
| TaqI | Thermus aquaticus | 5'TCGA | 5'---T CGA---3' |
| | | 3'AGCT | 3'---AGC T---5' |
| NotI | Nocardia otitidis | 5'GCGGCCGC | 5'---GC GGCCGC---3' |
| | | 3'CGCCGGCG | 3'---CGCCGG CG---5' |
| HinFI | Haemophilus influenzae | 5'GANTC | 5'---G ANTC---3' |
| | | 3'CTNAG | 3'---CTNA G---5' |
| Sau3AI | Staphylococcus aureus | 5'GATC | 5'--- GATC---3' |
| | | 3'CTAG | 3'---CTAG ---5' |
| PvuII | Proteus vulgaris | 5'CAGCTG | 5'---CAG CTG---3' |
| | | 3'GTCGAC | 3'---GTC GAC---5' |
| SmaI | Serratia marcescens | 5'CCCGGG | 5'---CCC GGG---3' |
| | | 3'GGGCCC | 3'---GGG CCC---5' |

(continued)

| Enzyme | Source | Recognition Sequence | Cut |
|---|---|---|---|
| HaeIII | Haemophilus aegyptius | 5'GGCC | 5'---GG CC---3' |
| | | 3'CCGG | 3'---CC GG---5' |
| HgaI | Haemophilus gallinarum | 5'GACGC | 5'---NN NN---3' |
| | | 3'CTGCG | 3'---NN NN---5' |
| AluI | Arthrobacter luteus | 5'AGCT | 5'---AG CT---3' |
| | | 3'TCGA | 3'---TC GA---5' |
| EcoRV | Escherichia coli | 5'GATATC | 5'---GAT ATC---3' |
| | | 3'CTATAG | 3'---CTA TAG---5' |
| EcoP15I | Escherichia coli | 5'CAGCAGN$_{25}$NN | 5'---CAGCAGN$_{25}$ NN-3' |
| | | 3'GTCGTCN$_{25}$NN | 3'---GTCGTCN$_{25}$NN-5' |
| KpnI | Klebsiella pneumoniae | 5'GGTACC | 5'---GGTAC C---3' |
| | | 3'CCATGG | 3'---C CATGG---5' |
| PstI | Providencia stuartii | 5'CTGCAG | 5'---CTGCA G---3' |
| | | 3'GACGTC | 3'---G ACGTC---5' |
| SacI | Streptomyces achromogenes | 5'GAGCTC | 5'---GAGCT C---3' |
| | | 3'CTCGAG | 3'---C TCGAG---5' |
| SalI | Streptomyces albus | 5'GTCGAC | 5'---G TCGAC---3' |
| | | 3'CAGCTG | 3'---CAGCT G---5' |
| ScaI | Streptomyces caespitosus | 5'AGTACT | 5'---AGT ACT---3' |
| | | 3'TCATGA | 3'---TCA TGA---5' |
| SpeI | Sphaerotilus natans | 5'ACTAGT | 5'---A CTAGT---3' |
| | | 3'TGATCA | 3'---TGATC A---5' |
| SphI | Streptomyces phaeochromogenes | 5'GCATGC | 5'---GCATG C---3' |
| | | 3'CGTACG | 3'---C GTACG---5' |
| StuI | Streptomyces tubercidicus | 5'AGGCCT | 5'---AGG CCT---3' |
| | | 3'TCCGGA | 3'---TCC GGA---5' |
| XbaI | Xanthomonas badrii | 5'TCTAGA | 5'---T CTAGA---3' |
| | | 3'AGATCT | 3'---AGATC T---5' |

A cloning vector can have features that can allow a gene to be conveniently inserted into the vector or removed from it. Examples can include a multiple cloning site (MCS) or polylinker, which can contain unique restriction site(s). The restriction site(s) in the MCS can be first cleaved by restriction enzymes, then a PCR-amplified target gene, e.g. nucleic acid sequence of interest, also digested with the same enzymes is ligated into the vectors using DNA ligase. It can be inserted into the vector in a specific direction if so desired. The restriction sites may be further used for sub-cloning into another vector if necessary.

Other cloning vectors may employ topoisomerase instead of ligase, and cloning can be performed more rapidly without the need for restriction digest of the vector or insert. In this TOPO cloning method, a linearized vector can be activated by attaching topoisomerase I to its ends, and this "TOPO-activated" vector may then accept a PCR product by ligating both the 5' ends of the PCR product, releasing the topoisomerase and forming a circular vector in the process. Another method of cloning without the use of DNA digest and ligase can be by DNA recombination, for example as used in the Gateway cloning system. The gene, once cloned into the cloning vector, may be conveniently introduced into a variety of expression vectors by recombination.

A vector can comprise a reporter gene. A reporter gene can be used in some cloning vectors to facilitate the screening of successful clones by using features of these genes that allow successful clone to be easily identified. Such features can include the lacZα fragment for α complementation in blue-white selection, and/or marker gene or reporter genes in frame with and flanking the MCS to facilitate the production of fusion proteins. Examples of fusion partners that may be used for screening can include the green fluorescent protein (GFP) and luciferase.

In some embodiments, cloning can comprise combining two or more nucleic acid sequences. For example, two or more nucleic acid sequences can be joined to yield a coding sequence for an amino acid sequence of interest (e.g., a T cell receptor, a B cell receptor, or an antibody or antigen binding fragment thereof). Two or more nucleic acid sequences can comprise a nucleic acid sequence of a heavy chain of an antibody or antigen binding fragment and a nucleic acid sequence of a light chain. Two or more nucleic acid sequences can comprise a nucleic acid sequence of an alpha chain of a T cell receptor and a nucleic acid sequence of a beta chain of a T cell receptor. In such a method, a full antibody or antigen binding fragment thereof, B cell receptor, T cell receptor or other amino acid can be cloned in a single vector and expressed as a single nucleic acid sequence or amino acid sequence.

After cloning, the nucleic acid sequence of interest or the amino acid product of the nucleic acid sequence of interest can be expressed. Expression can be performed in any acceptable expression system, including a bacterial expression system, a yeast expression system, an insect cell expression system, a viral expression system, or a mammalian cell expression system. In some embodiments, expression can be in a live animal.

The protein product of the nucleic acid sequence of interest can be analyzed. For example, the affinity, specificity, enzymatic activity, solubility, stability, or other property of the protein product can be analyzed. Examples of assays can include ELISA, western blot, enzymatic assay, dot blot, Bradford protein assay, neutralization assay, immunoassay, or another assay.

## Systems and methods for sample compartmentalization

In an aspect, the systems and methods described herein provide for the compartmentalization, depositing, or partitioning of one or more particles (e.g., biological particles, macromolecular constituents of biological particles, beads, reagents, etc.) into discrete compartments or partitions (referred to interchangeably herein as partitions), where each partition maintains separation of its own contents from the contents of other partitions. The partition can be a droplet in an emulsion. A partition may comprise one or more other partitions.

A partition may include one or more particles. A partition may include one or more types of particles. For example, a partition of the present disclosure may comprise one or more biological particles and/or macromolecular constituents thereof. A partition may comprise one or more gel beads. A partition may comprise one or more cell beads. A partition may include a single gel bead, a single cell bead, or both a single cell bead and single gel bead. A partition may include one or more reagents. Alternatively, a partition may be unoccupied. For example, a partition may not comprise a bead. A cell bead can be a biological particle and/or one or more of its macromolecular constituents encased inside of a gel or polymer matrix, such as via polymerization of a droplet containing the biological particle and precursors capable of being polymerized or gelled. Unique identifiers, such as barcodes, may be injected into the droplets previous to, subsequent to, or concurrently with droplet generation, such as via a microcapsule (e.g., bead), as described elsewhere herein. Microfluidic channel networks (e.g., on a chip) can be utilized to generate partitions as described herein. Alternative mechanisms may also be employed in the partitioning of individual biological particles, including porous membranes through which aqueous mixtures of cells are extruded into non-aqueous fluids.

The partitions can be flowable within fluid streams. The partitions may comprise, for example, micro-vesicles that have an outer barrier surrounding an inner fluid center or core. In some cases, the partitions may comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In some examples, the partitions may be provided in a water-in-oil emulsion or oil-in-water emulsion. A variety of different vessels are described in, for example, U.S. Patent Application Publication No. 2014/0155295. Emulsion systems for creating stable droplets in non-aqueous or oil continuous phases are described in, for example, U.S. Patent Application Publication No. 2010/0105112.

In the case of droplets in an emulsion, allocating individual particles to discrete partitions may in one non-limiting example be accomplished by introducing a flowing stream of particles in an aqueous fluid into a flowing stream of a non-aqueous fluid, such that droplets are generated at the junction of the two streams. Fluid properties (e.g., fluid flow rates, fluid viscosities, etc.), particle properties (e.g., volume fraction, particle size, particle concentration, etc.), microfluidic architectures (e.g., channel geometry, etc.), and other parameters may be adjusted to control the occupancy of the resulting partitions (e.g., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions may contain less than one biological particle per partition in order to ensure that those partitions that are occupied are primarily singly occupied. In some cases, partitions among a plurality of partitions may contain at most one biological particle (e.g., bead, DNA, cell or cellular material). In some embodiments, the various parameters (e.g., fluid properties, particle properties, microfluidic architectures, etc.) may be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

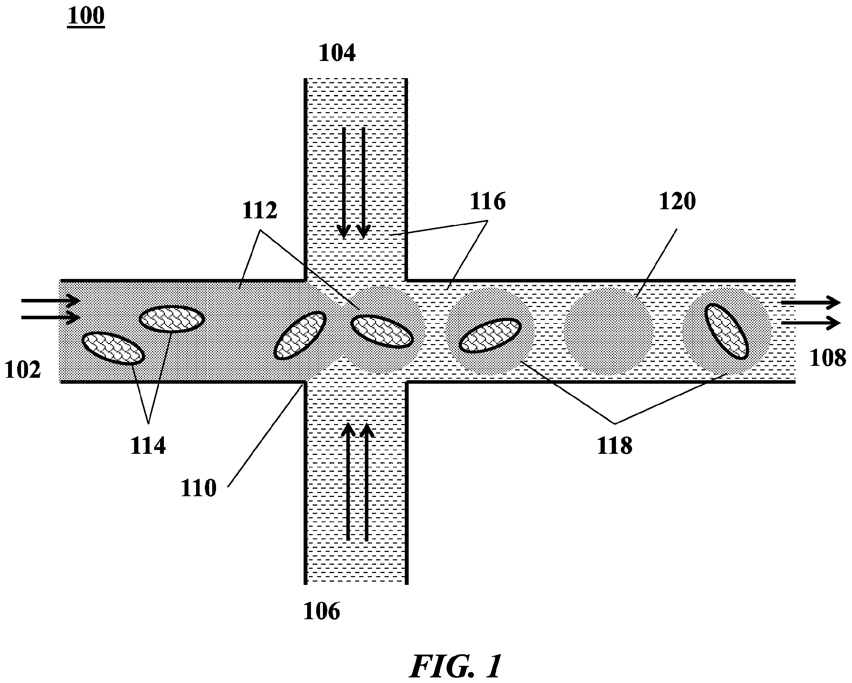

**FIG. 1** shows an example of a microfluidic channel structure **100** for partitioning individual biological particles. The channel structure **100** can include channel segments **102, 104, 106** and **108** communicating at a channel junction **110.** In operation, a first aqueous fluid **112** that includes suspended biological particles (or cells) **114** may be transported along channel segment **102** into junction **110,** while a second fluid **116** that is immiscible with the aqueous fluid **112** is delivered to the junction **110** from each of channel segments **104** and **106** to create discrete droplets **118, 120** of the first aqueous fluid **112** flowing into channel segment **108,** and flowing away from junction **110.** The channel segment **108** may be fluidically coupled to an outlet reservoir where the discrete droplets can be stored and/or harvested. A discrete droplet generated may include an individual biological particle **114** (such as droplets **118**). A discrete droplet generated may include more than one individual biological particle **114** (not shown in **FIG. 1**). A discrete droplet may contain no biological particle **114** (such as droplet **120**). Each discrete partition may maintain separation of its own contents (e.g., individual biological particle **114**) from the contents of other partitions.

The second fluid **116** can comprise an oil, such as a fluorinated oil, that includes a fluorosurfactant for stabilizing the resulting droplets, for example, inhibiting subsequent coalescence of the resulting droplets **118, 120.** Examples of particularly useful partitioning fluids and fluorosurfactants are described, for example, in U.S. Patent Application Publication No. 2010/0105112.

As will be appreciated, the channel segments described herein may be coupled to any of a variety of different fluid sources or receiving components, including reservoirs, tubing, manifolds, or fluidic components of other systems. As will be appreciated, the microfluidic channel structure **100** may have other geometries. For example, a microfluidic channel structure can have more than one channel junction. For example, a microfluidic channel structure can have 2, 3, 4, or 5 channel segments each carrying particles (e.g., biological particles, cell beads, and/or gel beads) that meet at a channel junction. Fluid may be directed to flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

The generated droplets may comprise two subsets of droplets: (1) occupied droplets **118,** containing one or more biological particles **114,** and (2) unoccupied droplets **120,** not containing any biological particles **114.** Occupied droplets **118** may comprise singly occupied droplets (having one biological particle) and multiply occupied droplets (having more than one biological particle). As described elsewhere herein, in some cases, the majority of occupied partitions can include no more than one biological particle per occupied partition and some of the generated partitions can be unoccupied (of any biological particle). In some cases, though, some of the occupied partitions may include more than one biological particle. In some cases, the partitioning process may be controlled such that fewer than about 25% of the occupied partitions contain more than one biological particle, and in many cases, fewer than about 20% of the occupied partitions have more than one biological particle, while in some cases, fewer than about 10% or even fewer than about 5% of the occupied partitions include more than one biological particle per partition.

In some cases, it may be desirable to minimize the creation of excessive numbers of empty partitions, such as to reduce costs and/or increase efficiency. While this minimization may be achieved by providing a sufficient number of biological particles (e.g., biological particles **114**) at the partitioning junction **110,** such as to ensure that at least one biological particle is encapsulated in a partition, the Poissonian distribution may expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, the flow of one or more of the biological particles (e.g., in channel segment **102**), or other fluids directed into the partitioning junction (e.g., in channel segments **104, 106)** can be controlled such that, in many cases, no more than about 50% of the generated partitions, no more than about 25% of the generated partitions, or no more than about 10% of the generated partitions are unoccupied. These flows can be controlled so as to present a non-Poissonian distribution of single-occupied partitions while providing lower levels of unoccupied partitions. The above noted ranges of unoccupied partitions can be achieved while still providing any of the single occupancy rates described above. For example, in many cases, the use of the systems and methods described herein can create resulting partitions that have multiple occupancy rates of less than about 25%, less than about 20%, less than about 15%, less than about 10%, and in many cases, less than about 5%, while having unoccupied partitions of less than about 50%, less than about 40%, less than about 30%, less than about 20%, less than about 10%, less than about 5%, or less.

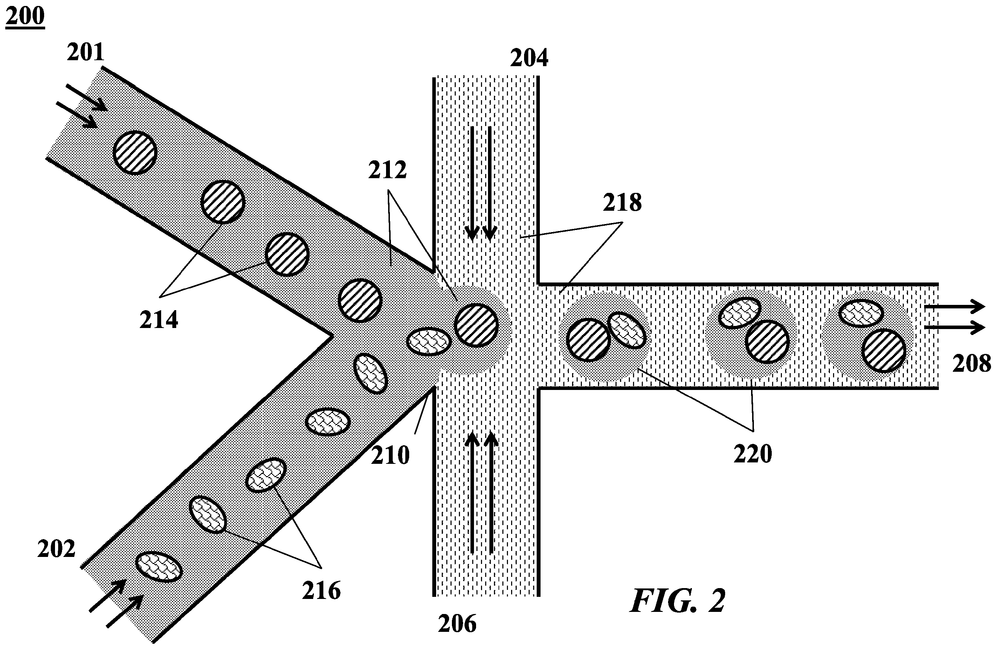

As will be appreciated, the above-described occupancy rates are also applicable to partitions that include both biological particles and additional reagents, including, but not limited to, microcapsules or beads (e.g., gel beads) carrying barcoded nucleic acid molecules (e.g., oligonucleotides) (described in relation to **FIG. 2**). The occupied partitions (e.g., at least about 10%, 20%, 30%, 40%, 50%, 60%, 70%, 80%, 90%, 95%, or 99% of the occupied partitions) can include both a microcapsule (e.g., bead) comprising barcoded nucleic acid molecules and a biological particle.

In another aspect, in addition to or as an alternative to droplet based partitioning, biological particles may be encapsulated within a microcapsule that comprises an outer shell, layer or porous matrix in which is entrained one or more individual biological particles or small groups of biological particles. In some embodiments, biological particles (e.g., cells) are comprised within (e.g., encapsulated within) a particulate material to form a "cell bead". The microcapsule (e.g., cell bead) may include other reagents. Encapsulation of biological particles may be performed by a variety of processes. Such processes may combine an aqueous fluid containing the biological particles with a polymeric precursor material that may be capable of being formed into a gel or other solid or semi-solid matrix upon application of a particular stimulus to the polymer precursor. Such stimuli can include, for example, thermal stimuli (e.g., either heating or cooling), photo-stimuli (e.g., through photo-curing), chemical stimuli (e.g., through crosslinking, polymerization initiation of the precursor (e.g., through added initiators)), mechanical stimuli, or a combination thereof.

Preparation of microcapsules comprising biological particles may be performed by a variety of methods. For example, air knife droplet or aerosol generators may be used to dispense droplets of precursor fluids into gelling solutions in order to form microcapsules that include individual biological particles or small groups of biological particles. Likewise, membrane based encapsulation systems may be used to generate microcapsules comprising encapsulated biological particles as described herein. Microfluidic systems of the present disclosure, such as that shown in **FIG. 1**, may be readily used in encapsulating cells as described herein. In particular, and with reference to **FIG. 1**, the aqueous fluid **112** comprising (i) the biological particles **114** and (ii) the polymer precursor material (not shown) is flowed into channel junction **110**, where it is partitioned into droplets **118, 120** through the flow of non-aqueous fluid **116**. In the case of encapsulation methods, non-aqueous fluid **116** may also include an initiator (not shown) to cause polymerization and/or crosslinking of the polymer precursor to form the microcapsule that includes the entrained biological particles. Examples of polymer precursor/initiator pairs include those described in U.S. Patent Application Publication No. 2014/0378345.

For example, in the case where the polymer precursor material comprises a linear polymer material, such as a linear polyacrylamide, PEG, or other linear polymeric material, the activation agent may comprise a cross-linking agent, or a chemical that activates a cross-linking agent within the formed droplets. Likewise, for polymer precursors that comprise polymerizable monomers, the activation agent may comprise a polymerization initiator. For example, in certain cases, where the polymer precursor comprises a mixture of acrylamide monomer with a N,N'-bis-(acryloyl)cystamine (BAC) comonomer, an agent such as tetraethylmethylenediamine (TEMED) may be provided within the second fluid streams **116** in channel segments **104** and **106**, which can initiate the copolymerization of the acrylamide and BAC into a cross-linked polymer network, or hydrogel.

Upon contact of the second fluid stream **116** with the first fluid stream **112** at junction **110**, during formation of droplets, the TEMED may diffuse from the second fluid **116** into the aqueous fluid **112** comprising the linear polyacrylamide, which will activate the crosslinking of the polyacrylamide within the droplets **118, 120**, resulting in the formation of gel (e.g., hydrogel) microcapsules, as solid or semi-solid beads or particles entraining the cells **114**. Although described in terms of polyacrylamide encapsulation, other 'activatable' encapsulation compositions may also be employed in the context of the methods and compositions described herein. For example, formation of alginate droplets followed by exposure to divalent metal ions (e.g., $Ca^{2+}$ ions), can be used as an encapsulation process using the described processes. Likewise, agarose droplets may also be transformed into capsules through temperature based gelling (e.g., upon cooling, etc.).

In some cases, encapsulated biological particles can be selectively releasable from the microcapsule, such as through passage of time or upon application of a particular stimulus, that degrades the microcapsule sufficiently to allow the biological particles (e.g., cell), or its other contents to be released from the microcapsule, such as into a partition (e.g., droplet). For example, in the case of the polyacrylamide polymer described above, degradation of the microcapsule may be accomplished through the introduction of an appropriate reducing agent, such as DTT or the like, to cleave disulfide bonds that cross-link the polymer matrix. See, for example, U.S. Patent Application Publication No. 2014/0378345.

The biological particle can be subjected to other conditions sufficient to polymerize or gel the precursors. The conditions sufficient to polymerize or gel the precursors may comprise exposure to heating, cooling, electromagnetic radiation, and/or light. The conditions sufficient to polymerize or gel the precursors may comprise any conditions sufficient to polymerize or gel the precursors. Following polymerization or gelling, a polymer or gel may be formed around the biological particle. The polymer or gel may be diffusively permeable to chemical or biochemical reagents. The polymer or gel may be diffusively impermeable to macromolecular constituents of the biological particle. In this manner, the polymer or gel may act to allow the biological particle to be subjected to chemical or biochemical operations while spatially confining the macromolecular constituents to a region of the droplet defined by the polymer or gel. The polymer or gel may include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel may comprise any other polymer or gel.

The polymer or gel may be functionalized to bind to targeted analytes, such as nucleic acids, proteins, carbohydrates, lipids or other analytes. The polymer or gel may be polymerized or gelled via a passive mechanism. The polymer or gel may be stable in alkaline conditions or at elevated temperature. The polymer or gel may have mechanical properties similar to the mechanical properties of the bead. For instance, the polymer or gel may be of a similar size to the bead. The polymer or gel may have a mechanical strength (e.g. tensile strength) similar to that of the bead. The polymer or gel may be of a lower density than an oil. The polymer or gel may be of a density that is roughly similar to that of a buffer. The polymer or gel may have a tunable pore size. The pore size may be chosen to, for instance, retain denatured nucleic acids. The pore size may be chosen to maintain diffusive permeability to exogenous chemicals such as sodium hydroxide (NaOH) and/or endogenous chemicals such as inhibitors. The polymer or gel may be biocompatible. The polymer or gel may maintain or enhance cell viability. The polymer or gel may be biochemically compatible. The polymer or gel may be polymerized and/or depolymerized thermally, chemically, enzymatically, and/or optically.

The polymer may comprise poly(acrylamide-co-acrylic acid) crosslinked with disulfide linkages. The preparation of the polymer may comprise a two-step reaction. In the first activation step, poly(acrylamide-co-acrylic acid) may be exposed to an acylating agent to convert carboxylic acids to esters. For instance, the poly(acrylamide-co-acrylic acid) may be exposed to 4-(4,6-dimethoxy-1,3,5-triazin-2-yl)-4-methylmorpholinium chloride (DMTMM). The polyacrylamide-co-acrylic acid may be exposed to other salts of 4-(4,6-dimethoxy-1,3,5-triazin-2-yl)-4-methylmorpholinium. In the second cross-linking step, the ester formed in the first step may be exposed to a disulfide crosslinking agent. For instance, the ester may be exposed to cystamine (2,2'-dithiobis(ethylamine)). Following the two steps, the biological particle may be surrounded by polyacrylamide strands linked together by disulfide bridges. In this manner, the biological particle may be encased inside of or comprise a gel or matrix (e.g., polymer matrix) to form a "cell bead." A cell bead can contain biological particles (e.g., a cell) or macromolecular constituents (e.g., RNA, DNA, proteins, etc.) of biological particles. A cell bead may include a single cell or multiple cells, or a derivative of the single cell or multiple cells. For example after lysing and washing the cells, inhibitory components from cell lysates can be washed away and the macromolecular constituents can be bound as cell beads. Systems and methods disclosed herein can be applicable to both cell beads (and/or droplets or other partitions) containing biological particles and cell beads (and/or droplets or other partitions) containing macromolecular constituents of biological particles.

Encapsulated biological particles can provide certain potential advantages of being more storable and more portable than droplet-based partitioned biological particles. Furthermore, in some cases, it may be desirable to allow biological particles to incubate for a select period of time before analysis, such as in order to characterize changes in such biological particles over time, either in the presence or absence of different stimuli. In such cases, encapsulation may allow for longer incubation than partitioning in emulsion droplets, although in some cases, droplet partitioned biological particles may also be incubated for different periods of time, e.g., at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of biological particles may constitute the partitioning of the biological particles into which other reagents are co-partitioned. Alternatively or in addition, encapsulated biological particles may be readily deposited into other partitions (e.g., droplets) as described above.

## Beads

A partition may comprise one or more unique identifiers, such as barcodes. Barcodes may be previously, subsequently or concurrently delivered to the partitions that hold the compartmentalized or partitioned biological particle. For example, barcodes may be injected into droplets previous to, subsequent to, or concurrently with droplet generation. The delivery of the barcodes to a particular partition allows for the later attribution of the characteristics of the individual biological particle to the particular partition. Barcodes may be delivered, for example on a nucleic acid molecule (e.g., an oligonucleotide), to a partition via any suitable mechanism. Barcoded nucleic acid molecules, e.g., barcode containing oligonucleotides, can be delivered to a partition via a microcapsule. A microcapsule, in some instances, can comprise a bead. Beads are described in further detail below.

In some cases, barcoded nucleic acid molecules, e.g., barcode containing oligonucleotides, can be initially associated with the microcapsule and then released from the microcapsule. Release of the barcoded nucleic acid molecules, e.g., barcode containing oligonucleotides, can be passive (e.g., by diffusion out of the microcapsule). In addition or alternatively, release from the microcapsule can be upon application of a stimulus which allows the barcoded nucleic acid nucleic acid molecules, e.g., barcode containing oligonucleotides, to dissociate or to be released from the microcapsule. Such stimulus may disrupt the microcapsule, an interaction that couples the barcoded nucleic acid molecules, e.g., barcode containing oligonucleotides, to or within the microcapsule, or both. Such stimulus can include, for example, a thermal stimulus, photo-stimulus, chemical stimulus (e.g., change in pH or use of a reducing agent(s)), a mechanical stimulus, a radiation stimulus; a biological stimulus (e.g., enzyme), or any combination thereof.

**FIG. 2** shows an example of a microfluidic channel structure **200** for delivering barcode carrying beads to droplets. The channel structure **200** can include channel segments **201, 202, 204, 206** and **208** communicating at a channel junction **210.** In operation, the channel segment **201** may transport an aqueous fluid **212** that includes a plurality of beads **214** (e.g., with nucleic acid molecules, oligonucleotides, molecular tags) along the channel segment **201** into junction **210.** The plurality of beads **214** may be sourced from a suspension of beads. For example, the channel segment **201** may be connected to a reservoir comprising an aqueous suspension of beads **214.** The channel segment **202** may transport the aqueous fluid **212** that includes a plurality of biological particles **216** along the channel segment **202** into junction **210.** The plurality of biological particles **216** may be sourced from a suspension of biological particles. For example, the channel segment **202** may be connected to a reservoir comprising an aqueous suspension of biological particles **216.** In some instances, the aqueous fluid **212** in either the first channel segment **201** or the second channel segment **202,** or in both segments, can include one or more reagents, as further described below. A second fluid **218** that is immiscible with the aqueous fluid **212** (e.g., oil) can be delivered to the junction **210** from each of channel segments **204** and **206.** Upon meeting of the aqueous fluid **212** from each of channel segments **201** and **202** and the second fluid **218** from each of channel segments **204** and **206** at the channel junction **210,** the aqueous fluid **212** can be partitioned as discrete droplets **220** in the second fluid **218** and flow away from the junction **210** along channel segment **208.** The channel segment **208** may deliver the discrete droplets to an outlet reservoir fluidly coupled to the channel segment **208,** where they may be harvested.

As an alternative, the channel segments **201** and **202** may meet at another junction upstream of the junction **210.** At such junction, beads and biological particles may form a mixture that is directed along another channel to the junction **210** to yield droplets **220.** The mixture may provide the beads and biological particles in an alternating fashion, such that, for example, a droplet comprises a single bead and a single biological particle.

Beads, biological particles and droplets may flow along channels at substantially regular flow profiles (e.g., at regular flow rates). Such regular flow profiles may permit a droplet to include a single bead and a single biological particle. Such regular flow profiles may permit the droplets to have an occupancy (e.g., droplets having beads and biological particles) greater than 5%, 10%, 20%, 30%, 40%, 50%, 60%, 70%, 80%, 90%, or 95%. Such regular flow profiles and devices that may be used to provide such regular flow profiles are provided in, for example, U.S. Patent Publication No. 2015/0292988.

The second fluid **218** can comprise an oil, such as a fluorinated oil, that includes a fluorosurfactant for stabilizing the resulting droplets, for example, inhibiting subsequent coalescence of the resulting droplets **220.**

A discrete droplet that is generated may include an individual biological particle **216.** A discrete droplet that is generated may include a barcode or other reagent carrying bead **214.** A discrete droplet generated may include both an individual biological particle and a barcode carrying bead, such as droplets **220.** In some instances, a discrete droplet may include more than one individual biological particle or no biological particle. In some instances, a discrete droplet may include more than one bead or no bead. A discrete droplet may be unoccupied (e.g., no beads, no biological particles).

Beneficially, a discrete droplet partitioning a biological particle and a barcode carrying bead may effectively allow the attribution of the barcode to macromolecular constituents of the biological particle within the partition. The contents of a partition may remain discrete from the contents of other partitions.

As will be appreciated, the channel segments described herein may be coupled to any of a variety of different fluid sources or receiving components, including reservoirs, tubing, manifolds, or fluidic components of other systems. As will be appreciated, the microfluidic channel structure **200** may have other geometries. For example, a microfluidic channel structure can have more than one channel junctions. For example, a microfluidic channel structure can have 2, 3, 4, or 5 channel segments each carrying beads that meet at a channel junction. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

A bead may be porous, non-porous, solid, semi-solid, semi-fluidic, fluidic, and/or a combination thereof. In some instances, a bead may be dissolvable, disruptable, and/or degradable. In some cases, a bead may not be degradable. In some cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the bead may be a silica bead. In some cases, the bead can be rigid. In other cases, the bead may be flexible and/or compressible.

A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In some cases, the diameter of a bead may be at least about 10 nanometers (nm), 100 nm, 500 nm, 1 micrometer ($\mu$m), 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or greater. In some cases, a bead may have a diameter of less than about 10 nm, 100 nm, 500 nm, 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or less. In some cases, a bead may have a diameter in the range of about 40-75$\mu$m, 30-75$\mu$m, 20-75$\mu$m, 40-85$\mu$m, 40-95$\mu$m, 20-100$\mu$m, 10-100$\mu$m, 1-100$\mu$m, 20-250$\mu$m, or 20-500$\mu$m.

In certain aspects, beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their cross-sectional dimensions of less than 50%, less than 40%, less than 30%, less than 20%, and in some cases less than 15%, less than 10%, less than 5%, or less.

A bead may comprise natural and/or synthetic materials. For example, a bead can comprise a natural polymer, a synthetic polymer or both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, Corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and/or combinations (e.g., co-polymers) thereof. Beads may also be formed from materials other than polymers, including lipids, micelles, ceramics, glass-ceramics, material composites, metals, other inorganic materials, and others.

In some instances, the bead may contain molecular precursors (e.g., monomers or polymers), which may form a polymer network via polymerization of the molecular precursors. In some cases, a precursor may be an already polymerized species capable of undergoing further polymerization via, for example, a chemical cross-linkage. In some cases, a precursor can comprise one or more of an acrylamide or a methacrylamide monomer, oligomer, or polymer. In some cases, the bead may comprise prepolymers, which are oligomers capable of further polymerization. For example, polyurethane beads may be prepared using prepolymers. In some cases, the bead may contain individual polymers that may be further polymerized together. In some cases, beads may be generated via polymerization of different precursors, such that they comprise mixed polymers, co-polymers, and/or block co-polymers. In some cases, the bead may comprise covalent or ionic bonds between polymeric precursors (e.g., monomers, oligomers, linear polymers), nucleic acid molecules (e.g., oligonucleotides), primers, and other entities. In some cases, the covalent bonds can be carbon-carbon bonds, thioether bonds, or carbon-heteroatom bonds.

In some cases, a plurality of nucleic acid barcode molecules may be attached to a bead. The nucleic acid barcode molecules may be attached directly or indirectly to the bead. In some cases, the nucleic acid barcode molecules may be covalently linked to the bead. In some cases, the nucleic acid barcode molecules are covalently linked to the bead via a linker. In some cases, the linker is a degradable linker. In some cases, the linker comprises a labile bond configured to release said nucleic acid barcode molecule of said plurality of nucleic acid barcode molecules. In some cases, the labile bond comprises a disulfide linkage.

Cross-linking may be permanent or reversible, depending upon the particular cross-linker used. Reversible cross-linking may allow for the polymer to linearize or dissociate under appropriate conditions. In some cases, reversible cross-linking may also allow for reversible attachment of a material bound to the surface of a bead. In some cases, a cross-linker may form disulfide linkages. In some cases, the chemical cross-linker forming disulfide linkages may be cystamine or a modified cystamine.

In some cases, disulfide linkages can be formed between molecular precursor units (e.g., monomers, oligomers, or linear polymers) or precursors incorporated into a bead and nucleic acid molecules (e.g., oligonucleotides). Cystamine (including modified cystamines), for example, is an organic agent comprising a disulfide bond that may be used as a crosslinker agent between individual monomeric or polymeric precursors of a bead. Polyacrylamide may be polymerized in the presence of cystamine or a species comprising cystamine (e.g., a modified cystamine) to generate polyacrylamide gel beads comprising disulfide linkages (e.g., chemically degradable beads comprising chemically-reducible crosslinkers). The disulfide linkages may permit the bead to be degraded (or dissolved) upon exposure of the bead to a reducing agent.

In some cases, chitosan, a linear polysaccharide polymer, may be crosslinked with glutaraldehyde via hydrophilic chains to form a bead. Crosslinking of chitosan polymers may be achieved by chemical reactions that are initiated by heat, pressure, change in pH, and/or radiation.

In some cases, a bead may comprise an acrydite moiety, which in certain aspects may be used to attach one or more nucleic acid molecules (e.g., barcode sequence, barcoded nucleic acid molecule, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as a nucleic acid molecule (e.g., barcode sequence, barcoded nucleic acid molecule, barcoded oligonucleotide, primer, or other oligonucleotide). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment can be reversible, such that when the disulfide bond is broken (e.g., in the presence of a reducing agent), the attached species is released from the bead. In other cases, an acrydite moiety can comprise a reactive hydroxyl group that may be used for attachment.

Functionalization of beads for attachment of nucleic acid molecules (e.g., oligonucleotides) may be achieved through a wide range of different approaches, including activation of chemical groups within a polymer, incorporation of active or activatable functional groups in the polymer structure, or attachment at the pre-polymer or monomer stage in bead production.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule (e.g., oligonucleotide), which may include a priming sequence (e.g., a primer for amplifying target nucleic acids, random primer, primer sequence for messenger RNA) and/or one or more barcode sequences. The one more barcode sequences may include sequences that are the same for all nucleic acid molecules coupled to a given bead and/or sequences that are different across all nucleic acid molecules coupled to the given bead. The nucleic acid molecule may be incorporated into the bead.

In some cases, the nucleic acid molecule can comprise a functional sequence, for example, for attachment to a sequencing flow cell, such as, for example, a P5 sequence for Illumina® sequencing. In some cases, the nucleic acid molecule or derivative thereof (e.g., oligonucleotide or polynucleotide generated from the nucleic acid molecule) can comprise another functional sequence, such as, for example, a P7 sequence for attachment to a sequencing flow cell for Illumina sequencing. In some cases, the nucleic acid molecule can comprise a barcode sequence. In some cases, the primer can further comprise a unique molecular identifier (UMI). In some cases, the primer can comprise an R1 primer sequence for Illumina sequencing. In some cases, the primer can comprise an R2 primer sequence for Illumina sequencing. Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609.

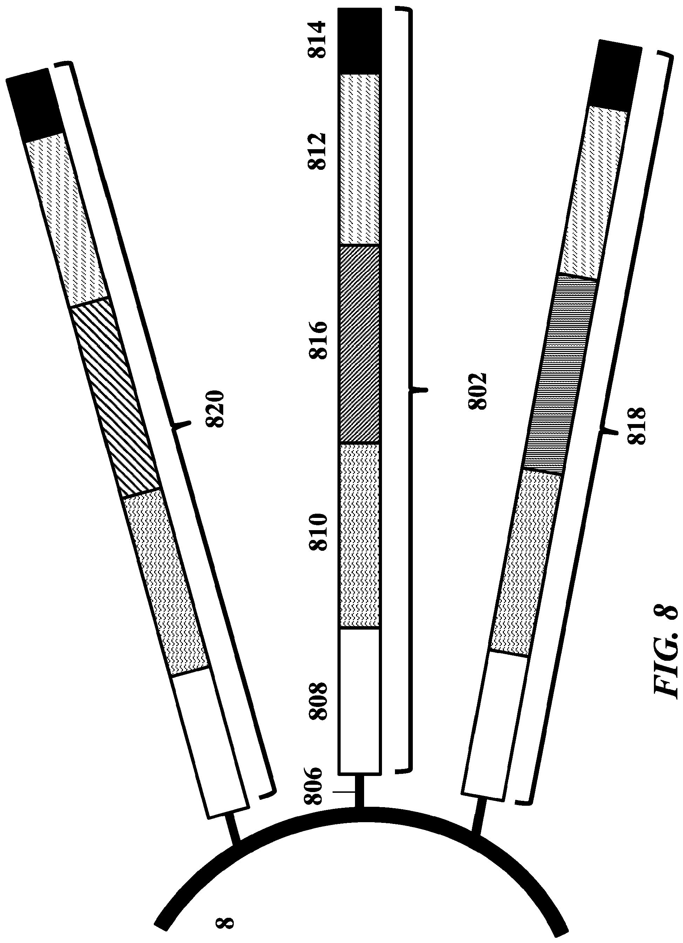

**FIG. 8** illustrates an example of a barcode carrying bead. A nucleic acid molecule **802,** e.g., a nucleic acid barcode molecule such as an oligonucleotide, can be coupled to a bead **804** by a releasable linkage **806,** such as, for example, a disulfide linker. The same bead **804** may be coupled (e.g., via releasable linkage) to one or more other nucleic acid molecules **818, 820.** The nucleic acid molecule **802** may be or comprise a barcode. As noted elsewhere herein, the structure of the barcode may comprise a number of sequence elements. The nucleic acid molecule **802** may comprise a functional sequence **808** that may be used in subsequent processing. For example, the functional sequence **808** may include one or more of a sequencer specific flow cell attachment sequence (e.g., a P5 sequence for Illumina® sequencing systems) and a sequencing primer sequence (e.g., a R1 primer for Illumina® sequencing systems). The nucleic acid molecule **802** may comprise a barcode sequence **810** for use in barcoding the sample (e.g., DNA, RNA, protein, etc.). In some cases, the barcode sequence **810** can be bead-specific such that the barcode sequence **810** is common to all nucleic acid molecules (e.g., including nucleic acid molecule **802**) coupled to the same bead **804.** Alternatively or in addition, the barcode sequence **810** can be partition-specific such that the barcode sequence **810** is common to all nucleic acid molecules coupled to one or more beads that are partitioned into the same partition. The nucleic acid molecule **802** may comprise a specific priming sequence **812,** such as an mRNA specific priming sequence (e.g., poly-T sequence), a targeted priming sequence, and/or a random priming sequence. The nucleic acid molecule **802** may comprise an anchoring sequence **814** to ensure that the specific priming sequence **812** hybridizes at the sequence end (e.g., of the mRNA). For example, the anchoring sequence **814** can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid molecule **802** may comprise a unique molecular identifying sequence **816** (e.g., unique molecular identifier (UMI)). In some cases, the unique molecular identifying sequence **816** may comprise from about 5 to about 8 nucleotides. Alternatively, the unique molecular identifying sequence **816** may compress less than about 5 or more than about 8 nucleotides. The unique molecular identifying sequence **816** may be a unique sequence that varies across individual nucleic acid molecules (e.g., **802, 818, 820,** etc.) coupled to a single bead (e.g., bead **804**). In some cases, the unique molecular identifying sequence **816** may be a random sequence (e.g., such as a random N-mer sequence). For example, the UMI may provide a unique identifier of the starting mRNA molecule that was captured, in order to allow quantitation of the number of original expressed RNA. As will be appreciated, although **FIG. 8** shows three nucleic acid molecules **802, 818, 820** coupled to the surface of the bead **804,** an individual bead may be coupled to any number of individual nucleic acid molecules, for example, from one to tens to hundreds of thousands or even millions of individual nucleic acid molecules. The respective barcodes for the individual nucleic acid molecules can comprise both common sequence segments or relatively common sequence segments (e.g., **808, 810, 812,** etc.) and variable or unique sequence segments (e.g., **816**) between different individual nucleic acid molecules coupled to the same bead.

In operation, a biological particle (e.g., cell, DNA, RNA, etc.) can be co-partitioned along with a barcode bearing bead **804.** The barcoded nucleic acid molecules **802, 818, 820** can be released from the bead **804** in the partition. By way of example, in the context of analyzing sample RNA, the poly-T segment (e.g., **812**) of one of the released nucleic acid molecules (e.g., **802**) can hybridize to the poly-A tail of a mRNA molecule. Reverse transcription may result in a cDNA transcript of the mRNA, but which transcript includes each of the sequence segments **808, 810, 816** of the nucleic acid molecule **802.** Because the nucleic acid molecule **802** comprises an anchoring sequence **814,** it will more likely hybridize to and prime reverse transcription at the sequence end of the poly-A tail of the mRNA. Within any given partition, all of the cDNA transcripts of the individual mRNA molecules may include a common barcode sequence segment **810**. However, the transcripts made from the different mRNA molecules within a given partition may vary at the unique molecular identifying sequence **812** segment (e.g., UMI segment). Beneficially, even following any subsequent amplification of the contents of a given partition, the number of different UMIs can be indicative of the quantity of mRNA originating from a given partition, and thus from the biological particle (e.g., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences may also be used in priming the reverse transcription reaction. Likewise, although described as releasing the barcoded oligonucleotides into the partition, in some cases, the nucleic acid molecules bound to the bead (e.g., gel bead) may be used to hybridize and capture the mRNA on the solid phase of the bead, for example, in order to facilitate the separation of the RNA from other cell contents.

In some cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. For example, some precursors comprising a carboxylic acid (COOH) group can copolymerize with other precursors to form a gel bead that also comprises a COOH functional group. In some cases, acrylic acid (a species comprising free COOH groups), acrylamide, and bis(acryloyl)cystamine can be co-polymerized together to generate a gel bead comprising free COOH groups. The COOH groups of the gel bead can be activated (e.g., via 1-Ethyl-3-(3-dimethylaminopropyl)carbodiimide (EDC) and N-Hydroxysuccinimide (NHS) or 4-(4,6-Dimethoxy-1,3,5-triazin-2-yl)-4-methylmorpholinium chloride (DMTMM)) such that they are reactive (e.g., reactive to amine functional groups where EDC/NHS or DMTMM are used for activation). The activated COOH groups can then react with an appropriate species (e.g., a species comprising an amine functional group where the carboxylic acid groups are activated to be reactive with an amine functional group) comprising a moiety to be linked to the bead.

Beads comprising disulfide linkages in their polymeric network may be functionalized with additional species via reduction of some of the disulfide linkages to free thiols. The disulfide linkages may be reduced via, for example, the action of a reducing agent (e.g., DTT, TCEP, etc.) to generate free thiol groups, without dissolution of the bead. Free thiols of the beads can then react with free thiols of a species or a species comprising another disulfide bond (e.g., via thiol-disulfide exchange) such that the species can be linked to the beads (e.g., via a generated disulfide bond). In some cases, free thiols of the beads may react with any other suitable group. For example, free thiols of the beads may react with species comprising an acrydite moiety. The free thiol groups of the beads can react with the acrydite via Michael addition chemistry, such that the species comprising the acrydite is linked to the bead. In some cases, uncontrolled reactions can be prevented by inclusion of a thiol capping agent such as N-ethylmalieamide or iodoacetate.

Activation of disulfide linkages within a bead can be controlled such that only a small number of disulfide linkages are activated. Control may be exerted, for example, by controlling the concentration of a reducing agent used to generate free thiol groups and/or concentration of reagents used to form disulfide bonds in bead polymerization. In some cases, a low concentration (e.g., molecules of reducing agent:gel bead ratios of less than or equal to about 1:100,000,000,000, less than or equal to about 1:10,000,000,000, less than or equal to about 1:1,000,000,000, less than or equal to about 1:100,000,000, less than or equal to about 1:10,000,000, less than or equal to about 1:1,000,000, less than or equal to about 1:100,000, less than or equal to about 1:10,000) of reducing agent may be used for reduction. Controlling the number of disulfide linkages that are reduced to free thiols may be useful in ensuring bead structural integrity during functionalization. In some cases, optically-active agents, such as fluorescent dyes may be coupled to beads via free thiol groups of the beads and used to quantify the number of free thiols present in a bead and/or track a bead.

In some cases, addition of moieties to a gel bead after gel bead formation may be advantageous. For example, addition of an oligonucleotide (e.g., barcoded oligonucleotide) after gel bead formation may avoid loss of the species during chain transfer termination that can occur during polymerization. Moreover, smaller precursors (e.g., monomers or cross linkers that do not comprise side chain groups and linked moieties) may be used for polymerization and can be minimally hindered from growing chain ends due to viscous effects. In some cases, functionalization after gel bead synthesis can minimize exposure of species (e.g., oligonucleotides) to be loaded with potentially damaging agents (e.g., free radicals) and/or chemical environments. In some cases, the generated gel may possess an upper critical solution temperature (UCST) that can permit temperature driven swelling and collapse of a bead. Such functionality may aid in oligonucleotide (e.g., a primer) infiltration into the bead during subsequent functionalization of the bead with the oligonucleotide. Post-production functionalization may also be useful in controlling loading ratios of species in beads, such that, for example, the variability in loading ratio is minimized. Species loading may also be performed in a batch process such that a plurality of beads can be functionalized with the species in a single batch.

A bead injected or otherwise introduced into a partition may comprise releasably, cleavably, or reversibly attached barcodes. A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to, or as an alternative to the cleavable linkages between the beads and the associated molecules, such as barcode containing nucleic acid molecules (e.g., barcoded oligonucleotides), the beads may be degradable, disruptable, or dissolvable spontaneously or upon exposure to one or more stimuli (e.g., temperature changes, pH changes, exposure to particular chemical species or phase, exposure to light, reducing agent, etc.). In some cases, a bead may be dissolvable, such that material components of the beads are solubilized when exposed to a particular chemical species or an environmental change, such as a change temperature or a change in pH. In some cases, a gel bead can be degraded or dissolved at elevated temperature and/or in basic conditions. In some cases, a bead may be thermally degradable such that when the bead is exposed to an appropriate change in temperature (e.g., heat), the bead degrades. Degradation or dissolution of a bead bound to a species (e.g., a nucleic acid molecule, e.g., barcoded oligonucleotide) may result in release of the species from the bead.

As will be appreciated from the above disclosure, the degradation of a bead may refer to the disassociation of a bound or entrained species from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. For example, a polyacrylamide bead comprising cystamine and linked, via a disulfide bond, to a barcode sequence, may be combined with a reducing agent within a droplet of a water-in-oil emulsion. Within the droplet, the reducing agent can break the various disulfide bonds, resulting in bead degradation and release of the barcode sequence into the aqueous, inner environment of the droplet. In another example, heating of a droplet comprising a bead-bound barcode sequence in basic solution may also result in bead degradation and release of the attached barcode sequence into the aqueous, inner environment of the droplet.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the predefined concentration of the primer can be limited by the process of producing nucleic acid molecule (e.g., oligonucleotide) bearing beads.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads may be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. For instance, the transfer may be achieved by moving the beads from one co-flowing solvent stream to a different co-flowing solvent stream. The swellability and/or pore size of the beads may be adjusted by changing the polymer composition of the bead.

In some cases, an acrydite moiety linked to a precursor, another species linked to a precursor, or a precursor itself can comprise a labile bond, such as chemically, thermally, or photosensitive bond e.g., disulfide bond, UV sensitive bond, or the like. Once acrydite moieties or other moieties comprising a labile bond are incorporated into a bead, the bead may also comprise the labile bond. The labile bond may be, for example, useful in reversibly linking (e.g., covalently linking) species (e.g., barcodes, primers, etc.) to a bead. In some cases, a thermally labile bond may include a nucleic acid hybridization based attachment, e.g., where an oligonucleotide is hybridized to a complementary sequence that is attached to the bead, such that thermal melting of the hybrid releases the oligonucleotide, e.g., a barcode containing sequence, from the bead or microcapsule.

The addition of multiple types of labile bonds to a gel bead may result in the generation of a bead capable of responding to varied stimuli. Each type of labile bond may be sensitive to an associated stimulus (e.g., chemical stimulus, light, temperature, enzymatic, etc.) such that release of species attached to a bead via each labile bond may be controlled by the application of the appropriate stimulus. Such functionality may be useful in controlled release of species from a gel bead. In some cases, another species comprising a labile bond may be linked to a gel bead after gel bead formation via, for example, an activated functional group of the gel bead as described above. As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that may be coupled to a precursor or bead include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)). A bond may be cleavable via other nucleic acid molecule targeting enzymes, such as restriction enzymes (e.g., restriction endonucleases), as described further below.

Species may be encapsulated in beads during bead generation (e.g., during polymerization of precursors). Such species may or may not participate in polymerization. Such species may be entered into polymerization reaction mixtures such that generated beads comprise the species upon bead formation. In some cases, such species may be added to the gel beads after formation. Such species may include, for example, nucleic acid molecules (e.g., oligonucleotides), reagents for a nucleic acid amplification reaction (e.g., primers, polymerases, dNTPs, co-factors (e.g., ionic co-factors), buffers) including those described herein, reagents for enzymatic reactions (e.g., enzymes, co-factors, substrates, buffers), reagents for nucleic acid modification reactions such as polymerization, ligation, or digestion, and/or reagents for template preparation (e.g., tagmentation) for one or more sequencing platforms (e.g., Nextera® for Illumina®). Such species may include one or more enzymes described herein, including without limitation, polymerase, reverse transcriptase, restriction enzymes (e.g., endonuclease), transposase, ligase, proteinase K, DNAse, etc. Such species may include one or more reagents described elsewhere herein (e.g., lysis agents, inhibitors, inactivating agents, chelating agents, stimulus). Trapping of such species may be controlled by the polymer network density generated during polymerization of precursors, control of ionic charge within the gel bead (e.g., via ionic species linked to polymerized species), or by the release of other species. Encapsulated species may be released from a bead upon bead degradation and/or by application of a stimulus capable of releasing the species from the bead. Alternatively or in addition, species may be partitioned in a partition (e.g., droplet) during or subsequent to partition formation. Such species may include, without limitation, the abovementioned species that may also be encapsulated in a bead.

A degradable bead may comprise one or more species with a labile bond such that, when the bead/species is exposed to the appropriate stimuli, the bond is broken and the bead degrades. The labile bond may be a chemical bond (e.g., covalent bond, ionic bond) or may be another type of physical interaction (e.g., van der Waals interactions, dipole-dipole interactions, etc.). In some cases, a crosslinker used to generate a bead may comprise a labile bond. Upon exposure to the appropriate conditions, the labile bond can be broken and the bead degraded. For example, upon exposure of a polyacrylamide gel bead comprising cystamine crosslinkers to a reducing agent, the disulfide bonds of the cystamine can be broken and the bead degraded.

A degradable bead may be useful in more quickly releasing an attached species (e.g., a nucleic acid molecule, a barcode sequence, a primer, etc) from the bead when the appropriate stimulus is applied to the bead as compared to a bead that does not degrade. For example, for a species bound to an inner surface of a porous bead or in the case of an encapsulated species, the species may have greater mobility and accessibility to other species in solution upon degradation of the bead. In some cases, a species may also be attached to a degradable bead via a degradable linker (e.g., disulfide linker). The degradable linker may respond to the same stimuli as the degradable bead or the two degradable species may respond to different stimuli. For example, a barcode sequence may be attached, via a disulfide bond, to a polyacrylamide bead comprising cystamine. Upon exposure of the barcoded-bead to a reducing agent, the bead degrades and the barcode sequence is released upon breakage of both the disulfide linkage between the barcode sequence and the bead and the disulfide linkages of the cystamine in the bead.

As will be appreciated from the above disclosure, while referred to as degradation of a bead, in many instances as noted above, that degradation may refer to the disassociation of a bound or entrained species from a bead, both with and without structurally degrading the physical bead itself. For example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

Where degradable beads are provided, it may be beneficial to avoid exposing such beads to the stimulus or stimuli that cause such degradation prior to a given time, in order to, for example, avoid premature bead degradation and issues that arise from such degradation, including for example poor flow characteristics and aggregation. By way of example, where beads comprise reducible cross-linking groups, such as disulfide groups, it will be desirable to avoid contacting such beads with reducing agents, e.g., DTT or other disulfide cleaving reagents. In such cases, treatment to the beads described herein will, in some cases be provided free of reducing agents, such as DTT. Because reducing agents are often provided in commercial enzyme preparations, it may be desirable to provide reducing agent free (or DTT free) enzyme preparations in treating the beads described herein. Examples of such enzymes include, e.g., polymerase enzyme preparations, reverse transcriptase enzyme preparations, ligase enzyme preparations, as well as many other enzyme preparations that may be used to treat the beads described herein. The terms "reducing agent free" or "DTT free" preparations can refer to a preparation having less than about 1/10th, less than about 1/50th, or even less than about 1/100th of the lower ranges for such materials used in degrading the beads. For example, for DTT, the reducing agent free preparation can have less than about 0.01 millimolar (mM), 0.005 mM, 0.001 mM DTT, 0.0005 mM DTT, or even less than about 0.0001 mM DTT. In many cases, the amount of DTT can be undetectable.

Numerous chemical triggers may be used to trigger the degradation of beads. Examples of these chemical changes may include, but are not limited to pH-mediated changes to the integrity of a component within the bead, degradation of a component of a bead via cleavage of cross-linked bonds, and depolymerization of a component of a bead.

In some embodiments, a bead may be formed from materials that comprise degradable chemical crosslinkers, such as BAC or cystamine. Degradation of such degradable crosslinkers may be accomplished through a number of mechanisms. In some examples, a bead may be contacted with a chemical degrading agent that may induce oxidation, reduction or other chemical changes. For example, a chemical degrading agent may be a reducing agent, such as dithiothreitol (DTT). Additional examples of reducing agents may include β-mercaptoethanol, (2S)-2-amino-1,4-dimercaptobutane (dithiobutylamine or DTBA), tris(2-carboxyethyl) phosphine (TCEP), or combinations thereof. A reducing agent may degrade the disulfide bonds formed between gel precursors forming the bead, and thus, degrade the bead. In other cases, a change in pH of a solution, such as an increase in pH, may trigger degradation of a bead. In other cases, exposure to an aqueous solution, such as water, may trigger hydrolytic degradation, and thus degradation of the bead. In some cases, any combination of stimuli may trigger degradation of a bead. For example, a change in pH may enable a chemical agent (e.g., DTT) to become an effective reducing agent.

Beads may also be induced to release their contents upon the application of a thermal stimulus. A change in temperature can cause a variety of changes to a bead. For example, heat can cause a solid bead to liquefy. A change in heat may cause melting of a bead such that a portion of the bead degrades. In other cases, heat may increase the internal pressure of the bead components such that the bead ruptures or explodes. Heat may also act upon heat-sensitive polymers used as materials to construct beads.

Any suitable agent may degrade beads. In some embodiments, changes in temperature or pH may be used to degrade thermo-sensitive or pH-sensitive bonds within beads. In some embodiments, chemical degrading agents may be used to degrade chemical bonds within beads by oxidation, reduction or other chemical changes. For example, a chemical degrading agent may be a reducing agent, such as DTT, wherein DTT may degrade the disulfide bonds formed between a crosslinker and gel precursors, thus degrading the bead. In some embodiments, a reducing agent may be added to degrade the bead, which may or may not cause the bead to release its contents. Examples of reducing agents may include dithiothreitol (DTT), β-mercaptoethanol, (2S)-2-amino-1,4-dimercaptobutane (dithiobutylamine or DTBA), tris(2-carboxyethyl) phosphine (TCEP), or combinations thereof. The reducing agent may be present at a concentration of about 0.1mM, 0.5mM, 1mM, 5mM, 10mM. The reducing agent may be present at a concentration of at least about 0.1mM, 0.5mM, 1mM, 5mM, 10mM, or greater than 10 mM. The reducing agent may be present at concentration of at most about 10mM, 5mM, 1mM, 0.5mM, 0.1mM, or less.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the predefined concentration of the primer can be limited by the process of producing oligonucleotide bearing beads.

Although **FIG. 1** and **FIG. 2** have been described in terms of providing substantially singly occupied partitions, above, in certain cases, it may be desirable to provide multiply occupied partitions, e.g., containing two, three, four or more cells and/or microcapsules (e.g., beads) comprising barcoded nucleic acid molecules (e.g., oligonucleotides) within a single partition. Accordingly, as noted above, the flow characteristics of the biological particle and/or bead containing fluids and partitioning fluids may be controlled to provide for such multiply occupied partitions. In particular, the flow parameters may be controlled to provide a given occupancy rate at greater than about 50% of the partitions, greater than about 75%, and in some cases greater than about 80%, 90%, 95%, or higher.

In some cases, additional microcapsules (e.g., beads) can be used to deliver additional reagents to a partition. In such cases, it may be advantageous to introduce different beads into a common channel or droplet generation junction, from different bead sources (e.g., containing different associated reagents) through different channel inlets into such common channel or droplet generation junction (e.g., junction **210**). In such cases, the flow and frequency of the different beads into the channel or junction may be controlled to provide for a certain ratio of microcapsules from each source, while ensuring a given pairing or combination of such beads into a partition with a given number of biological particles (e.g., one biological particle and one bead per partition).

The partitions described herein may comprise small volumes, for example, less than about 10 microliters ($\mu$L), 5$\mu$L, 1$\mu$L, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet based partitions, the droplets may have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with microcapsules (e.g., beads), it will be appreciated that the sample fluid volume, e.g., including co-partitioned biological particles and/or beads, within the partitions may be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (e.g., empty partitions) and occupied partitions.

## Reagents

In accordance with certain aspects, biological particles may be partitioned along with lysis reagents in order to release the contents of the biological particles within the partition. In such cases, the lysis agents can be contacted with the biological particle suspension concurrently with, or immediately prior to, the introduction of the biological particles into the partitioning junction/droplet generation zone (e.g., junction **210**), such as through an additional channel or channels upstream of the channel junction. In accordance with other aspects, additionally or alternatively, biological particles may be partitioned along with other reagents, as will be described further below.

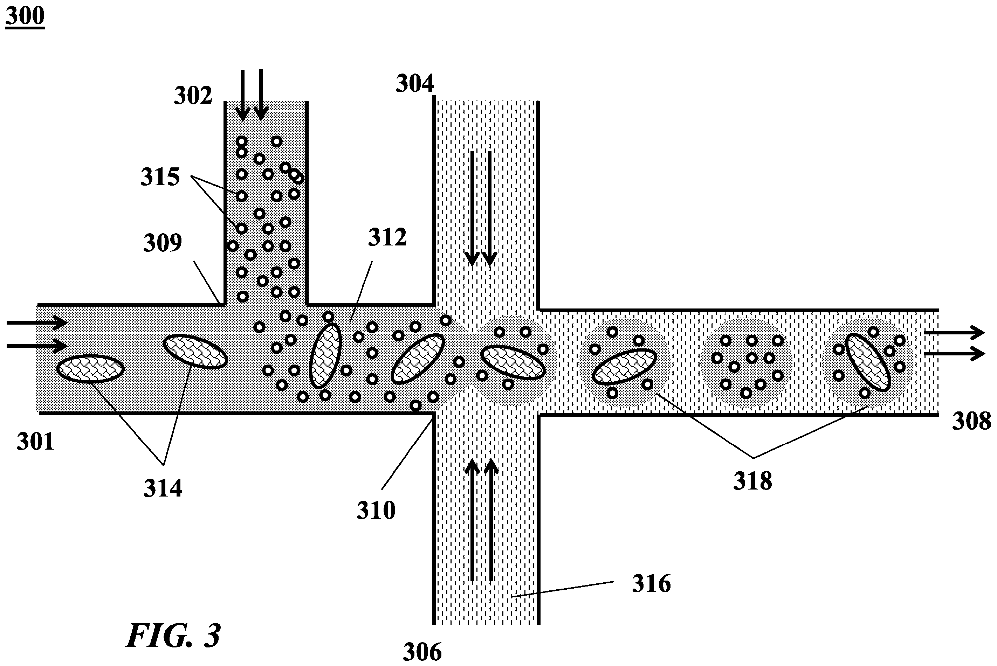

**FIG. 3** shows an example of a microfluidic channel structure 300 for co-partitioning biological particles and reagents. The channel structure **300** can include channel segments **301, 302, 304, 306** and **308**. Channel segments **301** and **302** communicate at a first channel junction **309**. Channel segments **302, 304, 306,** and **308** communicate at a second channel junction **310**.

In an example operation, the channel segment **301** may transport an aqueous fluid **312** that includes a plurality of biological particles **314** along the channel segment **301** into the second junction **310**. As an alternative or in addition to, channel segment **301** may transport beads (e.g., gel beads). The beads may comprise barcode molecules.

For example, the channel segment **301** may be connected to a reservoir comprising an aqueous suspension of biological particles **314**. Upstream of, and immediately prior to reaching, the second junction **310,** the channel segment **301** may meet the channel segment **302** at the first junction **309.** The channel segment **302** may transport a plurality of reagents **315** (e.g., lysis agents) suspended in the aqueous fluid **312** along the channel segment **302** into the first junction **309.** For example, the channel segment **302** may be connected to a reservoir comprising the reagents **315.** After the first junction **309,** the aqueous fluid **312** in the channel segment **301** can carry both the biological particles **314** and the reagents **315** towards the second junction **310.** In some instances, the aqueous fluid **312** in the channel segment **301** can include one or more reagents, which can be the same or different reagents as the reagents **315.** A second fluid **316** that is immiscible with the aqueous fluid **312** (e.g., oil) can be delivered to the second junction **310** from each of channel segments **304** and **306.** Upon meeting of the aqueous fluid **312** from the channel segment **301** and the second fluid **316** from each of channel segments **304** and **306** at the second channel junction **310,** the aqueous fluid **312** can be partitioned as discrete droplets **318** in the second fluid **316** and flow away from the second junction **310** along channel segment **308.** The channel segment **308** may deliver the discrete droplets **318** to an outlet reservoir fluidly coupled to the channel segment **308,** where they may be harvested.

The second fluid **316** can comprise an oil, such as a fluorinated oil, that includes a fluorosurfactant for stabilizing the resulting droplets, for example, inhibiting subsequent coalescence of the resulting droplets **318.**

A discrete droplet generated may include an individual biological particle **314** and/or one or more reagents **315.** In some instances, a discrete droplet generated may include a barcode carrying bead (not shown), such as via other microfluidics structures described elsewhere herein. In some instances, a discrete droplet may be unoccupied (e.g., no reagents, no biological particles).

Beneficially, when lysis reagents and biological particles are co-partitioned, the lysis reagents can facilitate the release of the contents of the biological particles within the partition. The contents released in a partition may remain discrete from the contents of other partitions.

As will be appreciated, the channel segments described herein may be coupled to any of a variety of different fluid sources or receiving components, including reservoirs, tubing, manifolds, or fluidic components of other systems. As will be appreciated, the microfluidic channel structure **300** may have other geometries. For example, a microfluidic channel structure can have more than two channel junctions. For example, a microfluidic channel structure can have 2, 3, 4, 5 channel segments or more each carrying the same or different types of beads, reagents, and/or biological particles that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the biological particles to cause the release of the biological particle's contents into the partitions. For example, in some cases, surfactant-based lysis solutions may be used to lyse cells, although these may be less desirable for emulsion based systems where the surfactants can interfere with stable emulsions. In some cases, lysis solutions may include non-ionic surfactants such as, for example, TritonX-100 and Tween 20. In some cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion based partitioning such as encapsulation of biological particles that may be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles, the biological particles may be exposed to an appropriate stimulus to release the biological particles or their contents from a co-partitioned microcapsule. For example, in some cases, a chemical stimulus may be co-partitioned along with an encapsulated biological particle to allow for the degradation of the microcapsule and release of the cell or its contents into the larger partition. In some cases, this stimulus may be the same as the stimulus described elsewhere herein for release of nucleic acid molecules (e.g., oligonucleotides) from their respective microcapsule (e.g., bead). In alternative aspects, this may be a different and non-overlapping stimulus, in order to allow an encapsulated biological particle to be released into a partition at a different time from the release of nucleic acid molecules into the same partition.

Additional reagents may also be co-partitioned with the biological particles, such as endonucleases to fragment a biological particle's DNA, DNA polymerase enzymes and dNTPs used to amplify the biological particle's nucleic acid fragments and to attach the barcode molecular tags to the amplified fragments. Other enzymes may be co-partitioned, including without limitation, polymerase, transposase, ligase, proteinase K, DNAse, etc. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching. In some cases, template switching can be used to increase the length of a cDNA. In some cases, template switching can be used to append a predefined nucleic acid sequence to the cDNA. In an example of template switching, cDNA can be generated from reverse transcription of a template, e.g., cellular mRNA, where a reverse transcriptase with terminal transferase activity can add additional nucleotides, e.g., polyC, to the cDNA in a template independent manner. Switch oligos can include sequences complementary to the additional nucleotides, e.g., polyG. The additional nucleotides (e.g., polyC) on the cDNA can hybridize to the additional nucleotides (e.g., polyG) on the switch oligo, whereby the switch oligo can be used by the reverse transcriptase as template to further extend the cDNA. Template switching oligonucleotides may comprise a hybridization region and a template region. The hybridization region can comprise any sequence capable of hybridizing to the target. In some cases, as previously described, the hybridization region comprises a series of G bases to complement the overhanging C bases at the 3' end of a cDNA molecule. The series of G bases may comprise 1 G base, 2 G bases, 3 G bases, 4 G bases, 5 G bases or more than 5 G bases. The template sequence can comprise any sequence to be incorporated into the cDNA. In some cases, the template region comprises at least 1 (e.g., at least 2, 3, 4, 5 or more) tag sequences and/or functional sequences. Switch oligos may comprise deoxyribonucleic acids; ribonucleic acids; modified nucleic acids including 2-Aminopurine, 2,6-Diaminopurine (2-Amino-dA), inverted dT, 5-Methyl dC, 2'-deoxyInosine, Super T (5-hydroxybutynl-2'-deoxyuridine), Super G (8-aza-7-deaza-guanosine), locked nucleic acids (LNAs), unlocked nucleic acids (UNAs, e.g., UNA-A, UNA-U, UNA-C, UNA-G), Iso-dG, Iso-dC, 2' Fluoro bases (e.g., Fluoro C, Fluoro U, Fluoro A, and Fluoro G), or any combination.

In some cases, the length of a switch oligo may be at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides or longer.

In some cases, the length of a switch oligo may be at most about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides.

Once the contents of the cells are released into their respective partitions, the macromolecular components (e.g., macromolecular constituents of biological particles, such as RNA, DNA, or proteins) contained therein may be further processed within the partitions. In accordance with the methods and systems described herein, the macromolecular component contents of individual biological particles can be provided with unique identifiers such that, upon characterization of those macromolecular components they may be attributed as having been derived from the same biological particle or particles. The ability to attribute characteristics to individual biological particles or groups of biological particles is provided by the assignment of unique identifiers specifically to an individual biological particle or groups of biological particles. Unique identifiers, e.g., in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles.

In some aspects, this is performed by co-partitioning the individual biological particle or groups of biological particles with the unique identifiers, such as described above (with reference to FIG. 2). In some aspects, the unique identifiers are provided in the form of nucleic acid molecules (e.g., oligonucleotides) that comprise nucleic acid barcode sequences that may be attached to or otherwise associated with the nucleic acid contents of individual biological particle, or to other components of the biological particle, and particularly to fragments of those nucleic acids. The nucleic acid molecules are partitioned such that as between nucleic acid molecules in a given partition, the nucleic acid barcode sequences contained therein are the same, but as between different partitions, the nucleic acid molecule can, and do have differing barcode sequences, or at least represent a large number of different barcode sequences across all of the partitions in a given analysis. In some aspects, only one nucleic acid barcode sequence can be associated with a given partition, although in some cases, two or more different barcode sequences may be present.

The nucleic acid barcode sequences can include from about 6 to about 20 or more nucleotides within the sequence of the nucleic acid molecules (e.g., oligonucleotides). The nucleic acid barcode sequences can include from about 6 to about 20, 30, 40, 50, 60, 70, 80, 90, 100 or more nucleotides. In some cases, the length of a barcode sequence may be about 6, 7, 8, 9, 10, 11, 12, 13, 14, 15, 16, 17, 18, 19, 20 nucleotides or longer. In some cases, the length of a barcode sequence may be at least about 6, 7, 8, 9, 10, 11, 12, 13, 14, 15, 16, 17, 18, 19, 20 nucleotides or longer. In some cases, the length of a barcode sequence may be at most about 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be about 4, 5, 6, 7, 8, 9, 10, 11, 12, 13, 14, 15, 16 nucleotides or longer. In some cases, the barcode subsequence may be at least about 4, 5, 6, 7, 8, 9, 10, 11, 12, 13, 14, 15, 16 nucleotides or longer. In some cases, the barcode subsequence may be at most about 4, 5, 6, 7, 8, 9, 10, 11, 12, 13, 14, 15, 16 nucleotides or shorter.

The co-partitioned nucleic acid molecules can also comprise other functional sequences useful in the processing of the nucleic acids from the co-partitioned biological particles. These sequences include, e.g., targeted or random/universal amplification primer sequences for amplifying the genomic DNA from the individual biological particles within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides into partitions, e.g., droplets within microfluidic systems.

In an example, microcapsules, such as beads, are provided that each include large numbers of the above described barcoded nucleic acid molecules (e.g., barcoded oligonucleotides) releasably attached to the beads, where all of the nucleic acid molecules attached to a particular bead will include the same nucleic acid barcode sequence, but where a large number of diverse barcode sequences are represented across the population of beads used. In some embodiments, hydrogel beads, e.g., comprising polyacrylamide polymer matrices, are used as a solid support and delivery vehicle for the nucleic acid molecules into the partitions, as they are capable of carrying large numbers of nucleic acid molecules, and may be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. In some cases, the population of beads provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences.

Additionally, each bead can be provided with large numbers of nucleic acid (e.g., oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. In some embodiments, the number of nucleic acid molecules including the barcode sequence on an individual bead is between about 1,000 to about 10,000 nucleic acid molecules, about 5,000 to about 50,000 nucleic acid molecules, about 10,000 to about 100,000 nucleic acid molecules, about 50,000 to about 1,000,000 nucleic acid molecules, about 100,000 to about 10,000,000 nucleic acid molecules, about 1,000,000 to about 1 billion nucleic acid molecules.

Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules.

In some cases, the resulting population of partitions provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences. Additionally, each partition of the population can include between about 1,000 to about 10,000 nucleic acid barcode molecules, about 5,000 to about 50,000 nucleic acid barcode molecules, about 10,000 to about 100,000 nucleic acid barcode molecules, about 50,000 to about 1,000,000 nucleic acid barcode molecules, about 100,000 to about 10,000,000 nucleic acid barcode molecules, about 1,000,000 to about 1 billion nucleic acid barcode molecules.

In some cases, it may be desirable to incorporate multiple different barcodes within a given partition, either attached to a single or multiple beads within the partition. For example, in some cases, a mixed, but known set of barcode sequences may provide greater assurance of identification in the subsequent processing, e.g., by providing a stronger address or attribution of the barcodes to a given partition, as a duplicate or independent confirmation of the output from a given partition.

The nucleic acid molecules (e.g., oligonucleotides) are releasable from the beads upon the application of a particular stimulus to the beads. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

In some aspects, provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

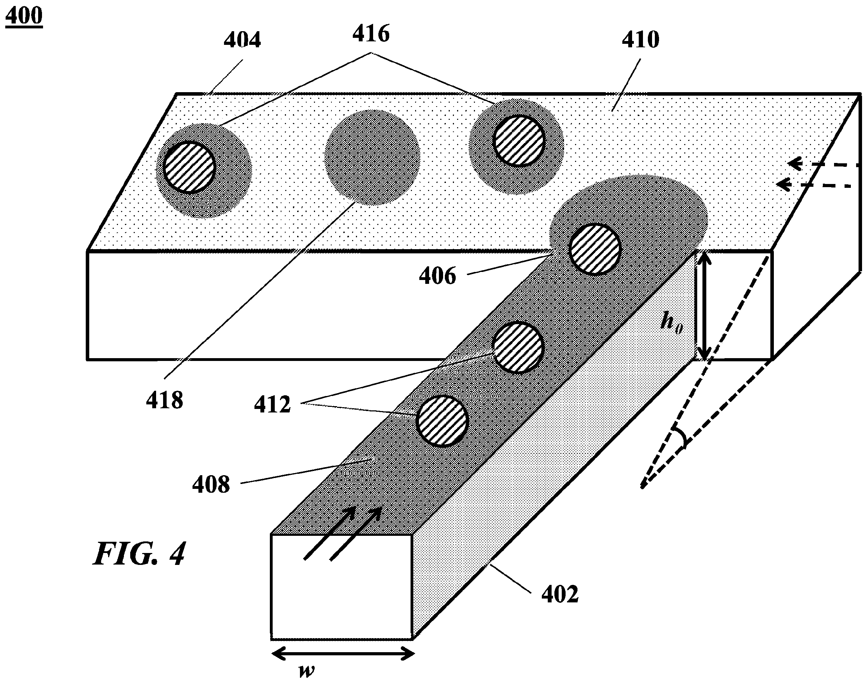

FIG. 4 shows an example of a microfluidic channel structure for the controlled partitioning of beads into discrete droplets. A channel structure **400** can include a channel segment **402** communicating at a channel junction **406** (or intersection) with a reservoir **404**. The reservoir **404** can be a chamber. Any reference to "reservoir," as used herein, can also refer to a "chamber." In operation, an aqueous fluid **408** that includes suspended beads **412** may be transported along the channel segment **402** into the junction **406** to meet a second fluid **410** that is immiscible with the aqueous fluid **408** in the reservoir **404** to create droplets **416, 418** of the aqueous fluid **408** flowing into the reservoir **404**. At the junction **406** where the aqueous fluid **408** and the second fluid **410** meet, droplets can form based on factors such as the hydrodynamic forces at the junction **406**, flow rates of the two fluids **408, 410**, fluid properties, and certain geometric parameters (e.g., $w$, $h_0$, $\alpha$, etc.) of the channel structure **400**. A plurality of droplets can be collected in the reservoir **404** by continuously injecting the aqueous fluid **408** from the channel segment **402** through the junction **406**.

A discrete droplet generated may include a bead (e.g., as in occupied droplets **416**). Alternatively, a discrete droplet generated may include more than one bead. Alternatively, a discrete droplet generated may not include any beads (e.g., as in unoccupied droplet **418**). In some instances, a discrete droplet generated may contain one or more biological particles, as described elsewhere herein. In some instances, a discrete droplet generated may comprise one or more reagents, as described elsewhere herein.

In some instances, the aqueous fluid **408** can have a substantially uniform concentration or frequency of beads **412**. The beads **412** can be introduced into the channel segment **402** from a separate channel (not shown in **FIG. 4**). The frequency of beads **412** in the channel segment **402** may be controlled by controlling the frequency in which the beads **412** are introduced into the channel segment **402** and/or the relative flow rates of the fluids in the channel segment **402** and the separate channel. In some instances, the beads can be introduced into the channel segment **402** from a plurality of different channels, and the frequency controlled accordingly.

In some instances, the aqueous fluid **408** in the channel segment **402** can comprise biological particles (e.g., described with reference to **FIGS. 1** and **2**). In some instances, the aqueous fluid **408** can have a substantially uniform concentration or frequency of biological particles. As with the beads, the biological particles can be introduced into the channel segment **402** from a separate channel. The frequency or concentration of the biological particles in the aqueous fluid **408** in the channel segment **402** may be controlled by controlling the frequency in which the biological particles are introduced into the channel segment **402** and/or the relative flow rates of the fluids in the channel segment **402** and the separate channel. In some instances, the biological particles can be introduced into the channel segment **402** from a plurality of different channels, and the frequency controlled accordingly. In some instances, a first separate channel can introduce beads and a second separate channel can introduce biological particles into the channel segment **402.** The first separate channel introducing the beads may be upstream or downstream of the second separate channel introducing the biological particles.

The second fluid **410** can comprise an oil, such as a fluorinated oil, that includes a fluorosurfactant for stabilizing the resulting droplets, for example, inhibiting subsequent coalescence of the resulting droplets.

In some instances, the second fluid **410** may not be subjected to and/or directed to any flow in or out of the reservoir **404**. For example, the second fluid **410** may be substantially stationary in the reservoir **404.** In some instances, the second fluid **410** may be subjected to flow within the reservoir **404,** but not in or out of the reservoir **404,** such as via application of pressure to the reservoir **404** and/or as affected by the incoming flow of the aqueous fluid **408** at the junction **406.** Alternatively, the second fluid **410** may be subjected and/or directed to flow in or out of the reservoir **404.** For example, the reservoir **404** can be a channel directing the second fluid **410** from upstream to downstream, transporting the generated droplets.

The channel structure **400** at or near the junction **406** may have certain geometric features that at least partly determine the sizes of the droplets formed by the channel structure **400.** The channel segment **402** can have a height, $h_0$ and width, $w$, at or near the junction **406.** By way of example, the channel segment **402** can comprise a rectangular cross-section that leads to a reservoir **404** having a wider cross-section (such as in width or diameter). Alternatively, the cross-section of the channel segment **402** can be other shapes, such as a circular shape, trapezoidal shape, polygonal shape, or any other shapes. The top and bottom walls of the reservoir **404** at or near the junction **406** can be inclined at an expansion angle, $\alpha$. The expansion angle, $\alpha$, allows the tongue (portion of the aqueous fluid **408** leaving channel segment **402** at junction **406** and entering the reservoir **404** before droplet formation) to increase in depth and facilitate decrease in curvature of the intermediately formed droplet. Droplet size may decrease with increasing expansion angle. The resulting droplet radius, $R_d$, may be predicted by the following equation for the aforementioned geometric parameters of $h_0$, $w$, and $\alpha$:

$$R_d \approx 0.44 \left( 1 + 2.2\sqrt{\tan \alpha} \; \frac{w}{h_0} \right) \frac{h_0}{\sqrt{\tan \alpha}}$$

By way of example, for a channel structure with $w = 21\ \mu$m, $h = 21\ \mu$m, and $\alpha = 3°$, the predicted droplet size is 121 $\mu$m. In another example, for a channel structure with $w = 25\ \mu$m, $h = 25\ \mu$m, and $\alpha = 5°$, the predicted droplet size is 123 $\mu$m. In another example, for a channel structure with $w = 28\ \mu$m, $h = 28\ \mu$m, and $\alpha = 7°$, the predicted droplet size is 124 $\mu$m.

In some instances, the expansion angle, $\alpha$, may be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. In some instances, the expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less. In some instances, the width, $w$, can be between a range of from about 100 micrometers ($\mu$m) to about 500 $\mu$m. In some instances, the width, $w$, can be between a range of from about 10 $\mu$m to about 200 $\mu$m. Alternatively, the width can be less than about 10 $\mu$m. Alternatively, the width can be greater than about 500 $\mu$m. In some instances, the flow rate of the aqueous fluid **408** entering the junction **406** can be between about 0.04 microliters ($\mu$L)/minute (min) and about 40 $\mu$L/min. In some instances, the flow rate of the aqueous fluid **408** entering the junction **406** can be between about 0.01 microliters ($\mu$L)/minute (min) and about 100 $\mu$L/min. Alternatively, the flow rate of the aqueous fluid **408** entering the junction **406** can be less than about 0.01 $\mu$L/min. Alternatively, the flow rate of the aqueous fluid **408** entering the junction **406** can be greater than about 40 $\mu$L/min, such as 45 $\mu$L/min, 50 $\mu$L/min, 55 $\mu$L/min, 60 $\mu$L/min, 65 $\mu$L/min, 70 $\mu$L/min, 75 $\mu$L/min, 80 $\mu$L/min, 85 $\mu$L/min, 90 $\mu$L/min, 95 $\mu$L/min, 100 $\mu$L/min, 110 $\mu$L/min , 120 $\mu$L/min, 130 $\mu$L/min , 140 $\mu$L/min , 150 $\mu$L/min, or greater. At lower flow rates, such as flow rates of about less than or equal to 10 microliters/minute, the droplet radius may not be dependent on the flow rate of the aqueous fluid **408** entering the junction **406.**

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

The throughput of droplet generation can be increased by increasing the points of generation, such as increasing the number of junctions (e.g., junction **406**) between aqueous fluid **408** channel segments (e.g., channel segment **402**) and the reservoir **404**. Alternatively or in addition, the throughput of droplet generation can be increased by increasing the flow rate of the aqueous fluid **408** in the channel segment **402.**

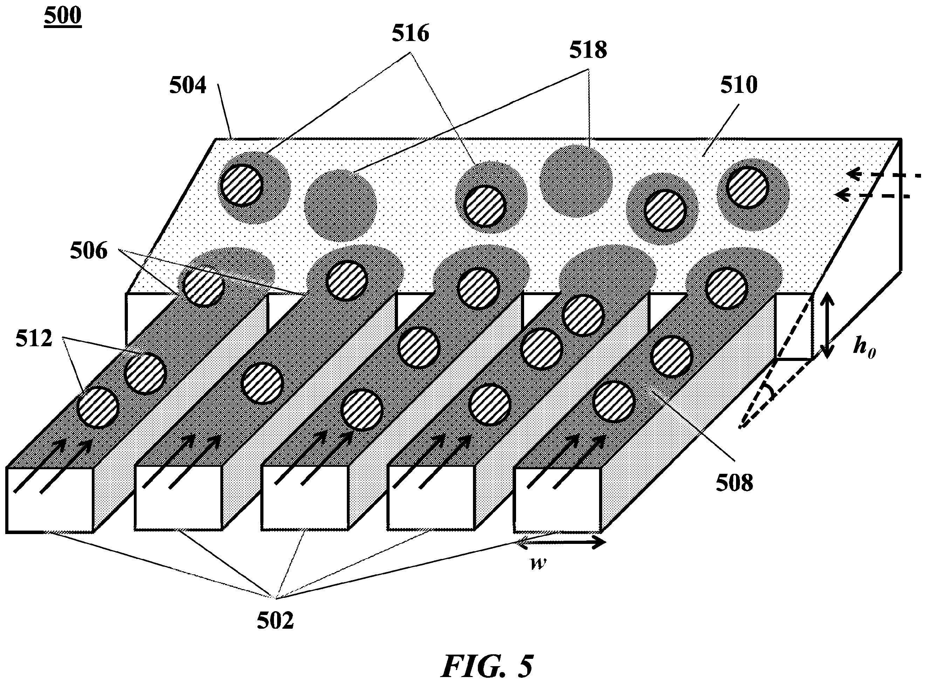

**FIG. 5** shows an example of a microfluidic channel structure for increased droplet generation throughput. A microfluidic channel structure **500** can comprise a plurality of channel segments **502** and a reservoir **504**. Each of the plurality of channel segments **502** may be in fluid communication with the reservoir **504**. The channel structure **500** can comprise a plurality of channel junctions **506** between the plurality of channel segments **502** and the reservoir **504**. Each channel junction can be a point of droplet generation. The channel segment **402** from the channel structure **400** in **FIG. 4** and any description to the components thereof may correspond to a given channel segment of the plurality of channel segments **502** in channel structure **500** and any description to the corresponding components thereof. The reservoir **404** from the channel structure **400** and any description to the components thereof may correspond to the reservoir **504** from the channel structure **500** and any description to the corresponding components thereof.

Each channel segment of the plurality of channel segments **502** may comprise an aqueous fluid **508** that includes suspended beads **512**. The reservoir **504** may comprise a second fluid **510** that is immiscible with the aqueous fluid **508**. In some instances, the second fluid **510** may not be subjected to and/or directed to any flow in or out of the reservoir **504**. For example, the second fluid **510** may be substantially stationary in the reservoir **504**. In some instances, the second fluid **510** may be subjected to flow within the reservoir **504**, but not in or out of the reservoir **504**, such as via application of pressure to the reservoir **504** and/or as affected by the incoming flow of the aqueous fluid **508** at the junctions. Alternatively, the second fluid **510** may be subjected and/or directed to flow in or out of the reservoir **504**. For example, the reservoir **504** can be a channel directing the second fluid **510** from upstream to downstream, transporting the generated droplets.

In operation, the aqueous fluid **508** that includes suspended beads **512** may be transported along the plurality of channel segments **502** into the plurality of junctions **506** to meet the second fluid **510** in the reservoir **504** to create droplets **516, 518**. A droplet may form from each channel segment at each corresponding junction with the reservoir **504**. At the junction where the aqueous fluid **508** and the second fluid **510** meet, droplets can form based on factors such as the hydrodynamic forces at the junction, flow rates of the two fluids **508, 510,** fluid properties, and certain geometric parameters (e.g., $w$, $h_0$, $\alpha$, etc.) of the channel structure **500,** as described elsewhere herein. A plurality of droplets can be collected in the reservoir **504** by continuously injecting the aqueous fluid **508** from the plurality of channel segments **502** through the plurality of junctions **506**. Throughput may significantly increase with the parallel channel configuration of channel structure **500**. For example, a channel structure having five inlet channel segments comprising the aqueous fluid **508** may generate droplets five times as frequently than a channel structure having one inlet channel segment, provided that the fluid flow rate in the channel segments are substantially the same. The fluid flow rate in the different inlet channel segments may or may not be substantially the same. A channel structure may have as many parallel channel segments as is practical and allowed for the size of the reservoir. For example, the channel structure may have at least about 2, 3, 4, 5, 6, 7, 8, 9, 10, 20, 30, 40, 50, 60, 70, 80, 90, 100, 150, 500, 250, 300, 350, 400, 450, 500, 600, 700, 800, 900, 1000, 1500, 5000 or more parallel or substantially parallel channel segments.

The geometric parameters, $w$, $h_0$, and $\alpha$, may or may not be uniform for each of the channel segments in the plurality of channel segments **502**. For example, each channel segment may have the same or different widths at or near its respective channel junction with the reservoir **504**. For example, each channel segment may have the same or different height at or near its respective channel junction with the reservoir **504**. In another example, the reservoir **504** may have the same or different expansion angle at the different channel junctions with the plurality of channel segments **502**. When the geometric parameters are uniform, beneficially, droplet size may also be controlled to be uniform even with the increased throughput. In some instances, when it is desirable to have a different distribution of droplet sizes, the geometric parameters for the plurality of channel segments **502** may be varied accordingly.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

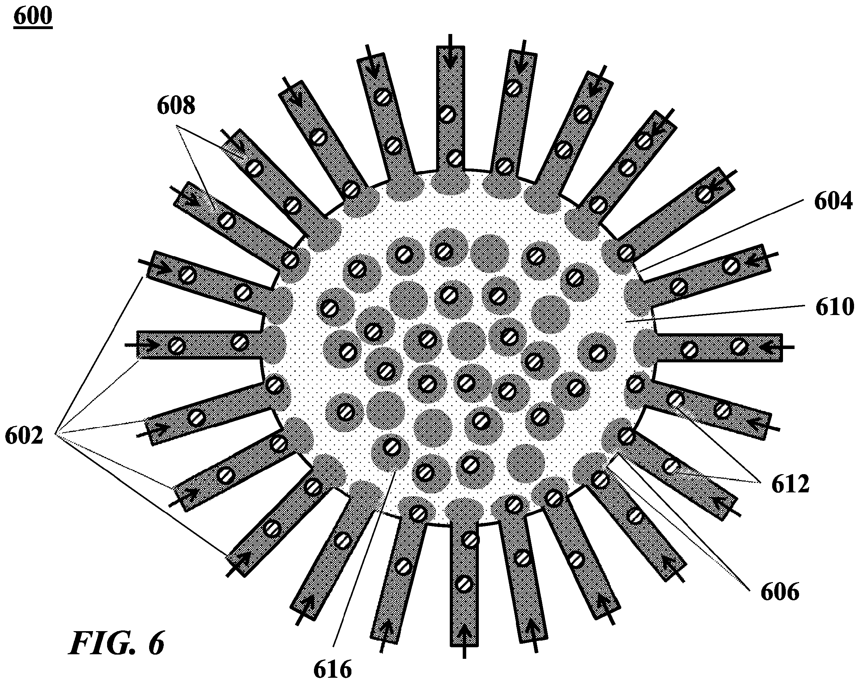

**FIG. 6** shows another example of a microfluidic channel structure for increased droplet generation throughput. A microfluidic channel structure **600** can comprise a plurality of channel segments **602** arranged generally circularly around the perimeter of a reservoir **604**. Each of the plurality of channel segments **602** may be in fluid communication with the reservoir **604**. The channel structure **600** can comprise a plurality of channel junctions **606** between the plurality of channel segments **602** and the reservoir **604**. Each channel junction can be a point of droplet generation. The channel segment **402** from the channel structure **400** in **FIG. 4** and any description to the components thereof may correspond to a given channel segment of the plurality of channel segments **602** in channel structure **600** and any description to the corresponding components thereof. The reservoir **404** from the channel structure **400** and any description to the components thereof may correspond to the reservoir **604** from the channel structure **600** and any description to the corresponding components thereof.

Each channel segment of the plurality of channel segments **602** may comprise an aqueous fluid **608** that includes suspended beads **612**. The reservoir **604** may comprise a second fluid **610** that is immiscible with the aqueous fluid **608**. In some instances, the second fluid **610** may not be subjected to and/or directed to any flow in or out of the reservoir **604**. For example, the second fluid **610** may be substantially stationary in the reservoir **604**. In some instances, the second fluid **610** may be subjected to flow within the reservoir **604**, but not in or out of the reservoir **604**, such as via application of pressure to the reservoir **604** and/or as affected by the incoming flow of the aqueous fluid **608** at the junctions. Alternatively, the second fluid **610** may be subjected and/or directed to flow in or out of the reservoir **604**. For example, the reservoir **604** can be a channel directing the second fluid **610** from upstream to downstream, transporting the generated droplets.

In operation, the aqueous fluid **608** that includes suspended beads **612** may be transported along the plurality of channel segments **602** into the plurality of junctions **606** to meet the second fluid **610** in the reservoir **604** to create a plurality of droplets **616**. A droplet may form from each channel segment at each corresponding junction with the reservoir **604**. At the junction where the aqueous fluid **608** and the second fluid **610** meet, droplets can form based on factors such as the hydrodynamic forces at the junction, flow rates of the two fluids **608, 610,** fluid properties, and certain geometric parameters (e.g., widths and heights of the channel segments **602,** expansion angle of the reservoir **604,** etc.) of the channel structure **600,** as described elsewhere herein. A plurality of droplets can be collected in the reservoir **604** by continuously injecting the aqueous fluid **608** from the plurality of channel segments **602** through the plurality of junctions **606**. Throughput may significantly increase with the substantially parallel channel configuration of the channel structure **600**. A channel structure may have as many substantially parallel channel segments as is practical and allowed for by the size of the reservoir. For example, the channel structure may have at least about 2, 3, 4, 5, 6, 7, 8, 9, 10, 20, 30, 40, 50, 60, 70, 80, 90, 100, 150, 200, 250, 300, 350, 400, 450, 500, 600, 700, 800, 900, 1000, 1500, 5000 or more parallel or substantially parallel channel segments. The plurality of channel segments may be substantially evenly spaced apart, for example, around an edge or perimeter of the reservoir. Alternatively, the spacing of the plurality of channel segments may be uneven.

The reservoir **604** may have an expansion angle, $\alpha$ (not shown in **FIG. 6**) at or near each channel junction. Each channel segment of the plurality of channel segments **602** may have a width, $w$, and a height, $h_0$, at or near the channel junction. The geometric parameters, $w$, $h_0$, and $\alpha$, may or may not be uniform for each of the channel segments in the plurality of channel segments **602**. For example, each channel segment may have the same or different widths at or near its respective channel junction with the reservoir **604**. For example, each channel segment may have the same or different height at or near its respective channel junction with the reservoir **604**.

The reservoir **604** may have the same or different expansion angle at the different channel junctions with the plurality of channel segments **602**. For example, a circular reservoir (as shown in **FIG. 6**) may have a conical, dome-like, or hemispherical ceiling (e.g., top wall) to provide the same or substantially same expansion angle for each channel segments **602** at or near the plurality of channel junctions **606**. When the geometric parameters are uniform, beneficially, resulting droplet size may be controlled to be uniform even with the increased throughput. In some instances, when it is desirable to have a different distribution of droplet sizes, the geometric parameters for the plurality of channel segments **602** may be varied accordingly.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size. The beads and/or biological particle injected into the droplets may or may not have uniform size.

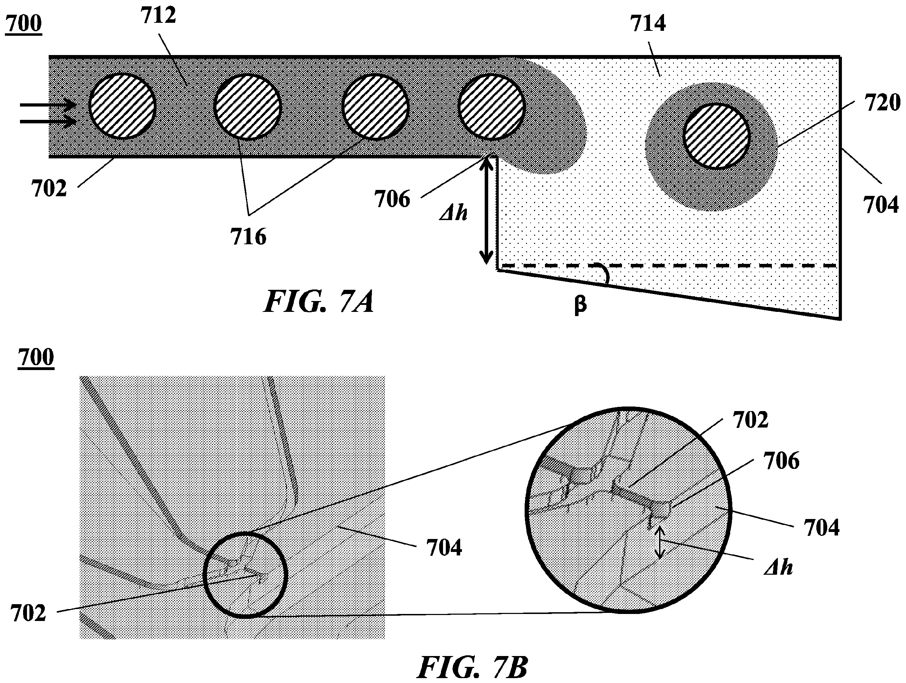

**FIG. 7A** shows a cross-section view of another example of a microfluidic channel structure with a geometric feature for controlled partitioning. A channel structure **700** can include a channel segment **702** communicating at a channel junction **706** (or intersection) with a reservoir **704**. In some instances, the channel structure **700** and one or more of its components can correspond to the channel structure **100** and one or more of its components. **FIG. 7B** shows a perspective view of the channel structure **700** of **FIG. 7A**.

An aqueous fluid **712** comprising a plurality of particles **716** may be transported along the channel segment **702** into the junction **706** to meet a second fluid **714** (e.g., oil, etc.) that is immiscible with the aqueous fluid **712** in the reservoir **704** to create droplets **720** of the aqueous fluid **712** flowing into the reservoir **704**. At the junction **706** where the aqueous fluid **712** and the second fluid **714** meet, droplets can form based on factors such as the hydrodynamic forces at the junction **706,** relative flow rates of the two fluids **712, 714,** fluid properties, and certain geometric parameters (e.g., $\Delta h$, etc.) of the channel structure **700**. A plurality of droplets can be collected in the reservoir **704** by continuously injecting the aqueous fluid **712** from the channel segment **702** at the junction **706**.

A discrete droplet generated may comprise one or more particles of the plurality of particles **716**. As described elsewhere herein, a particle may be any particle, such as a bead, cell bead, gel bead, biological particle, macromolecular constituents of biological particle, or other particles. Alternatively, a discrete droplet generated may not include any particles.

In some instances, the aqueous fluid **712** can have a substantially uniform concentration or frequency of particles **716**. As described elsewhere herein (e.g., with reference to **FIG. 4**), the particles **716** (e.g., beads) can be introduced into the channel segment **702** from a separate channel (not shown in **FIG. 7**). The frequency of particles **716** in the channel segment **702** may be controlled by controlling the frequency in which the particles **716** are introduced into the channel segment **702** and/or the relative flow rates of the fluids in the channel segment **702** and the separate channel. In some instances, the particles **716** can be introduced into the channel segment **702** from a plurality of different channels, and the frequency controlled accordingly. In some instances, different particles may be introduced via separate channels. For example, a first separate channel can introduce beads and a second separate channel can introduce biological particles into the channel segment **702**. The first separate channel introducing the beads may be upstream or downstream of the second separate channel introducing the biological particles.

In some instances, the second fluid **714** may not be subjected to and/or directed to any flow in or out of the reservoir **704.** For example, the second fluid **714** may be substantially stationary in the reservoir **704.** In some instances, the second fluid **714** may be subjected to flow within the reservoir **704,** but not in or out of the reservoir **704,** such as via application of pressure to the reservoir **704** and/or as affected by the incoming flow of the aqueous fluid **712** at the junction **706.** Alternatively, the second fluid **714** may be subjected and/or directed to flow in or out of the reservoir **704.** For example, the reservoir **704** can be a channel directing the second fluid **714** from upstream to downstream, transporting the generated droplets.

The channel structure **700** at or near the junction **706** may have certain geometric features that at least partly determine the sizes and/or shapes of the droplets formed by the channel structure **700**. The channel segment **702** can have a first cross-section height, $h_1$, and the reservoir **704** can have a second cross-section height, $h_2$. The first cross-section height, $h_1$, and the second cross-section height, $h_2$, may be different, such that at the junction **706,** there is a height difference of $\Delta h$. The second cross-section height, $h_2$, may be greater than the first cross-section height, $h_1$. In some instances, the reservoir may thereafter gradually increase in cross-section height, for example, the more distant it is from the junction **706.** In some instances, the cross-section height of the reservoir may increase in accordance with expansion angle, $\beta$, at or near the junction **706.** The height difference, $\Delta h$, and/or expansion angle, $\beta$, can allow the tongue (portion of the aqueous fluid **712** leaving channel segment **702** at junction **706** and entering the reservoir **704** before droplet formation) to increase in depth and facilitate decrease in curvature of the intermediately formed droplet. For example, droplet size may decrease with increasing height difference and/or increasing expansion angle.

The height difference, $\Delta h$, can be at least about 1 $\mu$m. Alternatively, the height difference can be at least about 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500 $\mu$m or more. Alternatively, the height difference can be at most about 500, 400, 300, 200, 100, 90, 80, 70, 60, 50, 45, 40, 35, 30, 25, 20, 19, 18, 17, 16, 15, 14, 13, 12, 11, 10, 9, 8, 7, 6, 5, 4, 3, 2, 1 $\mu$m or less. In some instances, the expansion angle, $\beta$, may be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. In some instances, the expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less.

In some instances, the flow rate of the aqueous fluid **712** entering the junction **706** can be between about 0.04 microliters ($\mu$L)/minute (min) and about 40 $\mu$L/min. In some instances, the flow rate of the aqueous fluid **712** entering the junction **706** can be between about 0.01 microliters ($\mu$L)/minute (min) and about 100 $\mu$L/min. Alternatively, the flow rate of the aqueous fluid **712** entering the junction **706** can be less than about 0.01 $\mu$L/min. Alternatively, the flow rate of the aqueous fluid **712** entering the junction **706** can be greater than about 40 $\mu$L/min, such as 45 $\mu$L/min, 50 $\mu$L/min, 55 $\mu$L/min, 60 $\mu$L/min, 65 $\mu$L/min, 70 $\mu$L/min, 75 $\mu$L/min, 80 $\mu$L/min, 85 $\mu$L/min, 90 $\mu$L/min, 95 $\mu$L/min, 100 $\mu$L/min, 110 $\mu$L/min , 120 $\mu$L/min , 130 $\mu$L/min , 140 $\mu$L/min , 150 $\mu$L/min, or greater. At lower flow rates, such as flow rates of about less than or equal to 10 microliters/minute, the droplet radius may not be dependent on the flow rate of the aqueous fluid **712** entering the junction **706**. The second fluid **714** may be stationary, or substantially stationary, in the reservoir **704.** Alternatively, the second fluid **714** may be flowing, such as at the above flow rates described for the aqueous fluid **712.**

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

While **FIGs. 7A** and **7B** illustrate the height difference, $\Delta h$, being abrupt at the junction **706** (e.g., a step increase), the height difference may increase gradually (e.g., from about 0 $\mu$m to a maximum height difference). Alternatively, the height difference may decrease gradually (e.g., taper) from a maximum height difference. A gradual increase or decrease in height difference, as used herein, may refer to a continuous incremental increase or decrease in height difference, wherein an angle between any one differential segment of a height profile and an immediately adjacent differential segment of the height profile is greater than 90°. For example, at the junction **706,** a bottom wall of the channel and a bottom wall of the reservoir can meet at an angle greater than 90°. Alternatively or in addition, a top wall (e.g., ceiling) of the channel and a top wall (e.g., ceiling) of the reservoir can meet an angle greater than 90°. A gradual increase or decrease may be linear or non-linear (e.g., exponential, sinusoidal, etc.). Alternatively or in addition, the height difference may variably increase and/or decrease linearly or non-linearly. While **FIGs. 7A** and **7B** illustrate the expanding reservoir cross-section height as linear (e.g., constant expansion angle, $\beta$), the cross-section height may expand non-linearly. For example, the reservoir may be defined at least partially by a dome-like (e.g., hemispherical) shape having variable expansion angles. The cross-section height may expand in any shape.

The channel networks, e.g., as described above or elsewhere herein, can be fluidly coupled to appropriate fluidic components. For example, the inlet channel segments are fluidly coupled to appropriate sources of the materials they are to deliver to a channel junction. These sources may include any of a variety of different fluidic components, from simple reservoirs defined in or connected to a body structure of a microfluidic device, to fluid conduits that deliver fluids from off-device sources, manifolds, fluid flow units (e.g., actuators, pumps, compressors) or the like. Likewise, the outlet channel segment (e.g., channel segment **208,** reservoir **604,** etc.) may be fluidly coupled to a receiving vessel or conduit for the partitioned cells for subsequent processing. Again, this may be a reservoir defined in the body of a microfluidic device, or it may be a fluidic conduit for delivering the partitioned cells to a subsequent process operation, instrument or component.

The methods and systems described herein may be used to greatly increase the efficiency of single cell applications and/or other applications receiving droplet-based input. For example, following the sorting of occupied cells and/or appropriately-sized cells, subsequent operations that can be performed can include generation of amplification products, purification (e.g., via solid phase reversible immobilization (SPRI)), further processing (e.g., shearing, ligation of functional sequences, and subsequent amplification (e.g., via PCR)). These operations may occur in bulk (e.g., outside the partition). In the case where a partition is a droplet in an emulsion, the emulsion can be broken and the contents of the droplet pooled for additional operations. Additional reagents that may be co-partitioned along with the barcode bearing bead may include oligonucleotides to block ribosomal RNA (rRNA) and nucleases to digest genomic DNA from cells. Alternatively, rRNA removal agents may be applied during additional processing operations. The configuration of the constructs generated by such a method can help minimize (or avoid) sequencing of the poly-T sequence during sequencing and/or sequence the 5' end of a polynucleotide sequence. The amplification products, for example, first amplification products and/or second amplification products, may be subject to sequencing for sequence analysis. In some cases, amplification may be performed using the Partial Hairpin Amplification for Sequencing (PHASE) method.

A variety of applications require the evaluation of the presence and quantification of different biological particle or organism types within a population of biological particles, including, for example, microbiome analysis and characterization, environmental testing, food safety testing, epidemiological analysis, e.g., in tracing contamination or the like.

In the methods and systems described herein, one or more labelling agents capable of binding to or otherwise coupling to one or more cell features may be used to characterize cells and/or cell features. In some instances, cell features include cell surface features. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof.

A labelling agent may include, but is not limited to, a protein (e.g., an antigen), a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have coupled thereto a first reporter oligonucleotide, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969.

In a particular example, a library of potential cell feature labelling agents may be provided associated with nucleic acid reporter molecules, e.g., where a different reporter oligonucleotide sequence is associated with each labelling agent capable of binding to a specific cell feature. In some aspects, different members of the library may be characterized by the presence of a different oligonucleotide sequence label, e.g., an antibody capable of binding to a first type of protein may have associated with it a first known reporter oligonucleotide sequence, while an antibody capable of binding to a second protein (i.e., different than the first protein) may have a different known reporter oligonucleotide sequence associated with it. Prior to partitioning, the cells may be incubated with the library of labelling agents, that may represent labelling agents to a broad panel of different cell features, e.g., receptors (e.g., BCRs, TCRs), proteins, etc., and which include their associated reporter oligonucleotides. Unbound labelling agents may be washed from the cells, and the cells may then be co-partitioned (e.g., into droplets or wells) along with partition-specific barcode oligonucleotides (e.g., attached to a bead, such as a gel bead) as described elsewhere herein. As a result, the partitions may include the cell or cells, as well as the bound labelling agents and their known, associated reporter oligonucleotides.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. In this way, different samples or groups can be independently processed and subsequently combined together for pooled analysis

EP 4 158 058 B1

(e.g., partition-based barcoding as described elsewhere herein). See, e.g., U.S. Pat. Pub. 20190323088.

In some aspects, these reporter oligonucleotides may comprise nucleic acid barcode sequences that permit identification of the labelling agent which the reporter oligonucleotide is coupled to. The selection of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link® antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715. Likewise, protein and peptide biotinylation techniques have been developed and are readily available. See, e.g., U.S. Pat. No. 6,265,552. Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to a first oligonucleotide that is complementary (e.g., hybridizes) to a sequence of the reporter oligonucleotide.

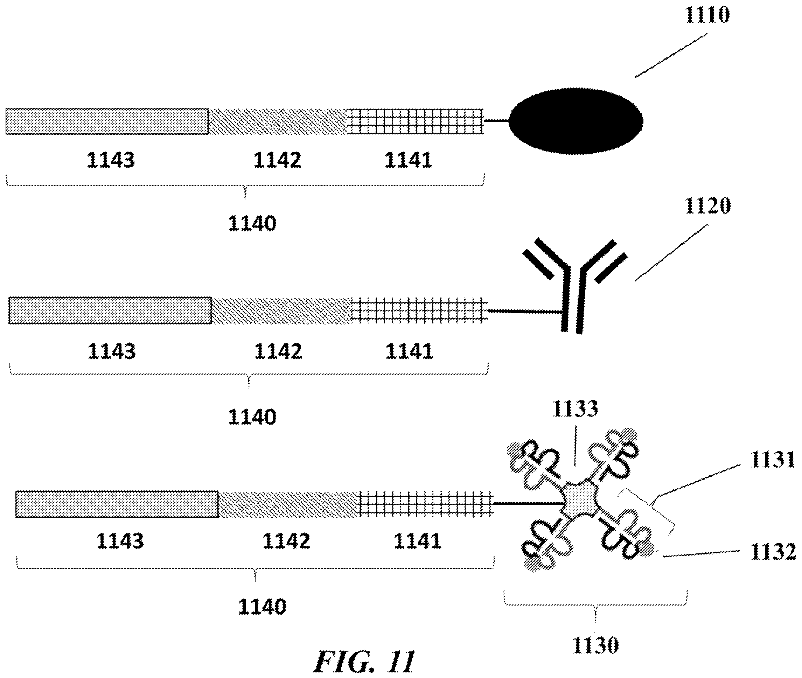

FIG. 11 describes exemplary labelling agents (1110, 1120, 1130) comprising reporter oligonucleotides (1140) attached thereto. Labelling agent 1110 (e.g., any of the labelling agents described herein) is attached (either directly, e.g., covalently attached, or indirectly) to reporter oligonucleotide 1140. Reporter oligonucleotide 1140 may comprise barcode sequence 1142 that identifies labelling agent 1110. Reporter oligonucleotide 1140 may also comprise one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to FIG. 11, in some instances, reporter oligonucleotide 1140 conjugated to a labelling agent (e.g., 1110, 1120, 1130) comprises a primer sequence 1141, a barcode sequence that identifies the labelling agent (e.g., 1110, 1120, 1130), and functional sequence 1143. Functional sequence 1143 may be configured to hybridize to a complementary sequence, such as a complementary sequence present on a nucleic acid barcode molecule 1190 (not shown), such as those described elsewhere herein. In some instances, nucleic acid barcode molecule 1190 is attached to a support (e.g., a bead, such as a gel bead), such as those described elsewhere herein. For example, nucleic acid barcode molecule 1190 may be attached to the support via a releasable linkage (e.g., comprising a labile bond), such as those described elsewhere herein. In some instances, reporter oligonucleotide 1140 comprises one or more additional functional sequences, such as those described above.

In some instances, the labelling agent 1110 is a protein or polypeptide (e.g., an antigen or prospective antigen) comprising reporter oligonucleotide 1140. Reporter oligonucleotide 1140 comprises barcode sequence 1142 that identifies polypeptide 1110 and can be used to infer the presence of, e.g., a binding partner of polypeptide 1110 (i.e., a molecule or compound to which the polypeptide binds). In some instances, the labelling agent 1110 is a lipophilic moiety (e.g., cholesterol) comprising reporter oligonucleotide **1140,** where the lipophilic moiety is selected such that labelling agent **1110** integrates into a membrane of a cell or nucleus. Reporter oligonucleotide **1140** comprises barcode sequence **1142** that identifies lipophilic moiety **1110** which in some instances is used to tag cells (e.g., groups of cells, cell samples, etc.) for multiplex analyses as described elsewhere herein. In some instances, the labelling agent is an antibody **1120** (or an epitope binding fragment thereof) comprising reporter oligonucleotide **1140**. Reporter oligonucleotide **1140** comprises barcode sequence **1142** that identifies antibody **1120** and can be used to infer the presence of, e.g., a target of antibody **1120** (i.e., a molecule or compound to which antibody **1120** binds). In other embodiments, labelling agent **1130** comprises an MHC molecule **1131** comprising peptide **1132** and reporter oligonucleotide **1140** that identifies peptide **1132**. In some instances, the MHC molecule is coupled to a support **1133**. In some instances, support **1133** is streptavidin (e.g., MHC molecule **1131** may comprise biotin). In other embodiments, support **1133** is a polysaccharide, such as dextran. In some instances, reporter oligonucleotide **1140** may be directly or indirectly coupled to MHC labelling agent **1130** in any suitable manner, such as to MCH molecule **1131,** support **1133,** or peptide **1132**. In some embodiments, labelling agent **1130** comprises a plurality of MHC molecules, i.e., is an MHC multimer, which may be coupled to a support (e.g., **1133**). There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5® MHC Class I Pentamers, (ProImmune, Ltd.), MHC octamers, MHC dodecamers, MHC decorated dextran molecules (e.g., MHC Dextramer® (Immudex)), etc. For a description of exemplary labelling agents, including antibody and MHC-based labelling agents, reporter oligonucleotides, and methods of use, see, e.g., U.S. Pat. 10,550,429, U.S. 10,954,562, U.S. Pat. Pub. 20190367969 and U.S. patent application serial number 63/135,514 filed January 8, 2021.

In some instances, analysis of one or more analytes (e.g., using the labelling agents described herein) comprises a workflow as generally depicted in **FIG. 12A.** For example, in some embodiments, cells are contacted with one or more reporter oligonucleotide **1220** conjugated labelling agents **1210** (e.g., polypeptide (e.g., antigen), antibody, or pMHC molecule or complex) and optionally further processed prior to barcoding. Optional processing steps may include one or more washing and/or cell sorting steps. In some instances, a cell bound to labelling agent **1210** (e.g., polypeptide, antibody, or pMHC molecule or complex) conjugated to oligonucleotide **1220** and support **1230** (e.g., a bead, such as a gel bead) comprising nucleic acid barcode molecule **1290** are partitioned into a partition amongst a plurality of partitions (e.g., a droplet of a droplet emulsion or a well of a micro/nanowell array). In some instances, the partition comprises at most a single cell bound to labelling agent **1210**. In some embodiments, nucleic acid barcode molecule **1290** is attached to support **1230** via a releasable linkage **1240** (e.g., comprising a labile bond) as described elsewhere herein.

With continued reference to **FIG. 12A,** in some instances, reporter oligonucleotide **1220** conjugated to labelling agent **1210** (e.g., polypeptide, an antibody, pMHC molecule such as an MHC multimer, etc.) comprises a first adapter sequence **1211** (e.g., a primer sequence), a barcode sequence **1212** that identifies the labelling agent **1210** (e.g., the polypeptide, antibody, or peptide of a pMHC molecule or complex), and an adapter sequence **1213.** Adapter sequence **1213** may be configured to hybridize to a complementary sequence, such as a complementary sequence **1223** present on a nucleic acid barcode molecule **1290,** such as those described elsewhere herein. In some instances, nucleic acid barcode molecule **1290** is attached to a support **1230** (e.g., a bead, such as a gel bead), such as those described elsewhere herein. For example, nucleic acid barcode molecule **1290** may be attached to support **1230** via a releasable linkage **1240** (e.g., comprising a labile bond), such as those described elsewhere herein. In some instances, oligonucleotide **1220** comprises one or more additional functional sequences, such as those described above.

In some instances, analysis of multiple analytes (e.g., RNA and one or more analytes using labelling agents described herein) comprises a workflow as generally depicted in **FIGS. 12A-C.** Cells are contacted with labeling agents and processed as generally described above and depicted in **FIG. 12A.** For example, sequence **1213** may then be hybridized to complementary sequence **1223** to generate (e.g., via a nucleic acid reaction, such as nucleic acid extension or ligation) a barcoded nucleic acid molecule comprising cell (e.g., partition specific) barcode sequence **1222** (or a reverse complement thereof) and reporter barcode sequence **1212** (or a reverse complement thereof). Referring to **FIGS. 12B-C,** in some instances, nucleic acid molecules derived from a cell (such as RNA molecules) can be similarly processed to append the cell (e.g., partition-specific) barcode sequence **1222** to these molecules or derivatives thereof (e.g., cDNA molecules). For example, referring to **FIG. 12B,** in some embodiments, primer **1250** comprises a sequence complementary to a sequence of RNA molecule **1260** (such as an RNA encoding for a BCR sequence) from a cell. In some instances, primer **1250** comprises one or more adapter sequences **1251** that are not complementary to RNA molecule **1260**. In some instances, primer **1250** comprises a poly-T sequence. In some instances, primer **1250** comprises a sequence complementary to a target sequence in an RNA molecule. In some instances, primer **1250** comprises a sequence complementary to a region of an immune molecule, such as the constant region of a TCR or BCR sequence. Primer **1250** is hybridized to RNA molecule **1260** and cDNA molecule **1270** is generated in a reverse transcription reaction. In some instances, the reverse transcriptase enzyme is selected such that several non-templated bases **1280** (e.g., a poly-C sequence) are appended to the cDNA. Nucleic acid barcode molecule **1290** comprises a sequence **1224** complementary to the non-templated bases, and the reverse transcriptase performs a template switching reaction onto nucleic acid barcode molecule **1290** to generate a barcoded nucleic acid molecule comprising cell (e.g., partition specific) barcode sequence **1222** (or a reverse complement thereof) and a sequence of cDNA **1270** (or a portion thereof). In another example, referring to **FIG. 12C,** in some embodiments, nucleic acid barcode molecule **1290** comprises sequence **1223** complementary to a sequence of RNA molecule **1260** from a cell. In some instances, sequence **1223** comprises a sequence specific for an RNA molecule. In some instances, sequence **1223** comprises a poly-T sequence. In some instances, sequence **1223** comprises a sequence specific for an RNA molecule. In some instances, sequence **1223** comprises a sequence complementary to a region of an immune molecule, such as the constant region of a TCR or BCR sequence. Sequence **1223** is hybridized to RNA molecule **1260** and a cDNA molecule **1270** is generated in a reverse transcription reaction generating a barcoded nucleic acid molecule comprising cell (e.g., partition specific) barcode sequence **1222** (or a reverse complement thereof) and a sequence of cDNA **1270** (or a portion thereof). Barcoded nucleic acid molecules can then be optionally processed as described elsewhere herein, e.g., to amplify the molecules and/or append sequencing platform specific sequences to the fragments. See, e.g., U.S. Pat. Pub. 20180105808. Barcoded nucleic acid molecules, or derivatives generated therefrom, can then be sequenced on a suitable sequencing platform.

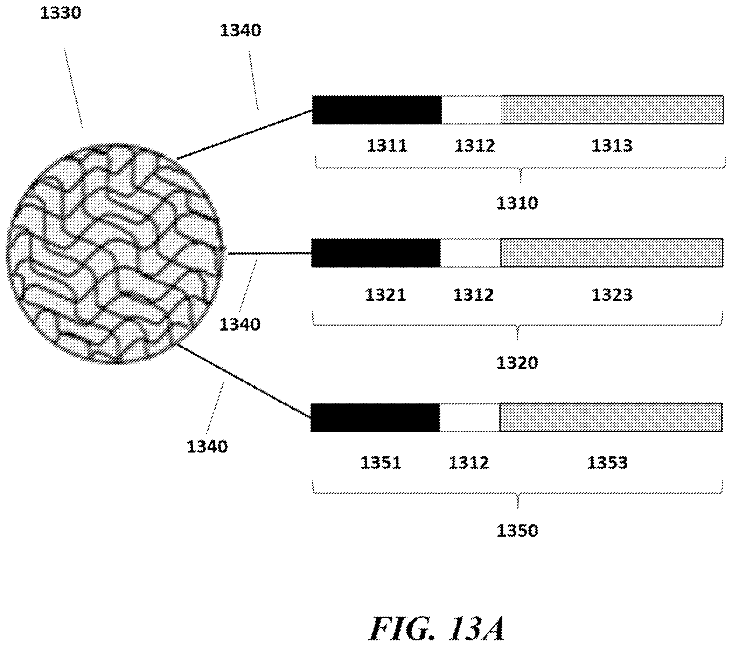

In some embodiments, analysis of multiple analytes (e.g., RNA and one or more analytes using labelling agents described herein) comprises a workflow as generally depicted in **FIGS. 13A-C.** For example, in some embodiments, cells are contacted with one or more reporter oligonucleotide **1220** conjugated labelling agents **1210** (e.g., polypeptide, antibody, or pMHC molecule or complex) and optionally further processed prior to barcoding. Optional processing steps may include one or more washing and/or cell sorting steps. In some instances, a cell bound to labelling agent **1210** (e.g., polypeptide (e.g., antigen), antibody, or pMHC molecule or complex) conjugated to oligonucleotide **1220** and support **1330** (e.g., a bead, such as a gel bead) comprising nucleic acid barcode molecules **1310** and **1320** comprising common barcode sequence **1314** are partitioned into a partition amongst a plurality of partitions (e.g., a droplet of a droplet emulsion or a well of a micro/nanowell array). In some instances, the partition comprises at most a single cell bound to labelling agent **1210.** In some embodiments, nucleic acid barcode molecules **1310** and **1320** are attached to support **1230** via a releasable linkage **1340** (e.g., comprising a labile bond) as described elsewhere herein. Nucleic acid barcode molecule **1310** may comprise adapter sequence **1311,** barcode sequence **1312** and adapter sequence **1313.** Nucleic acid barcode molecule **1320** may comprise adapter sequence **1321,** barcode sequence **1312,** and adapter sequence **1323,** wherein adapter sequence **1323** comprises a different sequence than adapter sequence **1313.** In some instances, adapter **1311** and adapter **1321** comprise the same sequence. In some instances, adapter **1311** and adapter **1321** comprise different sequences. Although support **1330** is shown comprising nucleic acid barcode molecules **1310** and **1320,** any suitable number of barcode molecules comprising common barcode sequence **1312** are contemplated herein. For example, in some embodiments, support **1330** further comprises nucleic acid barcode molecule **1350.** Nucleic acid barcode molecule **1350** may comprise adapter sequence **1351,** barcode sequence **1312** and adapter sequence **1353,** wherein adapter sequence **1353** comprises a different sequence than adapter sequence **1313** and **1323.** In some instances, nucleic acid barcode molecules (e.g., **1310, 1320, 1550)** comprise one or more additional functional sequences, such as a UMI or other sequences described herein.

Subsequent to partitioning, referring to **FIG. 13B,** in some embodiments, sequence **1213** is hybridized to complementary sequence **1313** of nucleic acid barcode molecule **1310** to generate (e.g., via a nucleic acid reaction, such as nucleic acid extension or ligation) a barcoded nucleic acid molecule comprising cell (e.g., partition specific) barcode sequence **1312** (or a reverse complement thereof) and reporter barcode sequence **1212** (or a reverse complement thereof). Nucleic acid molecules derived from a cell (such as RNA molecules) can be similarly processed to append the cell (e.g., partition-specific) barcode sequence **1312** to these molecules or derivatives thereof (e.g., cDNA molecules). For example, referring to **FIG. 13C,** in some embodiments, nucleic acid barcode molecule **1320** comprises sequence **1323** complementary to a sequence of RNA molecule **1260** from a cell. In some instances, sequence **1323** comprises a poly-T sequence. In other instances, sequence **1323** comprises a sequence complementary to a target sequence in an RNA molecule. In some instances, sequence **1323** comprises a sequence complementary to a region of an immune molecule, such as the constant region of a TCR or BCR sequence. Sequence **1323** is hybridized to RNA molecule **1260** and a barcoded cDNA molecule is generated in a reverse transcription reaction comprising cell (e.g., partition specific) barcode sequence **1323** (or a reverse complement thereof) and a cDNA sequence corresponding to mRNA **1260** (or a portion thereof). Barcoded nucleic acid molecules can then be optionally processed as described elsewhere herein, e.g., to amplify the molecules and/or append sequencing platform specific sequences to the fragments. See, e.g., U.S. Pat. Pub. 20180105808. Barcoded nucleic acid molecules, or derivatives generated therefrom, can then be sequenced on a suitable sequencing platform. Nucleic acid sequences of interest can be identified from the sequence data. Such nucleic acid sequences of interest can be enriched from the barcoded nucleic acid molecules or derivatives generated therefrom, according to methods disclosed herein.

## Computer systems

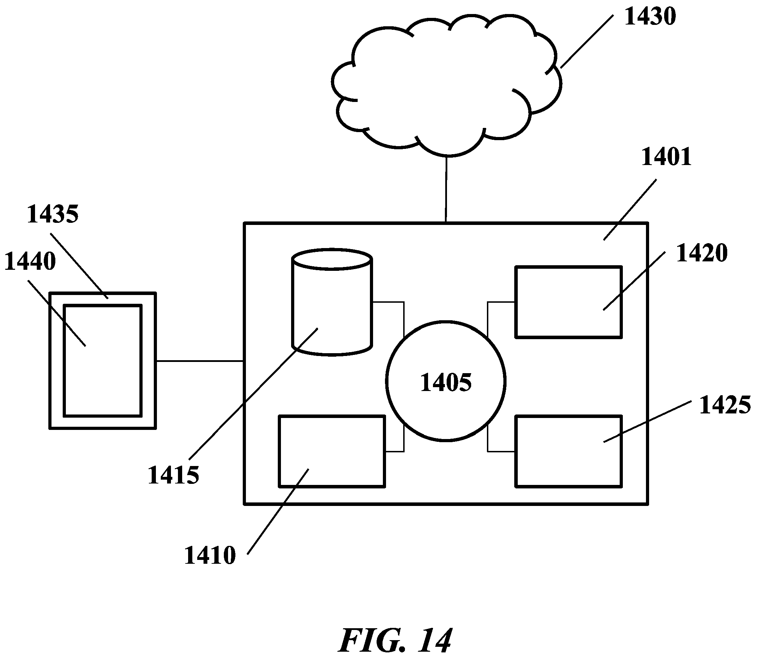

The present disclosure provides computer systems that are programmed to implement methods of the disclosure. **FIG. 14** shows a computer system **1401** that is programmed or otherwise configured to (i) design a nucleic acid primer as described herein, control an amplification reaction as provided herein, execute cloning and/or expression of a nucleic acid sequence of interest and/or protein product of a nucleic acid sequence of interest provided herein, or analyze a protein product of a nucleic acid sequence of interest provided herein. The computer system **1401** can regulate various aspects of the present disclosure, such as, for example, amount of primer, buffer, nucleic acid, or other reagent added to an amplification reaction, thermocycling of an amplification reaction, conditions for introducing an enriched nucleic acid sequence of interest to a vector, conditions for expressing a protein product of a nucleic acid sequence of interest, and/or providing reagents and/or adjusting conditions for an experiment for analysis of a protein product of a nucleic acid sequence of interest. The computer system **1401** can be an electronic device of a user or a computer system that is remotely located with respect to the electronic device. The electronic device can be a mobile electronic device.

The computer system **1401** includes a central processing unit (CPU, also "processor" and "computer processor" herein) **1405,** which can be a single core or multi core processor, or a plurality of processors for parallel processing. The computer system **1401** also includes memory or memory location **1410** (e.g., random-access memory, read-only memory, flash memory), electronic storage unit **1415** (e.g., hard disk), communication interface **1420** (e.g., network adapter) for communicating with one or more other systems, and peripheral devices **1425,** such as cache, other memory, data storage and/or electronic display adapters. The memory **1410,** storage unit **1415,** interface **1420** and peripheral devices **1425** are in communication with the CPU **1405** through a communication bus (solid lines), such as a motherboard. The storage unit **1415** can be a data storage unit (or data repository) for storing data. The computer system **1401** can be operatively coupled to a computer network ("network") **1430** with the aid of the communication interface **1420.** The network **1430** can be the Internet, an internet and/or extranet, or an intranet and/or extranet that is in communication with the Internet. The network **1430** in some cases is a telecommunication and/or data network. The network **1430** can include one or more computer servers, which can enable distributed computing, such as cloud computing. The network **1430,** in some cases with the aid of the computer system **1401,** can implement a peer-to-peer network, which may enable devices coupled to the computer system **1401** to behave as a client or a server.

The CPU **1405** can execute a sequence of machine-readable instructions, which can be embodied in a program or software. The instructions may be stored in a memory location, such as the memory **1410.** The instructions can be directed to the CPU **1405,** which can subsequently program or otherwise configure the CPU **1405** to implement methods of the present disclosure. Examples of operations performed by the CPU **1405** can include fetch, decode, execute, and writeback.

The CPU **1405** can be part of a circuit, such as an integrated circuit. One or more other components of the system **1401** can be included in the circuit. In some cases, the circuit is an application specific integrated circuit (ASIC).

The storage unit **1415** can store files, such as drivers, libraries and saved programs. The storage unit **1415** can store user data, e.g., user preferences and user programs. The computer system **1401** in some cases can include one or more additional data storage units that are external to the computer system **1401,** such as located on a remote server that is in communication with the computer system **1401** through an intranet or the Internet.

The computer system **1401** can communicate with one or more remote computer systems through the network **1430.** For instance, the computer system **1401** can communicate with a remote computer system of a user (e.g., operator). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system **1401** via the network **1430.**

Methods as described herein can be implemented by way of machine (e.g., computer processor) executable code stored on an electronic storage location of the computer system **1401,** such as, for example, on the memory **1410** or electronic storage unit **1415.** The machine executable or machine readable code can be provided in the form of software. During use, the code can be executed by the processor **1405.** In some cases, the code can be retrieved from the storage unit **1415** and stored on the memory **1410** for ready access by the processor **1405.** In some situations, the electronic storage unit **1415** can be precluded, and machine-executable instructions are stored on memory **1410.**

The code can be pre-compiled and configured for use with a machine having a processor adapted to execute the code, or can be compiled during runtime. The code can be supplied in a programming language that can be selected to enable the code to execute in a pre-compiled or as-compiled fashion.

Aspects of the systems and methods provided herein, such as the computer system **1401,** can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **1401** can include or be in communication with an electronic display **1435** that comprises a user interface (UI) **1440** for providing, for example, enrichment yield, results of analysis of a protein product of a nucleic acid sequence of interest, etc. Examples of UIs include, without limitation, a graphical user interface (GUI) and web-based user interface.

Methods and systems of the present disclosure can be implemented by way of one or more algorithms. An algorithm can be implemented by way of software upon execution by the central processing unit **1405.** The algorithm can, for example, control enrichment of a nucleic acid sequence of interest, control cloning of a nucleic acid sequence of interest, and/or assess or analyze a protein product of a nucleic acid sequence of interest.

Devices, systems, compositions and methods of the present disclosure may be used for various applications, such as, for example, processing a single analyte (e.g., RNA, DNA, or protein) or multiple analytes (e.g., DNA and RNA, DNA and protein, RNA and protein, or RNA, DNA and protein) from a single cell. For example, a biological particle (e.g., a cell or cell bead) is partitioned in a partition (e.g., droplet), and multiple analytes from the biological particle are processed for subsequent processing. The multiple analytes may be from the single cell. This may enable, for example, simultaneous proteomic, transcriptomic and genomic analysis of the cell.

## Examples

### Example 1: Generation of a cDNA library of antibodies

A library of cDNA molecules encoding a plurality of antibodies can be created. A plurality of B cells expressing antibodies can be isolated, for example in partitions with not more than one cell per partition. Cells can be lysed or permeabilized, and nucleic acids (e.g., RNA) coding for the antibodies can be isolated and tagged with an identification sequence comprising a barcode, a template switch oligonucleotide, and a unique molecular identifier as described in, e.g., FIG. 12B or FIG. 12C. The identification sequence can be used to identify the sample that a given nucleic acid originates from. RNA can be reverse transcribed to yield cDNA, and cDNA can be pooled to yield a cDNA library.

### Example 2: Amplification of a target cDNA of the cDNA library

A cDNA of the cDNA library (e.g., a whole transcriptome barcoded gene expression library) can be identified as a cDNA of interest. For example, a cDNA can be identified as corresponding to an antibody that has a desired activity, or can specifically bind or neutralize an antigen (e.g., using the labelling agents described elsewhere herein).

cDNA can be enriched using a PCR protocol. For example, a cDNA library can be incubated with a primer pair, a polymerase, nucleotides, and buffer. A primer pair can comprise a first primer at least partially complementary to the barcode of one of the cDNA library, and a second primer at least partially complementary to a sequence that is complementary to a sequence downstream of the barcode (e.g., in the constant region). cDNA can be subjected to thermocycling for between 15 cycles and 40 cycles, until the cDNA is enriched.

*Example 3: Further enrichment of a target cDNA*

A cDNA can be further enriched from the library, for example to increase the abundance of sequences of interest as compared to other cDNA molecules in the library and/or enriched product of Example 2.

Further enrichment can be performed using a second PCR reaction. For example, the enriched cDNA from Example 2 can be incubated with a primer pair, a polymerase, nucleotides, and buffer. A primer pair can comprise a first primer at least partially complementary to the V(D)J region of one of the cDNA library, and a second primer at least partially complementary to a sequence that is complementary to a sequence downstream of the V(D)J sequence (e.g., in the constant region). cDNA can be subjected to thermocycling for between 15 cycles and 40 cycles, until the cDNA is enriched.

*Example 4: Nested PCR*

A double (e.g., nested) PCR strategy can be employed for the enrichment of a nucleic acid sequence of interest. An example of a nested PCR scheme is illustrated in **FIG. 10.** In this example, a cDNA molecule of interest is illustrated which comprises a first read sequence, a barcode sequence (identified as a "10 X barcode sequence, which may be a partition-specific barcode), a unique molecular identifier sequence (UMI), a template switch oligonucleotide (TSO), a V sequence, a D sequence, a J sequence, a constant (C) sequence, and a second read sequence. Primers can be designed to enrich the sequence for an antibody (i.e., the V, D, J, and C sequences) using a double PCR strategy. Such a double PCR strategy can employ a first enrichment step and a second enrichment step. The outer F (forward) and outer R (reverse) primers can be primers employed for a first PCR enrichment step to enrich one of a plurality of cDNA molecules comprising barcodes. The outer F primer can comprise a sequence complementary to the barcode, and the outer R primer can comprise a sequence complementary to the complement of the second read sequence. The inner F (forward) and inner R (reverse) primers can be employed for a second enrichment step to further enrich the product of the first enrichment step. The inner F primer can be complementary to the V sequence, and optionally part of the TSO sequence. The inner R primer can be complementary to the C sequence and the J sequence. In some cases, the inner F and inner R primers can include non-binding handles that can allow cloning into a vector or enable pairing of sequences, for example using overlap extension.

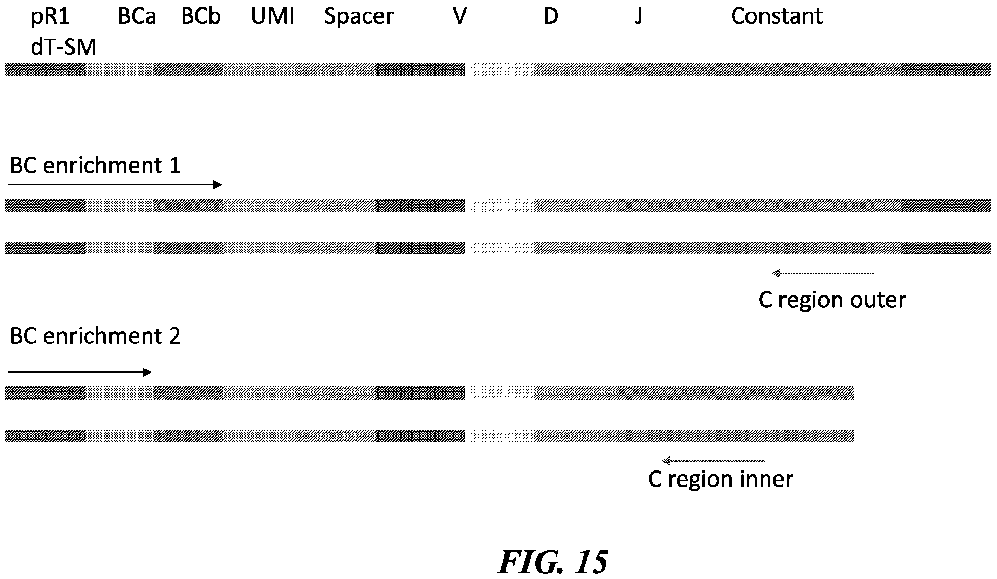

An example of primer design scheme for the first enrichment step and second enrichment step is provided in **FIG. 15.** Here, the primers shown for a first enrichment step can be used for a first PCR reaction, and primers shown for a second enrichment step can be used for a second PCR reaction.

*Example 5: Producing a clonable sequence.*

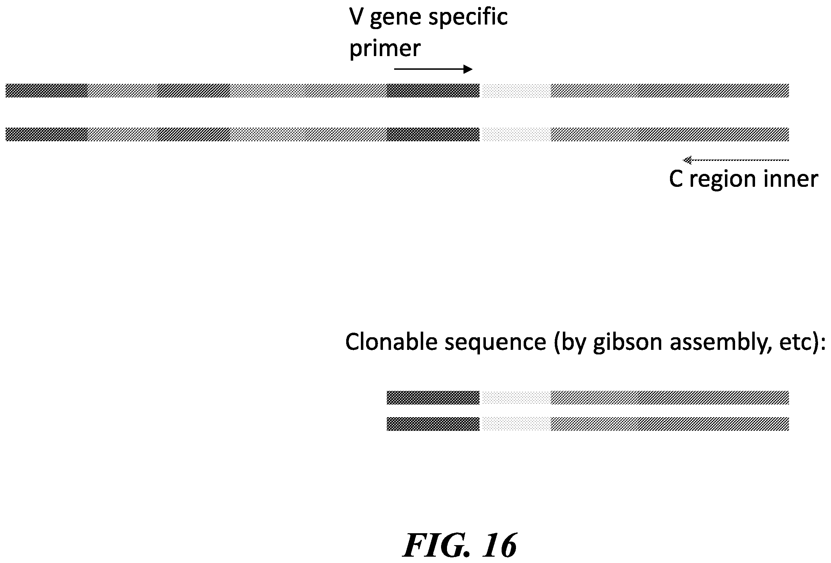

A sequence of a nucleic acid sequence of interest can be extracted to yield a clonable sequence. For example, primers can be designed to yield a clonable sequence (e.g., a sequence coding for an amino acid fragment) from enriched cDNA (e.g., the enriched library from Example 4). An example of primer design that can yield a clonable sequence is provided in **FIG. 16.** This can be accomplished, for example, by utilizing a forward primer that is V gene specific (e.g., specific to a V sequence) and a reverse primer specific to a constant sequence. The resultant nucleic acid molecule, shown in the bottom panel, can be cloned into a vector for expression or analysis. The expression vector may be configured to comprise a constant region sequence (or a portion thereof) such that, when cloned into the expression vector, the enriched V(D)J molecules (such as a paired light and heavy antibody chain) can be expressed as a fully functional immune molecule (e.g., comprising a full, intact constant region).

*Example 6: Cloning an enriched nucleic acid sequence.*

B cells (e.g., single B cells) can be captured, (e.g. partitioned with a barcoded bead), for example using techniques provided herein. The interior of cells can be accessed, for example by lysing or permeablizing the cells, and RNA of the cells can be reverse transcribed to generate barcoded cDNA from the RNA sequences. See, e.g., **FIG. 12B** or **FIG. 13C** and accompanying text. This can be performed, for example, by 2 rounds of targeted amplification; the first or second amplifications or the full-length unfragmented cDNA can be used in the following step(s). In some implementations, the partition can comprise a cell barcode and TSO sequence. In some implementations, a partition can comprise a cell barcode, UMI sequence as provided herein, and a TSO sequence as provided herein.

The resulting nucleic acid sequence (e.g., full length or a fragment thereof) can be sequenced. Sequencing can yield one or more paired heavy and light chain sequences (e.g., heavy and light chain sequence pairs) associated with a specific cell barcode. Some of the input cDNA subject to targeted amplification can be saved for later use (e.g., for capture of a specific input cDNA or other use).

One or more probes can be designed to target one or more V(D)J junction regions, which can comprise highly unique nucleotide sequences 60-150 base pairs in length. See, e.g., **FIG. 19.** Similarly, one or more probes can be designed to target the corresponding cell barcodes, or cell barcode and a chosen UMI sequence. These probes can be captured for example using a streptavidin/biotinylation approach, where the probes can be are annealed to the cDNA, and fragments not annealing to the probes can be washed away. In some embodiments, other suitable capture techniques can also be employed. In some cases, probes can be fluorescent, which can enable droplet sorting. In some such cases, the addition of probe reagents and annealing to existing nucleic acid(s) in the droplet can enable selection of droplet(s) of interest for further amplification or cloning. In some cases, a hydrogel can be selectively formed in a droplet containing a probe of interest. Such a hydrogen can be used as part of an enrichment step. Probes can be used to target specific V genes or J genes in addition to or without junction-specific probes.

Using the retained DNA of interest (containing sequences which the probes successfully annealed to), specific heavy and light chains can be amplified, for example by one or more rounds of PCR or linear amplification. Amplification can comprise targeting with forward primers the against one or more of the cell barcode, UMI, 5' UTR, and leader sequence; one or more of the cell barcode, UMI, 5' UTR, the cell barcode, UMI, the cell barcode, and the 5' UTR, or a region of the V gene (such as the framework region) and with reverse primers the constant region of the targeted antibodies, or a combination thereof. A primer can comprise overlap extension linkers to physically connect the targeted heavy and light chains, or to introduce restriction or Gibson assembly sites for optimized cloning.

In some cases, for example if the number of antibodies being targeted is large, a set of unique overlap extension or linker molecules can be designed in a plate-based reaction. Such overlap extension or linker molecules can be used to introduce clone-specific molecular tags.

*Example 7: Specific enrichment of BCR sequences from a pooled cDNA library*

*Sample selection:* A library of BCR enriched product from cells selected by PE+/APC+ gate was used as template in the nested PCR reactions. Two negative controls were included to verify product specificity: BCR enriched product from antigen negative cells from the same donor as the sample used to generate the target clones and BCR enriched product from purified B cells from a different donor.

*Clone selection for amplification:*

Nested PCR reactions were performed on the BCR enriched product (and negative controls) to enrich for sequences of four antigen-specific clonotypes, e.g., antibody sequences, in the library. The antigen-specific clonotypes were selected as belonging to one of four categories: (1) expanded clonotype with multiple unique subclones (Clone A) (2); expanded clonotype with a single unique subclone (Clone B); (3) single cell clonotype with many valid UMIs (Clone C); and (4) Single cell clonotypes with few valid UMIs (Clone D).

*Primer design:*

Commercially available software (Geneious Prime, primer3) was used to generate the primer sequences to be free of typical sequence weakness (such as hairpin Tm, self dimer Tm, and pair dimer Tm). Primers for the nested PCR reaction were designed to target: (1) in an outer reaction, the cell barcode and UMI (forward primer) and isotype and J region (reverse primer); and (2) in an inner reaction, the leader peptide or FWR1 (forward primer) and CDR3/junction (potentially extending into the J region, if necessary; reverse primer). Primer pairs were selected based on compatibility of the inner and outer pairs.

Default settings for Geneious Prime 2021.1.1 using primer3 Tm settings were as described in Santa Lucia et al. 1998 and salt correction settings as described in Owczarzy et al. 2004 were used. Monovalent, divalent, oligo, and dNTP concentrations were set to 50 mM, 1.5 mM, 50 nM, and 0.6 mM respectively. The minimum size allowed for each primer was 18 nucleotides, with a maximum of 27, and an optimal length of 20 nucleotides. The minimum, maximum, and optimum Tms for each primer were set to 57, 60, and 63°C. The allowed GC% content minimum, maximum, and optimum were set to 20, 80, and 50%. The maximum permitted dimer Tm was 47°C. The maximum permitted Tm difference was 100°C.

*Amplification reactions:*

Outer primer sequences targeting Cell Barcode + UMI and framework 4 regions were used for the first round of PCR for each of the 4 clones described above. Each amplification reaction contained 10nM of each primer, 1uL BCR enriched product, 25uM betaine (to increase polymerase processivity), and 50uL 2X hot start high fidelity PCR master mix in a total volume of 100uL. These were amplified for 10 cycles total, with annealing temperatures appropriate to the primer pair used (51-54C) and a 1 minute 72C extension. Reactions were cleaned up using 0.6X SPRIselect, and the entire volume was put into the second PCR. This reaction included 10nM of each inner primer (targeting leader and framework 4/constant regions), the amplified product from the first PCR, 25uM betaine, and 50uL 2X hot start high fidelity PCR master mix in a total volume of 100uL. These were amplified for 10 cycles total, with annealing temperatures appropriate to the primer pair used (54C) and a 1 minute 72C extension. Reactions were cleaned up using 0.6X SPRIselect.

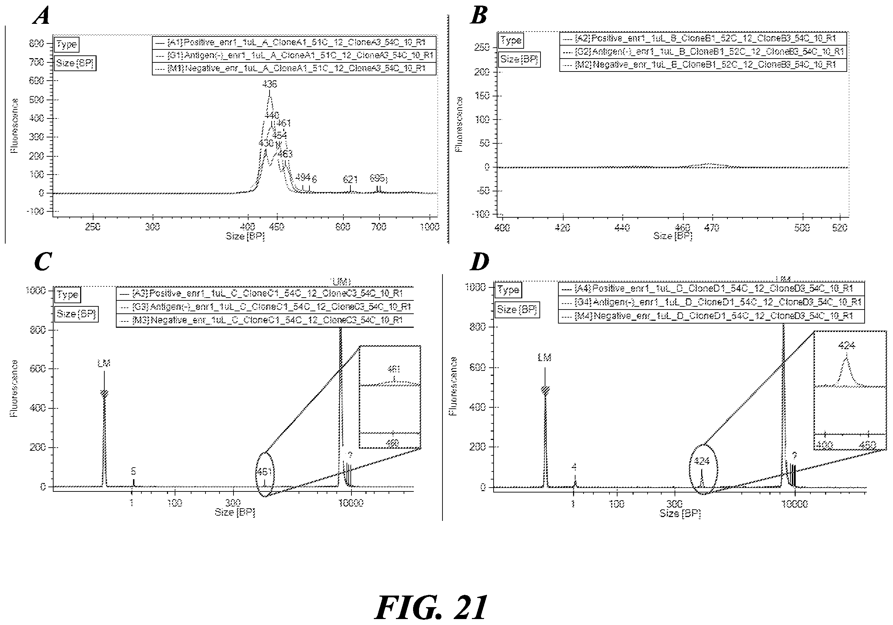

Nested PCR product was run on BioA and/or Labchip to assess product size and specificity. This process confirmed specific product in only antigen positive B cells for clones B (Fig. 21B), C (Fig. 21C), and D (Fig. 21D). The product for clone C was more varied in size and appeared in negative controls as well, suggesting more non-specific amplification for this clone. Results also showed several products for clone A, which was expected given clonotype A was associated with multiple unique subclones (FIG. 21A).

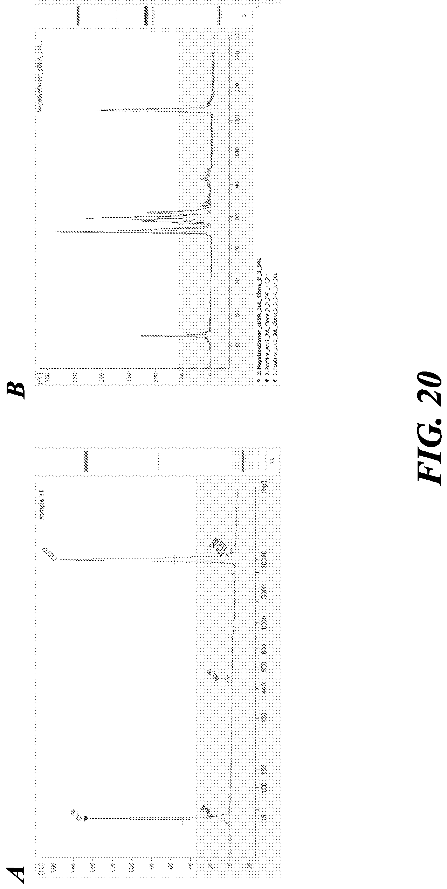

In addition, a single step PCR using the conditions and primers described or the second step of the nested PCR was run and analyzed as before. Significantly more nonspecific product was observed for all clones tested, demonstrating the advantages of the nested approach in improving specificity. See, for example, **Fig. 20A** and **B.**

*Sequencing:*

Finally, full length enriched clone sequences were converted into sequencing libraries using the commercially available Prism library prep kit from IDT and sequenced on a paired end 300bp MiSeq run to assay sequence purity.

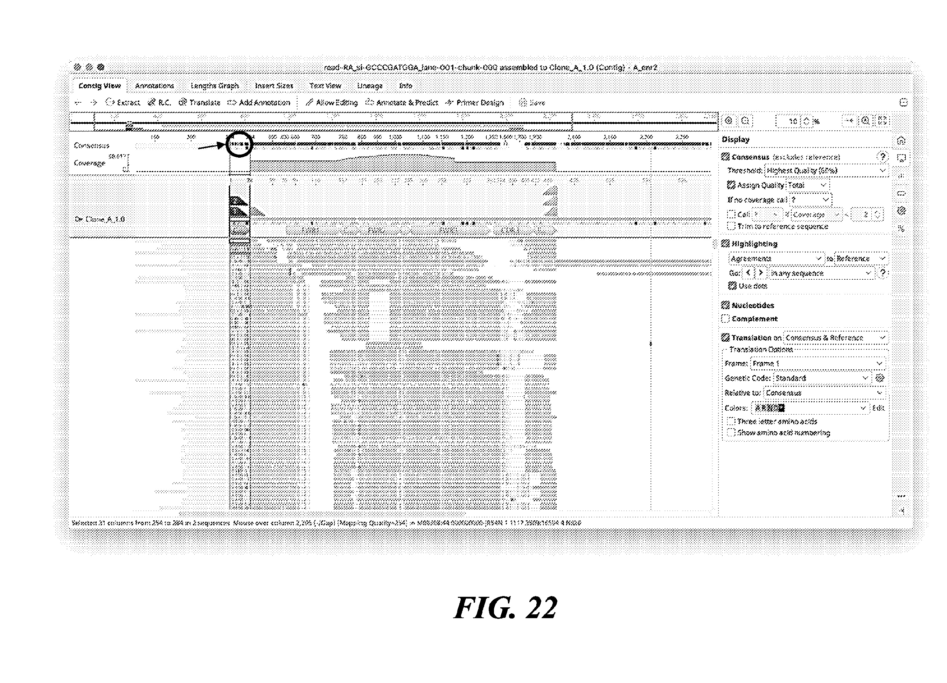

**FIG. 22** shows sequencing results of the enrichment products following nested amplification for a nucleic acid sequence of interest from a pooled barcoded cDNA library, e.g., a target nucleic acid sequence encoding a fragment of a BCR produced from Clone A (an expanded clonotype with multiple subclonotypes), when the forward outer primer lacked sufficient specificity. The consensus region from positions 254-284 depict the cell barcode + UMI region (indicated by circling) targeted by the forward outer primer. As shown, the consensus for the cell barcode + UMI had several variant positions, indicating poor forward outer primer specificity for the selected barcode/UMI combination. The results indicate retrieval of off-target sequences, due to off-target binding of cDNA library members having multiple cell barcode/UMI combinations.

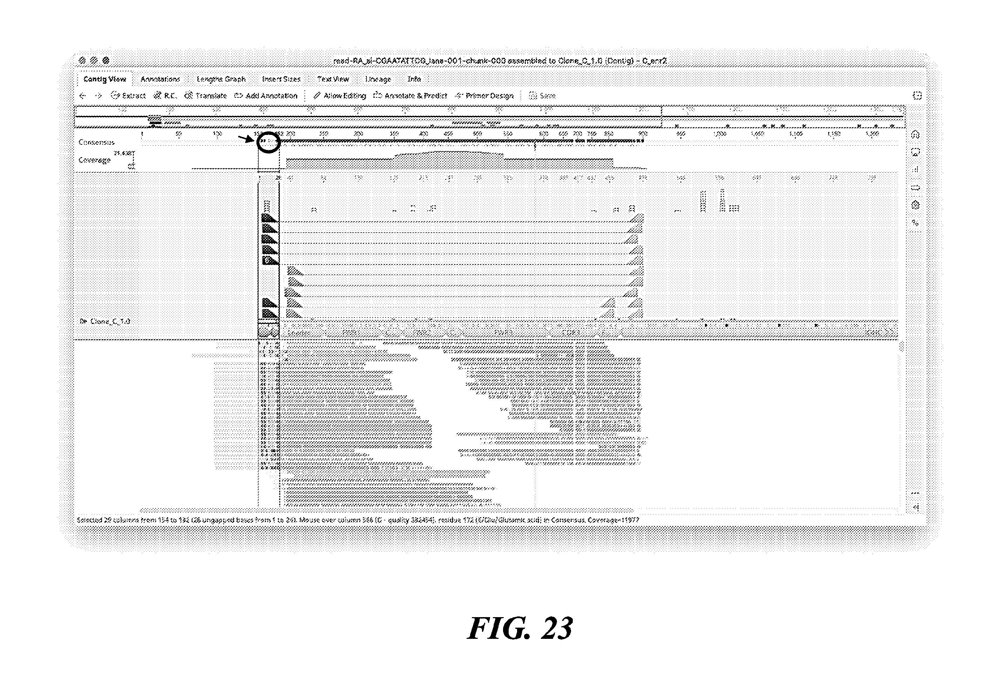

**FIG. 23** shows sequencing results of the enrichment products following nested amplification for a nucleic acid sequence of interest from a pooled barcoded cDNA library, e.g., a target nucleic acid sequence encoding a fragment of a BCR produced from Clone C (a single cell clone with many valid UMIs), when the forward outer primer lacked sufficient specificity. As shown (circled in consensus sequence), the cell barcode + UMI region largely lacked consensus, indicating poor forward outer primer specificity for the selected barcode/UMI combination. The consensus for the BCR sequence of interest had two variant positions in the CDR3 region, indicating retrieval of off-target sequences, due to binding of cDNA library members having multiple cell barcode/UMI combinations.

Similarly, sequencing results of the enrichment products produced from Clone D (a single cell clone with few valid UMIs) following nested amplification from the pooled barcoded cDNA library indicated that the forward outer primer lacked specificity for the cell barcode + UMI combination, and retrieved off-target sequences (data not shown).

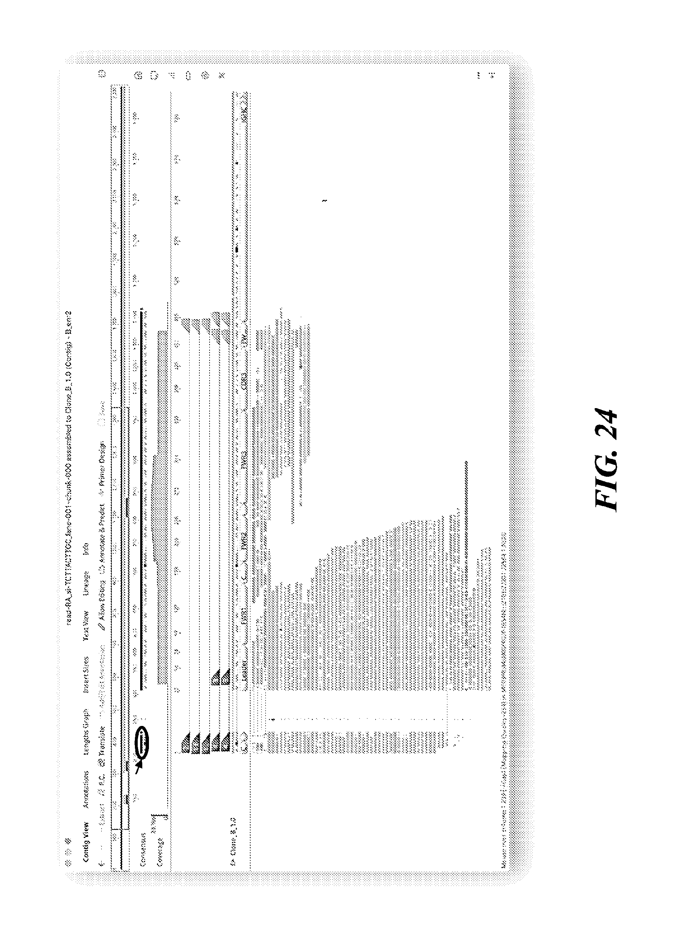

**FIG. 24** shows sequencing results of the enrichment products following nested amplification for a nucleic acid sequence of interest from a pooled barcoded cDNA library, e.g., a target nucleic acid sequence encoding a fragment of a BCR produced from Clone B (an expanded clonotype with a single unique subclone), when the forward outer primer bound with sufficient specificity to the cell barcode and UMI. As shown (circled in consensus sequence), the consensus for the cell barcode + UMI region had a single variant position, demonstrating that the forward outer primer bound with sufficient specificity to the cell barcode and UMI. Also as shown, the consensus for the BCR fragment had no variant positions, indicating successful retrieval of the full sequence of interest from the barcoded cDNA library with the nested amplification approach when the forward outer primer bound with sufficient specificity to the cell barcode and UMI.

**Claims**

1.   A method for enriching for a nucleic acid sequence of interest, comprising:

(a) providing a plurality of nucleic acid molecules comprising a plurality of identification sequences, wherein a nucleic acid molecule of said plurality of nucleic acid molecules comprises:

(i) an identification sequence of said plurality of identification sequences that identifies said nucleic acid molecule,
wherein the identification sequence comprises a barcode sequence, and/or a unique molecule identifier (UMI) sequence; and
(ii) the nucleic acid sequence of interest,
wherein the nucleic acid sequence of interest comprises a nucleic acid sequence coding for a T cell receptor (TCR), a B cell receptor (BCR), or a fragment thereof;

(b) using a first nucleic acid primer complementary to at least a portion of the identification sequence in a first round of amplification to amplify said nucleic acid sequence of interest; and
(c) using a second nucleic acid primer complementary to at least a portion of a V(D)J sequence in a second round of amplification to further amplify said nucleic acid sequence of interest.

2.  The method of claim 1, wherein the identification sequence comprises the barcode sequence.

3.  The method of claim 1 or 2, wherein the identification sequence comprises the UMI sequence.

4.  The method of any preceding claim, wherein the first round of amplification further comprises using a third primer comprising a sequence complementary to at least a portion of a complement of a nucleic acid sequence coding for a constant region of the TCR, BCR, or fragment thereof, optionally a sequence complementary to at least a portion of a complement of a nucleic acid sequence coding for a constant region of the BCR, or fragment thereof.

5.  The method of any preceding claim, wherein the second round of amplification further comprises using a fourth primer comprising a sequence complementary to at least a portion of a complement of a nucleic acid sequence coding for a sequence downstream of the V(D)J sequence, optionally wherein the fourth primer comprises a non-binding handle.

6.  The method of any preceding claim, wherein the second primer comprises a non-binding handle.

7.  The method of any preceding claim, wherein the plurality of provided nucleic acid molecules comprise complementary deoxyribonucleic acid (cDNA) molecules.

8.  The method of any preceding claim, wherein the nucleic acid sequence of interest codes for the BCR, or fragment thereof.

9.  A method for enriching a nucleic acid sequence of interest, comprising:

   (a) providing a plurality of nucleic acid molecules comprising a plurality of identification sequences, wherein a nucleic acid molecule of said plurality of nucleic acid molecules comprises:

      (i) identification sequence of said plurality of identification sequences that identifies said nucleic acid molecule,
      wherein the identification sequence comprises a barcode sequence and a unique molecule identifier (UMI) sequence; and
      (ii) the nucleic acid sequence of interest,
      wherein the nucleic acid sequence of interest comprises a nucleic acid sequence encoding a B cell receptor (BCR) or a fragment thereof;

   (b) performing a first amplification reaction with a first set of primers comprising:

      a first primer comprising a sequence complementary to at least a portion of the barcode sequence and/or the UMI sequence, and
      a second primer comprising a sequence complementary to a complement of at least a portion of the nucleic acid sequence of interest that encodes a junction (J) region and/or isotype region of the BCR or fragment thereof;

   (c) performing a second amplification reaction with a second set of primers comprising:

      a third primer comprising a sequence complementary to nucleotides of at least a portion of the leader sequence and/or encoding framework region (FWR)1 of the BCR, or fragment thereof; and
      a fourth primer comprising a sequence complementary to a complement of at least a portion of the nucleic acid sequence of interest that encodes a complementarity region (CDR)3, a FWR4, a J region, a D region, and/or a V region, or a junction between any one or more thereof, of the BCR or fragment thereof.

10. The method of claim 9, wherein the first primer comprises:

   (i) a sequence complementary to at least a portion of the barcode sequence and the UMI sequence; or

(ii) a sequence complementary to the barcode sequence and the UMI sequence.

11. The method of claim 9 or claim 10, wherein the second primer comprises:

(i) a sequence complementary to the complement of the nucleic acid sequence of interest that encodes at least a portion of the J region of the BCR or fragment thereof;
(ii) a sequence complementary to the complement of the nucleic acid sequence of interest that encodes at least a portion of the isotype region of the BCR or fragment thereof; or
(iii) a sequence complementary to the complement of the nucleic acid sequence of interest that encodes at least a portion of the J region and the isotype region of the BCR or fragment thereof.

12. The method of any of claims 9-11, wherein the third primer comprises:

(i) a sequence complementary to nucleotides of at least a portion of the leader sequence of the BCR, or fragment thereof; or
(ii) a sequence complementary to nucleotides encoding at least a portion of the FWR1 of the BCR, or fragment thereof.

13. The method of any of claims 9-12, wherein the fourth primer comprises:

(i) a sequence complementary to the complement of at least a portion of the nucleic acid sequence of interest that encodes the CDR3, and junction extending into the J region of the BCR or fragment thereof;
(ii) a sequence complementary to the complement of at least a portion of the nucleic acid sequence of interest that encodes the D and J regions, or a junction between the D and J regions of the BCR or fragment thereof;
(iii) a sequence complementary to the complement of at least a portion of the nucleic acid sequence of interest that encodes the V and J regions, or a junction between the V and J regions, of the BCR or fragment thereof;
(iv) a sequence complementary to the complement of at least a portion of the nucleic acid sequence of interest that encodes the V and D regions, or a junction between the V and D regions, of the BCR or fragment thereof; or
(v) a sequence complementary to the complement of at least a portion of the nucleic acid sequence of interest that encodes the V, D and J regions of the BCR or fragment thereof.

14. The method of any of claims 9-13:

(i) wherein the third and the fourth primer comprise a non-binding handle; and/or
(ii) further comprising cloning the enriched nucleic acid sequence of interest in a vector.

15. The method of any preceding claim, wherein the plurality of nucleic acid molecules are prepared from a cell sample of a donor or donors, optionally wherein:

(i) the donor or donors had been exposed to a target antigen and wherein the plurality of nucleic acid molecules that comprise the nucleic acid sequence of interest comprise a nucleic acid sequence encoding a selected B cell receptor (BCR) or a fragment thereof that binds the target antigen; or
(ii) the plurality of nucleic acid molecules comprising the nucleic acid sequence encoding the selected B cell receptor (BCR), or fragment thereof, that binds the target antigen are prepared from the cell sample of the donor or donors comprising steps of:

(a) partitioning a reaction mixture into a plurality of partitions, wherein the reaction mixture comprises:

(i) a plurality of cells of the cell sample, and
(ii) the target antigen,
wherein the target antigen is coupled to a reporter oligonucleotide,
wherein the reaction mixture comprises a cell bound to the target antigen,
wherein the partitioning provides a partition comprising:

(i) the partitioned cell bound to the target antigen, and
(ii) a plurality of nucleic acid barcode molecules comprising a partition-specific barcode sequence,

(b) in the partition, generating barcoded nucleic acid molecules, wherein the barcoded nucleic acid molecules comprise:

(i) a first barcoded nucleic acid molecule comprising a sequence of the reporter oligonucleotide or a reverse complement thereof and the partition-specific barcode sequence or reverse complement thereof, and
(ii) a second barcoded nucleic acid molecule comprising a nucleic acid molecule of interest for inclusion in the plurality of nucleic acid molecules if the first barcoded nucleic molecule is detected.

**Patentansprüche**

1. Verfahren zur Anreicherung für eine Nukleinsäuresequenz von Interesse, umfassend:

(a) Bereitstellen einer Vielzahl von Nukleinsäuremolekülen, die eine Vielzahl von Identifikationssequenzen umfasst, wobei ein Nukleinsäuremolekül der Vielzahl von Nukleinsäuremolekülen umfasst:

(i) eine Identifikationssequenz der Vielzahl von Identifikationssequenzen, die das Nukleinsäuremolekül identifiziert,
wobei die Identifikationssequenz eine Barcode-Sequenz und/oder eine eindeutige molekulare Identifikator(UMI)-Sequenz umfasst; und
(ii) die Nukleinsäuresequenz von Interesse,
wobei die Nukleinsäuresequenz von Interesse eine Nukleinsäuresequenz umfasst, die für einen T-Zellrezeptor (TCR), einen B-Zellrezeptor (BCR) oder ein Fragment davon codiert;

(b) Verwenden eines ersten Nukleinsäureprimers, der zu mindestens einem Teil der Identifikationssequenz komplementär ist, in einer ersten Amplifikationsrunde, um die Nukleinsäuresequenz von Interesse zu amplifizieren; und
(c) Verwenden eines zweiten Nukleinsäureprimers, der zu mindestens einem Teil einer V(D)J-Sequenz komplementär ist, in einer zweiten Amplifikationsrunde, um die Nukleinsäuresequenz von Interesse weiter zu amplifizieren.

2. Verfahren nach Anspruch 1, wobei die Identifikationssequenz die Barcode-Sequenz umfasst.

3. Verfahren nach Anspruch 1 oder 2, wobei die Identifikationssequenz die UMI-Sequenz umfasst.

4. Verfahren nach einem der vorhergehenden Ansprüche, wobei die erste Amplifikationsrunde ferner die Verwendung eines dritten Primers umfasst, der eine Sequenz umfasst, die zu mindestens einem Teil eines Komplements einer Nukleinsäuresequenz komplementär ist, die für eine konstante Region des TCR, BCR oder des Fragments davon codiert, optional eine Sequenz, die zu mindestens einem Teil eines Komplements einer Nukleinsäuresequenz komplementär ist, die für eine konstante Region des BCR oder des Fragments davon codiert.

5. Verfahren nach einem der vorhergehenden Ansprüche, wobei die zweite Amplifikationsrunde ferner die Verwendung eines vierten Primers umfasst, der eine Sequenz umfasst, die zu mindestens einem Teil eines Komplements einer Nukleinsäuresequenz komplementär ist, die für eine Sequenz stromabwärts der V(D)J-Sequenz codiert, wobei der vierte Primer optional einen nicht bindenden Abschnitt umfasst.

6. Verfahren nach einem der vorhergehenden Ansprüche, wobei der zweite Primer einen nicht bindenden Abschnitt umfasst.

7. Verfahren nach einem der vorhergehenden Ansprüche, wobei die Vielzahl von bereitgestellten Nukleinsäuremolekülen komplementäre Desoxyribonukleinsäure(cDNA)-Moleküle umfasst.

8. Verfahren nach einem der vorhergehenden Ansprüche, wobei die Nukleinsäuresequenz von Interesse für den BCR oder ein Fragment davon codiert.

9. Verfahren zur Anreicherung einer Nukleinsäuresequenz von Interesse, umfassend:

(a) Bereitstellen einer Vielzahl von Nukleinsäuremolekülen, die eine Vielzahl von Identifikationssequenzen umfasst, wobei ein Nukleinsäuremolekül der Vielzahl von Nukleinsäuremolekülen umfasst:

(i) Identifikationssequenz der Vielzahl von Identifikationssequenzen, die das Nukleinsäuremolekül identifiziert,
wobei die Identifikationssequenz eine Barcode-Sequenz und eine eindeutige molekulare Identifikator(UMI)-Sequenz umfasst; und
(ii) die Nukleinsäuresequenz von Interesse,
wobei die Nukleinsäuresequenz von Interesse eine Nukleinsäuresequenz umfasst, die einen B-Zellrezeptor (BCR) oder ein Fragment davon codiert;

(b) Durchführen einer ersten Amplifikationsreaktion
mit einem ersten Satz von Primern, umfassend:

einen ersten Primer, der eine Sequenz umfasst, die zu mindestens einem Teil der Barcode-Sequenz und/oder der UMI-Sequenz komplementär ist, und
einen zweiten Primer, der eine Sequenz umfasst, die zu einem Komplement von mindestens einem Teil der Nukleinsäuresequenz von Interesse komplementär ist, die eine Übergangsregion (J-Region) und/oder eine Isotyp-Region des BCR oder des Fragments davon codiert;

(c) Durchführen einer zweiten Amplifikationsreaktion
mit einem zweiten Satz von Primern, umfassend:

einen dritten Primer, der eine Sequenz umfasst, die komplementär zu Nukleotiden von mindestens einem Teil der Leadersequenz ist und/oder die Framework-Region (FWR)1 des BCR oder des Fragments davon codiert; und
einen vierten Primer, der eine Sequenz umfasst, die zu einem Komplement von mindestens einem Teil der Nukleinsäuresequenz von Interesse komplementär ist, die eine Komplementaritätsregion (CDR)3, eine FWR4, eine J-Region, eine D-Region und/oder eine V-Region oder einen Übergang zwischen einer oder mehreren davon des BCR oder des Fragments davon codiert.

10. Verfahren nach Anspruch 9, wobei der erste Primer ferner umfasst:

(i) eine Sequenz, die zu mindestens einem Teil der Barcode-Sequenz und der UMI-Sequenz komplementär ist; oder
(ii) eine Sequenz, die zu der Barcode-Sequenz und der UMI-Sequenz komplementär ist.

11. Verfahren nach Anspruch 9 oder Anspruch 10, wobei der zweite Primer umfasst:

(i) eine Sequenz, die zu dem Komplement der Nukleinsäuresequenz von Interesse komplementär ist, die mindestens einen Teil der J-Region des BCR oder des Fragments davon codiert;
(ii) eine Sequenz, die zu dem Komplement der Nukleinsäuresequenz von Interesse komplementär ist, die mindestens einen Teil der Isotyp-Region des BCR oder des Fragments davon codiert; oder
(iii) eine Sequenz, die zu dem Komplement der Nukleinsäuresequenz von Interesse komplementär ist, die mindestens einen Teil der J-Region und der Isotyp-Region des BCR oder des Fragments davon codiert.

12. Verfahren nach einem der Ansprüche 9 bis 11, wobei der dritte Primer umfasst:

(i) eine Sequenz, die zu Nukleotiden von mindestens einem Teil der Leadersequenz des BCR oder des Fragments davon komplementär ist; oder
(ii) eine Sequenz, die zu Nukleotiden komplementär ist, die mindestens einen Teil der FWR1 des BCR oder des Fragments davon codiert.

13. Verfahren nach einem der Ansprüche 9 bis 12, wobei der vierte Primer umfasst:

(i) eine Sequenz, die zu dem Komplement von mindestens einem Teil der Nukleinsäuresequenz von Interesse komplementär ist, die die CDR3 codiert, sowie den Übergang, der sich in die J-Region des BCR oder des Fragments davon erstreckt;
(ii) eine Sequenz, die zu dem Komplement von mindestens einem Teil der Nukleinsäuresequenz von Interesse komplementär ist, die die D- und J-Regionen codiert, oder einen Übergang zwischen den D- und J-Regionen des BCR oder des Fragments davon;

(iii) eine Sequenz, die zu dem Komplement von mindestens einem Teil der Nukleinsäuresequenz von Interesse komplementär ist, die die V- und J-Regionen codiert, oder einen Übergang zwischen den V- und J-Regionen des BCR oder des Fragments davon;

(iv) eine Sequenz, die zu dem Komplement von mindestens einem Teil der Nukleinsäuresequenz von Interesse komplementär ist, die die V- und D-Regionen codiert, oder einen Übergang zwischen den V- und D-Regionen des BCR oder des Fragments davon; oder

(v) eine Sequenz, die zu dem Komplement von mindestens einem Teil der Nukleinsäuresequenz von Interesse komplementär ist, die die V-, D- und J-Regionen des BCR oder des Fragments davon codiert.

14. Verfahren nach einem der Ansprüche 9-13:

(i) wobei der dritte und der vierte Primer einen nicht bindenden Abschnitt umfassen; und/oder

(ii) ferner umfassend das Klonieren der angereicherten Nukleinsäuresequenz von Interesse in einem Vektor.

15. Verfahren nach einem der vorhergehenden Ansprüche, wobei die Vielzahl von Nukleinsäuremolekülen aus einer Zellprobe eines Spenders oder mehrerer Spender hergestellt wird, optional wobei:

(i) der oder die Spender einem Zielantigen ausgesetzt worden waren, und wobei die Vielzahl von Nukleinsäuremolekülen, die die Nukleinsäuresequenz von Interesse umfasst, eine Nukleinsäuresequenz umfasst, die einen ausgewählten B-Zellrezeptor (BCR) oder ein Fragment davon, das das Zielantigen bindet, codiert; oder

(ii) die Vielzahl von Nukleinsäuremolekülen, die die Nukleinsäuresequenz umfasst, die den ausgewählten B-Zellrezeptor (BCR) oder ein Fragment davon, der/das das Zielantigen bindet, codiert, aus der Zellprobe des Spenders oder der Spender hergestellt werden, umfassend die folgenden Schritte:

(a) Aufteilen eines Reaktionsgemisches in eine Vielzahl von Aufteilungen, wobei das Reaktionsgemisch umfasst:

(i) eine Vielzahl von Zellen der Zellprobe, und
(ii) das Zielantigen,
wobei das Zielantigen an ein Reporter-Oligonukleotid gekoppelt ist,
wobei das Reaktionsgemisch eine Zelle umfasst, die an das Zielantigen gebunden ist,
wobei das Aufteilen eine Aufteilung bereitstellt, die umfasst:

(i) die aufgeteilte Zelle, die an das Zielantigen gebunden ist, und
(ii) eine Vielzahl von Nukleinsäure-Barcode-Molekülen, umfassend eine aufteilungsspezifische Barcode-Sequenz,

(b) Erzeugen von barcodierten Nukleinsäuremolekülen in der Aufteilung, wobei die barcodierten Nukleinsäuremoleküle umfassen:

(i) ein erstes Barcode-Nukleinsäuremolekül, umfassend eine Sequenz des Reporter-Oligonukleotids oder eines reversen Komplements davon sowie die aufteilungsspezifische Barcode-Sequenz oder das reverse Komplement davon, und
(ii) ein zweites barcodiertes Nukleinsäuremolekül, das ein Nukleinsäuremolekül von Interesse für die Einbeziehung in die Vielzahl von Nukleinsäuremolekülen umfasst, wenn das erste barcodierte Nukleinsäuremolekül erkannt wird.

**Revendications**

1. Procédé d'enrichissement pour une séquence d'acide nucléique d'intérêt, comprenant :

(a) la fourniture d'une pluralité de molécules d'acide nucléique comprenant une pluralité de séquences d'identification, dans lequel une molécule d'acide nucléique de ladite pluralité de molécules d'acide nucléique comprend :

(i) une séquence d'identification de ladite pluralité de séquences d'identification qui identifie ladite molécule d'acide nucléique,
dans lequel la séquence d'identification comprend une séquence de code à barres, et/ou une séquence d'identifiant de molécule unique (UMI) ; et
(ii) la séquence d'acide nucléique d'intérêt,
dans lequel la séquence d'acide nucléique d'intérêt comprend une séquence d'acide nucléique codant pour un récepteur des lymphocytes T (TCR), un récepteur des lymphocytes B (BCR), ou un fragment de ceux-ci ;

(b) l'utilisation d'une première amorce d'acide nucléique complémentaire d'au moins une partie de la séquence d'identification dans un premier cycle d'amplification pour amplifier ladite séquence d'acide nucléique d'intérêt ; et
(c) l'utilisation d'une deuxième amorce d'acide nucléique complémentaire d'au moins une partie d'une séquence V(D)J dans un deuxième cycle d'amplification pour amplifier encore ladite séquence d'acide nucléique d'intérêt.

2. Procédé selon la revendication 1, dans lequel la séquence d'identification comprend la séquence de code à barres.

3. Procédé selon la revendication 1 ou 2, dans lequel la séquence d'identification comprend la séquence UMI.

4. Procédé selon l'une quelconque des revendications précédentes, dans lequel le premier cycle d'amplification comprend en outre l'utilisation d'une troisième amorce comprenant une séquence complémentaire d'au moins une partie d'un complément d'une séquence d'acide nucléique codant pour une région constante du TCR, du BCR, ou du fragment de ceux-ci, éventuellement une séquence complémentaire d'au moins une partie d'un complément d'une séquence d'acide nucléique codant pour une région constante du BCR, ou du fragment de celui-ci.

5. Procédé selon l'une quelconque des revendications précédentes, dans lequel le deuxième cycle d'amplification comprend en outre l'utilisation d'une quatrième amorce comprenant une séquence complémentaire d'au moins une partie d'un complément d'une séquence d'acide nucléique codant pour une séquence en aval de la séquence V(D)J, éventuellement dans lequel la quatrième amorce comprend une poignée non liante.

6. Procédé selon l'une quelconque des revendications précédentes, dans lequel la deuxième amorce comprend une poignée non liante.

7. Procédé selon l'une quelconque des revendications précédentes, dans lequel la pluralité de molécules d'acide nucléique fournies comprennent des molécules d'acide désoxyribonucléique complémentaire (ADNc).

8. Procédé selon l'une quelconque des revendications précédentes, dans lequel la séquence d'acide nucléique d'intérêt code pour le BCR, ou le fragment de celui-ci.

9. Procédé pour enrichir une séquence d'acide nucléique d'intérêt, comprenant :

(a) la fourniture d'une pluralité de molécules d'acide nucléique comprenant une pluralité de séquences d'identification, dans lequel une molécule d'acide nucléique de ladite pluralité de molécules d'acide nucléique comprend :

(i) une séquence d'identification de ladite pluralité de séquences d'identification qui identifie ladite molécule d'acide nucléique,
dans lequel la séquence d'identification comprend une séquence de code à barres et une séquence d'identifiant de molécule unique (UMI) ; et
(ii) la séquence d'acide nucléique d'intérêt,
dans lequel la séquence d'acide nucléique d'intérêt comprend une séquence d'acide nucléique codant pour un récepteur des lymphocytes B (BCR) ou un fragment de celui-ci ;

(b) la réalisation d'une première réaction d'amplification avec un premier ensemble d'amorces comprenant :

une première amorce comprenant une séquence complémentaire d'au moins une partie de la séquence de code à barres et/ou de la séquence UMI, et
une deuxième amorce comprenant une séquence complémentaire d'un complément d'au moins une partie de la séquence d'acide nucléique d'intérêt qui code pour une région de jonction (J) et/ou une région d'isotype du BCR ou du fragment de celui-ci ;

(c) la réalisation d'une deuxième réaction d'amplification avec un deuxième ensemble d'amorces comprenant :

une troisième amorce comprenant une séquence complémentaire de nucléotides d'au moins une partie de la séquence de tête et/ou de la région charpente codante (FWR)1 du BCR, ou du fragment de celui-ci ; et une quatrième amorce comprenant une séquence complémentaire d'un complément d'au moins une partie de la séquence d'acide nucléique d'intérêt qui code pour une région de complémentarité (CDR)3, une FWR4, une région J, une région D, et/ou une région V, ou une jonction entre l'une quelconque ou plusieurs de celles-ci, du BCR ou du fragment de celui-ci.

10. Procédé selon la revendication 9, dans lequel la première amorce comprend :

(i) une séquence complémentaire d'au moins une partie de la séquence de code à barres et de la séquence UMI ; ou
(ii) une séquence complémentaire de la séquence de code à barres et de la séquence UMI.

11. Procédé selon la revendication 9 ou la revendication 10, dans lequel la deuxième amorce comprend :

(i) une séquence complémentaire du complément de la séquence d'acide nucléique d'intérêt qui code pour au moins une partie de la région J du BCR ou du fragment de celui-ci ;
(ii) une séquence complémentaire du complément de la séquence d'acide nucléique d'intérêt qui code pour au moins une partie de la région d'isotype du BCR ou du fragment de celui-ci ; ou
(iii) une séquence complémentaire du complément de la séquence d'acide nucléique d'intérêt qui code pour au moins une partie de la région J et de la région d'isotype du BCR ou du fragment de celui-ci.

12. Procédé selon l'une quelconque des revendications 9 à 11, dans lequel la troisième amorce comprend :

(i) une séquence complémentaire de nucléotides d'au moins une partie de la séquence de tête du BCR, ou du fragment de celui-ci ; ou
(ii) une séquence complémentaire de nucléotides codant pour au moins une partie de la FWR1 du BCR, ou du fragment de celui-ci.

13. Procédé selon l'une quelconque des revendications 9 à 12, dans lequel la quatrième amorce comprend :

(i) une séquence complémentaire du complément d'au moins une partie de la séquence d'acide nucléique d'intérêt qui code pour la CDR3, et une jonction s'étendant dans la région J du BCR ou du fragment de celui-ci ;
(ii) une séquence complémentaire du complément d'au moins une partie de la séquence d'acide nucléique d'intérêt qui code pour les régions D et J, ou une jonction entre les régions D et J du BCR ou du fragment de celui-ci ;
(iii) une séquence complémentaire du complément d'au moins une partie de la séquence d'acide nucléique d'intérêt qui code pour les régions V et J, ou une jonction entre les régions V et J, du BCR ou du fragment de celui-ci ;
(iv) une séquence complémentaire du complément d'au moins une partie de la séquence d'acide nucléique d'intérêt qui code pour les régions V et D, ou une jonction entre les régions V et D, du BCR ou du fragment de celui-ci ; ou
(v) une séquence complémentaire du complément d'au moins une partie de la séquence d'acide nucléique d'intérêt qui code pour les régions V, D et J du BCR ou du fragment de celui-ci.

14. Procédé selon l'une quelconque des revendications 9 à 13 :

(i) dans lequel les troisième et quatrième amorces comprennent une poignée non liante ; et/ou
(ii) comprenant en outre le clonage de la séquence d'acide nucléique enrichie d'intérêt dans un vecteur.

15. Procédé selon l'une quelconque des revendications précédentes, dans lequel la pluralité de molécules d'acide nucléique sont préparées à partir d'un échantillon cellulaire d'un donneur ou de donneurs, éventuellement dans lequel :

(i) le donneur ou les donneurs ont été exposés à un antigène cible et dans lequel la pluralité de molécules d'acide nucléique qui comprennent la séquence d'acide nucléique d'intérêt comprennent une séquence d'acide nucléique codant pour un récepteur des lymphocytes B (BCR) sélectionné ou un fragment de celui-ci qui se lie à l'antigène cible ; ou

(ii) la pluralité de molécules d'acide nucléique comprenant la séquence d'acide nucléique codant le récepteur des lymphocytes B (BCR) sélectionné, ou un fragment de celui-ci, qui se lie à l'antigène cible sont préparées à partir de l'échantillon cellulaire du donneur ou des donneurs comprenant les étapes consistant à :

  (a) diviser un mélange réactionnel en une pluralité de divisions, dans lequel le mélange réactionnel comprend :

    (i) une pluralité de cellules de l'échantillon cellulaire, et
    (ii) l'antigène cible,
    dans lequel l'antigène cible est couplé à un oligonucléotide rapporteur,
    dans lequel le mélange réactionnel comprend une cellule liée à l'antigène cible,
    dans lequel l'action de division fournit une division comprenant :

      (i) la cellule divisée liée à l'antigène cible, et
      (ii) une pluralité de molécules de code à barres d'acide nucléique comprenant une séquence de code à barres spécifique à la division,

  (b) dans la division, générer des molécules d'acide nucléique à code à barres, dans lequel les molécules d'acide nucléique à code à barres comprennent :

    (i) une première molécule d'acide nucléique à code à barres comprenant une séquence de l'oligonucléotide rapporteur ou d'un complément inverse de celui-ci et la séquence de code à barres spécifique à la division ou un complément inverse de celle-ci, et
    (ii) une deuxième molécule d'acide nucléique à code à barres comprenant une molécule d'acide nucléique d'intérêt pour une inclusion dans la pluralité de molécules d'acide nucléique si la première molécule nucléique à code à barres est détectée.

**FIG. 1**

EP 4 158 058 B1

FIG. 2

**FIG. 3**

EP 4 158 058 B1

**FIG. 4**

EP 4 158 058 B1

FIG. 5

FIG. 6

**FIG. 7A**

**FIG. 7B**

EP 4 158 058 B1

*FIG. 8*

*FIG. 9*

*FIG. 10*

*FIG. 11*

EP 4 158 058 B1

FIG. 12A

FIG. 12B

1290

1221 1222 1223

1260

AAAAAAAAAA

1230

1240

*FIG. 12C*

75

1340

1311    1312    1313

1310

1321    1312    1323

1340

1320

1340

1351    1312    1353

1350

*FIG. 13A*

EP 4 158 058 B1

**FIG. 13B**

**FIG. 13C**

*FIG. 14*

pR1     BCa   BCb   UMI   Spacer     V       D       J       Constant

dT-SM

BC enrichment 1

BC enrichment 2

C region outer

C region inner

*FIG. 15*

EP 4 158 058 B1

V gene specific
primer

C region inner

Clonable sequence (by gibson assembly, etc):

**FIG. 16**

Single cell sort

Total cDNA synthesis and amplification

HC-- V and J specific primers

LC-- V and J specific primers

Gene specific primers selected e.g.,
based on RACE sequencing

Addition of Gibson ends

A 2$^{nd}$ PCR is used to add Gibson
ends to the amplicons.

Addition of Gibson ends

*FIG. 17*

fspl

fspl

Vector-restriction
digest to create Gene
fragment of constant
and linker

HV          HC constant & linker          LV

## FIG. 18

EP 4 158 058 B1

**FIG. 19**

A

B

*FIG. 20*

*FIG. 21*

*FIG. 22*

FIG. 23

*FIG. 24*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018075693 A
- US 2018105808 A
- US 20180105808 A
- US 5545807 A
- US 5545806 A
- US 5569825 A
- US 5625126 A
- US 5633425 A
- US 5661016 A
- US 5750373 A
- WO 9404690 A
- US 20050037421 A
- US 10550429 B
- US 20140155295
- US 20100105112
- US 20140378345
- US 20150292988 A
- US 20140378345 A
- US 20150376609 A
- US 20190177800 A
- US 20190367969 A
- US 20190323088 A
- US 6265552 B
- US 10954562 B
- US 63135514

### Non-patent literature cited in the description

- **KABAT et al.** Sequences of Proteins of Immunological Interest. National Institutes of Health, 1991
- **AL-IAZIKANI et al.** *J. Molec. Biol.*, 1997, vol. 273, 927-948
- **KABAT et al.** Sequences of Proteins of Immunological Interest. Public Health Service, 1991, 647-669
- **VAUGHAN et al.** *Nature Biotechnology*, 1996, vol. 14, 309-314
- **SHEETS et al.** *PNAS USA*, 1998, vol. 95, 6157-6162
- **HOOGENBOOM** ; **WINTER**. *J. Mol. Biol.*, 1991, vol. 227, 381
- **MARKS et al.** *J. Mol. Biol.*, 1991, vol. 222, 581
- **COLE et al.** Monoclonal Antibodies and Cancer Therapy. Alan R. Liss, 1985, 77
- **BOERNER et al.** *J. Immunol.*, 1991, vol. 147 (1), 86-95
- **SURESH et al.** *Methods in Enzymology*, 1986, vol. 121, 210
- **MILLSTEIN** ; **CUELLO**. *Nature*, 1983, vol. 305, 537-539
- **REITER et al.** *Nature Biotechnology*, 1996, vol. 14, 1239-1245
- **BIRD et al.** *Science*, 1988, vol. 242, 423-426
- **HUSTON et al.** *Proc. Natl. Acad. Sci. USA*, 1988, vol. 85, 5879-5883
- **OSBOURN et al.** *Nat. Biotechnol.*, 1998, vol. 16, 778
- Pluckthun in The Pharmacology of Monoclonal Antibodies. Springer-Verlag, 1994, vol. 113, 269-315
- **SILVERMAN et al.** *Nat. Biotechnol.*, 2005, vol. 23, 1493-1494
- **SILVERMAN et al.** *Nat. Biotechnol.*, 2006, vol. 24, 220
- **MUYLDERMANS et al.** *Protein Engineering*, 1994, vol. 7 (9), 1129
- **FREDERICKS et al.** *Protein Engineering, Design & Selection*, 2004, vol. 17, 95-106
- **POWERS et al.** *Journal of Immunological Methods*, 2001, vol. 251, 123-135
- **HOLLIGER, P. et al.** *Proc. Natl. Acad. Sci. USA*, 1993, vol. 90, 6444-6448
- **POLJAK, R. J. et al.** *Structure*, 1994, vol. 2, 1121-1123
- **OLAFSEN et al.** *Protein Eng Des Sel.*, April 2004, vol. 17 (4), 315-23
- **BIOCCA et al.** *EMBO J.*, 1990, vol. 9, 101-108
- **COLBY et al.** *Proc Natl Acad Sci. USA.*, 2004, vol. 101, 17616-21
- **MHASHILKAR et al.** *EMBO J.*, 1995, vol. 14, 1542-51
- **WHEELER et al.** *FASEB J.*, 2003, vol. 17, 1733-5
- **HENG et al.** *Med Hypotheses.*, 2005, vol. 64, 1105-8
- *Nat Methods.*, 2009, vol. 6 (5), 343-345

- **FANG et al.** Fluoride-Cleavable Biotinylation Phosphoramidite for 5'-end-Labelling and Affinity Purification of Synthetic Oligonucleotides. *Nucleic Acids Res.*, 15 January 2003, vol. 31 (2), 708-715